# EUROPEAN PATENT SPECIFICATION

(11) **EP 3 449 255 B1**
(45) Date of publication and mention of the grant of the patent: **21.08.2024**
(21) Application number: 17790144.4
(22) Date of filing: 20.04.2017
(51) Int. Cl.: G01N 33/543, G01N 33/564, G01N 33/68, A61B 5/145, A61B 5/00

(54) **COMPOSITIONS, DEVICES, AND METHODS OF ULCERATIVE COLITIS SENSITIVITY TESTING**
ZUSAMMENSETZUNGEN, VORRICHTUNGEN UND VERFAHREN FÜR COLITIS-ULCEROSA-EMPFINDLICHKEITSTESTS
COMPOSITIONS, DISPOSITIFS, ET MÉTHODES D'ANALYSE DE LA SENSIBILITÉ À LA COLITE ULCÉREUSE

(30) Priority: 26.04.2016 US 201662327932 P
(43) Date of publication of application: 06.03.2019
(73) Proprietor: Biomerica, Inc., Irvine, California 92614 (US)
(72) Inventor: IRANI-COHEN, Zackary, Irvine, California 92614 (US); LADERMAN, Elisabeth, Irvine, California 92614 (US)
(74) Representative: Schlich, George
(86) International application number: PCT/US2017/028696
(87) International publication number: WO 2017/189338

(56) References cited:
- EP-B1- 1 051 626
- EP-B1- 1 051 626
- WO-A1-2016/077808
- US-A1- 2007 122 840
- US-A1- 2007 122 840
- US-B2- 7 601 509
- US-B2- 7 601 509
- QIANG ZENG ET AL: "Variable Food-Specific IgG Antibody Levels in Healthy and Symptomatic Chinese Adults", PLOS ONE, vol. 8, no. 1, 1 January 2013 (2013-01-01), pages 1 - 9, XP055367030, DOI: 10.1371/journal.pone.0053612
- CHENWEN CAI ET AL: "Serological Investigation of Food Specific Immunoglobulin G Antibodies in Patients with Inflammatory Bowel Diseases", PLOS ONE, vol. 9, no. 11, 13 November 2014 (2014-11-13), pages e112154, XP055459661, DOI: 10.1371/journal.pone.0112154
- XINLING MA ET AL: "Food intolerance prevalence in active ulcerative colitis in southwest China", ASIA PACIFIC JOURNAL OF CLINICAL NUTRITION, September 2016 (2016-09-01), Australia, pages 529 - 533, XP055629927, Retrieved from the Internet <URL:https://pdfs.semanticscholar.org/8eb1/be823ce246ed225762d263ee4b7120e9a549.pdf> DOI: 10.6133/apjcn.102015.04
- ZENG, QIANG ET AL.: "Variable food-specific IgG antibody levels in healthy and symptomatic Chinese adults", PLOS ONE, vol. 8, no. 1, 3 January 2013 (2013-01-03), pages e53612, XP055367030
- CAI, CHENWEN ET AL.: "Serological investigation of food specific immunoglobulin G antibodies in patients with inflammatory bowel diseases", PLOS ONE, vol. 9, no. 11, 13 November 2014 (2014-11-13), pages e112154, XP055459661

## Description

### Field of the Invention

The field of the invention is sensitivity testing for food intolerance, and especially as it relates to testing and possible elimination of selected food items as trigger foods for patients diagnosed with or suspected to have Ulcerative Colitis.

### Background

The background description includes information that may be useful in understanding the present invention. It is not an admission that any of the information provided herein is prior art or relevant to the presently claimed invention, or that any publication specifically or implicitly referenced is prior art.

Food sensitivity, especially as it relates to Ulcerative Colitis (a type of inflammatory bowel disease), often presents with diarrhea mixed with blood and mucus and underlying causes of Ulcerative Colitis are not well understood in the medical community. Most typically, Ulcerative Colitis is diagnosed by endoscopic and radiological tests, along with blood tests or electrolyte tests to identify inflammatory conditions. Unfortunately, treatment of Ulcerative Colitis is often less than effective and may present new difficulties due to immune suppressive or modulatory effects. Elimination of other one or more food items has also shown promise in at least reducing incidence and/or severity of the symptoms. However, Ulcerative Colitis is often quite diverse with respect to dietary items triggering symptoms, and no standardized test to help identify trigger food items with a reasonable degree of certainty is known, leaving such patients often to trial-and-error.

While there are some commercially available tests and labs to help identify trigger foods, the quality of the test results from these labs is generally poor as is reported by a consumer advocacy group (*e.g.*, http://www.which.co.uk/news/2008/08/food-allergy-tests-could-risk-your-health-154711/). Most notably, problems associated with these tests and labs were high false positive rates, high false negative rates, high intra-patient variability, and inter-laboratory variability, rendering such tests nearly useless. Similarly, further inconclusive and highly variable test results were also reported elsewhere (Alternative Medicine Review, Vol. 9, No. 2, 2004: pp 198-207), and the authors concluded that this may be due to food reactions and food sensitivities occurring via a number of different mechanisms. For example, not all Ulcerative Colitis patients show positive response to food A, and not all Ulcerative Colitis patients show negative response to food B. Thus, even if an Ulcerative Colitis patient shows positive response to food A, removal of food A from the patient's diet may not relieve the patient's Ulcerative Colitis symptoms. In other words, it is not well determined whether food samples used in the currently available tests are properly selected based on the high probabilities to correlate sensitivities to those food samples to Ulcerative Colitis.

Thus, even though various tests for food sensitivities are known in the art, all or almost all of them suffer from one or more disadvantages. Therefore, there is still a need for improved compositions, devices, and methods of food sensitivity testing, especially for identification and possible elimination of trigger foods for patients identified with or suspected of having Ulcerative Colitis.

### Summary

The present invention provides a test panel for testing food intolerance in patients diagnosed with or suspected to have ulcerative colitis, comprising:
a plurality of distinct food preparations, wherein each food preparation is independently coupled to an individually addressable solid carrier;
wherein the plurality of distinct food preparations consists of green pea, cantaloupe, pinto bean, cucumber, green pepper, grapefruit, carrot, orange, almond, sardine, sweet potato, broccoli, garlic, lima bean, squashes, celery, string bean, tomato, cauliflower, black walnut, sunflower seed, cane sugar, buck wheat, soybean, lemon, barley, oat, oyster, mustard, rye, peach, chili pepper, spinach, peanut, avocado, shrimp, pineapple, cola nut, rice, cabbage, butter, eggplant, apple, egg, wheat, cottage cheese, sole, cashew, olive, parsley, corn, honey, chocolate, cow's milk, potato, onion, tea, and tobacco.

The plurality of distinct food preparations have an average discriminatory p-value of ≤ 0.07 as determined by raw p-value or an average discriminatory p-value of ≤ 0.10 as determined by FDR multiplicity adjusted p-value.

The present invention also provides an *in vitro* method of testing food intolerance in patients diagnosed with or suspected to have ulcerative colitis, comprising:
contacting green pea, cantaloupe, pinto bean, cucumber, green pepper, grapefruit, carrot, orange, almond, sardine, sweet potato, broccoli, garlic, lima bean, squashes, celery, string bean, tomato, cauliflower, black walnut, sunflower seed, cane sugar, buck wheat, soybean, lemon, barley, oat, oyster, mustard, rye, peach, chili pepper, spinach, peanut, avocado, shrimp, pineapple, cola nut, rice, cabbage, butter, eggplant, apple, egg, wheat, cottage cheese, sole, cashew, olive, parsley, corn, honey, chocolate, cow's milk, potato, onion, tea, and tobacco with a bodily fluid of a patient that is diagnosed with or suspected to have ulcerative colitis, wherein the bodily fluid is associated with a gender identification, and wherein the step of contacting is performed under conditions that allow IgG from the bodily fluid to bind to at least one component of the food preparation;
measuring IgG bound to the at least one component of the food preparation to obtain a signal;
comparing the signal to a gender-stratified reference value for the food preparation using the gender identification to obtain a result; and
updating or generating a report using the result.

Various objects, features, aspects and advantages of the embodiments described herein will become more apparent from the following detailed description of preferred embodiments, along with the accompanying drawing figures in which like numerals represent like components.

### Brief Description of The Drawings

**Table 1** shows a list of food items from which food preparations can be prepared.
**Table 2** shows statistical data of foods ranked according to 2-tailed FDR multiplicity-adjusted p-values.
**Table 3** shows statistical data of ELISA score by food and gender.
**Table 4** shows cutoff values of foods for a predetermined percentile rank.
**Figure 1A** illustrates ELISA signal score of male Ulcerative Colitis patients and control tested with green pea.
**Figure 1B** illustrates a distribution of percentage of male Ulcerative Colitis subjects exceeding the 90^{th} and 95^{th} percentile tested with green pea.
**Figure 1C** illustrates a signal distribution in women along with the 95^{th} percentile cutoff as determined from the female control population tested with green pea.
**Figure 1D** illustrates a distribution of percentage of female Ulcerative Colitis subjects exceeding the 90^{th} and 95^{th} percentile tested with green pea.
**Figure 2A** illustrates ELISA signal score of male Ulcerative Colitis patients and control tested with cantaloupe.
**Figure 2B** illustrates a distribution of percentage of male Ulcerative Colitis subjects exceeding the 90^{th} and 95^{th} percentile tested with cantaloupe.
**Figure 2C** illustrates a signal distribution in women along with the 95^{th} percentile cutoff as determined from the female control population tested with cantaloupe.
**Figure 2D** illustrates a distribution of percentage of female Ulcerative Colitis subjects exceeding the 90^{th} and 95^{th} percentile tested with cantaloupe.
**Figure 3A** illustrates ELISA signal score of male Ulcerative Colitis patients and control tested with pinto bean.
**Figure 3B** illustrates a distribution of percentage of male Ulcerative Colitis subjects exceeding the 90^{th} and 95^{th} percentile tested with pinto bean.
**Figure 3C** illustrates a signal distribution in women along with the 95^{th} percentile cutoff as determined from the female control population tested with pinto bean.
**Figure 3D** illustrates a distribution of percentage of female Ulcerative Colitis subjects exceeding the 90^{th} and 95^{th} percentile tested with pinto bean.
**Figure 4A** illustrates ELISA signal score of male Ulcerative Colitis patients and control tested with cucumber.
**Figure 4B** illustrates a distribution of percentage of male Ulcerative Colitis subjects exceeding the 90^{th} and 95^{th} percentile tested with cucumber.
**Figure 4C** illustrates a signal distribution in women along with the 95^{th} percentile cutoff as determined from the female control population tested with cucumber.
**Figure 4D** illustrates a distribution of percentage of female Ulcerative Colitis subjects exceeding the 90^{th} and 95^{th} percentile tested with cucumber.
**Figure 5A** illustrates distributions of Ulcerative Colitis subjects by number of foods that were identified as trigger foods at the 90^{th} percentile.
**Figure 5B** illustrates distributions of Ulcerative Colitis subjects by number of foods that were identified as trigger foods at the 95^{th} percentile.
**Table 5A** shows raw data of Ulcerative Colitis patients and control with number of positive results based on the 90^{th} percentile.
**Table 5B** shows raw data of Ulcerative Colitis patients and control with number of positive results based on the 95^{th} percentile.
**Table 6A** shows statistical data summarizing the raw data of Ulcerative Colitis patient populations shown in Table 5A.
**Table 6B** shows statistical data summarizing the raw data of Ulcerative Colitis patient populations shown in Table 5B.
**Table 7A** shows statistical data summarizing the raw data of control populations shown in Table 5A.
**Table 7B** shows statistical data summarizing the raw data of control populations shown in Table 5B.
**Table 8A** shows statistical data summarizing the raw data of Ulcerative Colitis patient populations shown in Table 5A transformed by logarithmic transformation.
**Table 8B** shows statistical data summarizing the raw data of Ulcerative Colitis patient populations shown in Table 5B transformed by logarithmic transformation.
**Table 9A** shows statistical data summarizing the raw data of control populations shown in Table 5A transformed by logarithmic transformation.
**Table 9B** shows statistical data summarizing the raw data of control populations shown in Table 5B transformed by logarithmic transformation.
**Table 10A** shows statistical data of an independent T-test to compare the geometric mean number of positive foods between the Ulcerative Colitis and non-Ulcerative Colitis samples based on the 90^{th} percentile.
**Table 10B** shows statistical data of an independent T-test to compare the geometric mean number of positive foods between the Ulcerative Colitis and non-Ulcerative Colitis samples based on the 95^{th} percentile.
**Table 11A** shows statistical data of a Mann-Whitney test to compare the geometric mean number of positive foods between the Ulcerative Colitis and non-Ulcerative Colitis samples based on the 90^{th} percentile.
**Table 11B** shows statistical data of a Mann-Whitney test to compare the geometric mean number of positive foods between the Ulcerative Colitis and non-Ulcerative Colitis samples based on the 95^{th} percentile.

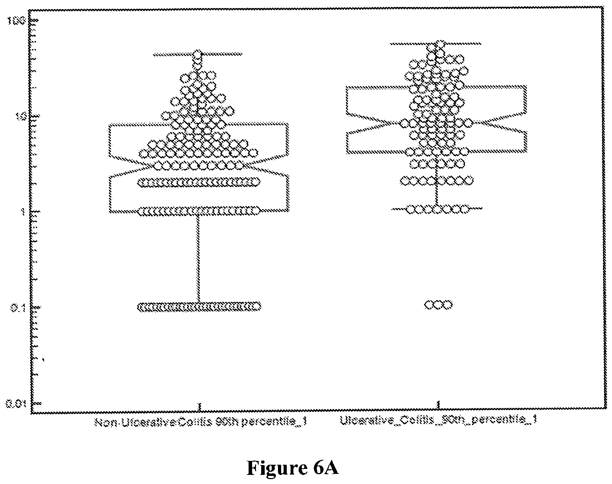

**Figure 6A** illustrates a box and whisker plot of data shown in Table 5A.

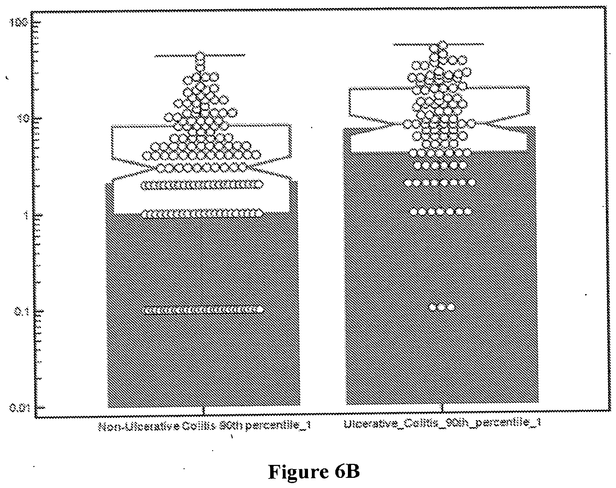

**Figure 6B** illustrates a notched box and whisker plot of data shown in Table 5A.

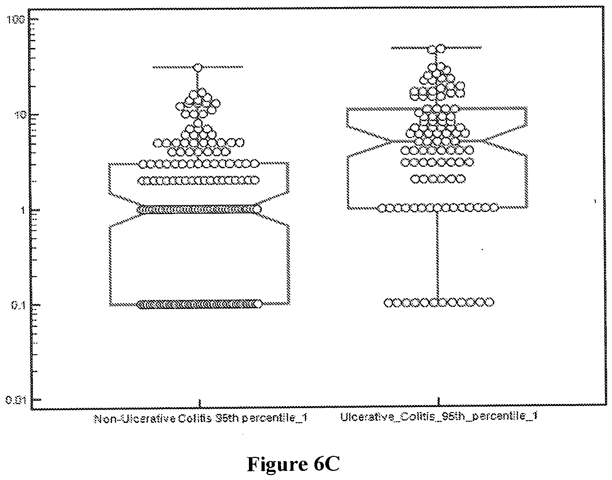

**Figure 6C** illustrates a box and whisker plot of data shown in Table 5B.

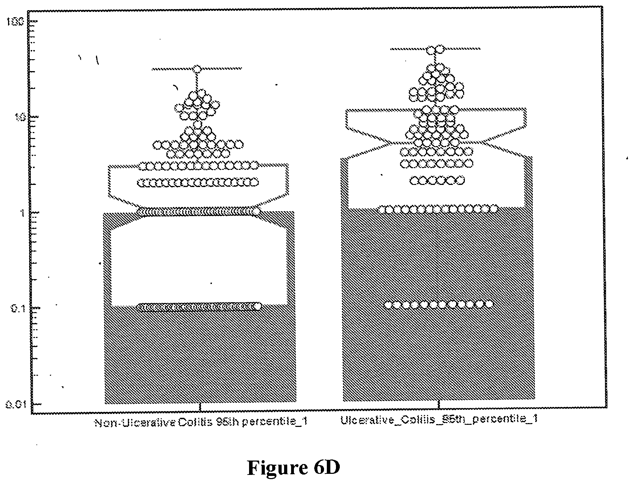

**Figure 6D** illustrates a notched box and whisker plot of data shown in Table 5B.
**Table 12A** shows statistical data of a Receiver Operating Characteristic (ROC) curve analysis of data shown in Tables 5A-11A.
**Table 12B** shows statistical data of a Receiver Operating Characteristic (ROC) curve analysis of data shown in Tables 5B-11B.

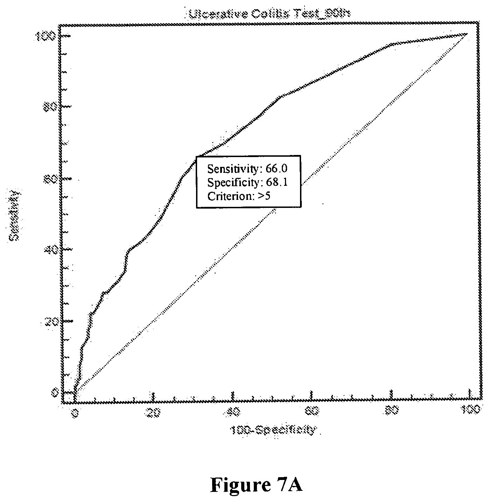

**Figure 7A** illustrates the ROC curve corresponding to the statistical data shown in Table 12A.

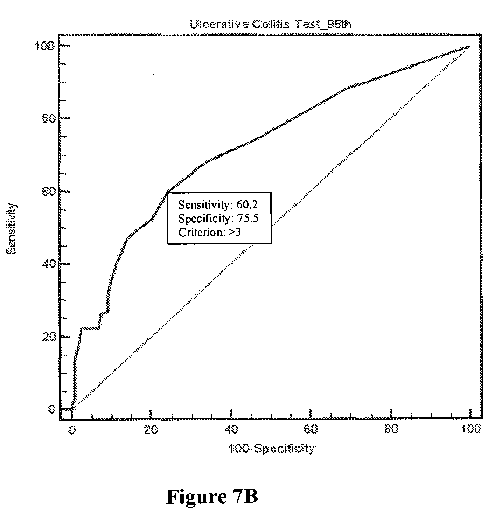

**Figure 7B** illustrates the ROC curve corresponding to the statistical data shown in Table 12B.
**Table 13A** shows a statistical data of performance metrics in predicting Ulcerative Colitis status among female patients from number of positive foods based on the 90^{th} percentile.
**Table 13B** shows a statistical data of performance metrics in predicting Ulcerative Colitis status among male patients from number of positive foods based on the 90^{th} percentile.
**Table 14A** shows a statistical data of performance metrics in predicting Ulcerative Colitis status among female patients from number of positive foods based on the 95^{th} percentile.
**Table 14B** shows a statistical data of performance metrics in predicting Ulcerative Colitis status among male patients from number of positive foods based on the 95^{th} percentile.

### Detailed Description

The inventors have discovered that food preparations used in food tests to identify trigger foods in patients diagnosed with or suspected to have Ulcerative Colitis are not equally well predictive and/or associated with Ulcerative Colitis/Ulcerative Colitis symptoms. Indeed, various experiments have revealed that among a wide variety of food items certain food items are highly predictive/associated with Ulcerative Colitis whereas others have no statistically significant association with Ulcerative Colitis.

Even more unexpectedly, the inventors discovered that in addition to the high variability of food items, gender variability with respect to response in a test plays a substantial role in the determination of association or a food item with Ulcerative Colitis. Consequently, based on the inventors' findings and further contemplations, test kits and methods are now presented with substantially higher predictive power in the choice of food items that could be eliminated for reduction of Ulcerative Colitis signs and symptoms.

The following discussion provides many example embodiments of the inventive subject matter. Although each embodiment represents a single combination of inventive elements, the inventive subject matter is considered to include all possible combinations of the disclosed elements. Thus if one embodiment comprises elements A, B, and C, and a second embodiment comprises elements B and D, then the inventive subject matter is also considered to include other remaining combinations of A, B, C, or D, even if not explicitly disclosed.

In some embodiments, the numbers expressing quantities or ranges, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements. Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements. Moreover, and unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

While not limiting to the inventive subject matter, food preparations will typically be drawn from foods generally known or suspected to trigger signs or symptoms of Ulcerative Colitis. Particularly suitable food preparations may be identified by the experimental procedures outlined below.

Therefore, exemplary food preparations include foods 1-58 of Table 2. Still further especially contemplated food items and food additives from which food preparations can be prepared are listed in Table 1.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Of course, it should be noted that the particular format of the test kit or panel may vary considerably and contemplated formats include micro well plates, dip sticks, membrane-bound arrays, etc. Consequently, the solid carrier to which the food preparations are coupled may include wells of a multiwell plate, a bead (e.g., color-coded or magnetic), or an adsorptive film (e.g., nitrocellulose or micro/nanoporous polymeric film), or an electrical sensor (e.g., a printed copper sensor or microchip).

Consequently, the inventors also contemplate a method of testing food intolerance in patients that are diagnosed with or suspected to have Ulcerative Colitis.

As also noted above, all of the different food preparations have an average discriminatory p-value of ≤ 0.07 as determined by raw p-value, and/or or an average discriminatory p-value of ≤ 0.10 as determined by FDR multiplicity adjusted p-value.

While in certain embodiments food preparations are prepared from single food items as crude extracts, or crude filtered extracts, it is contemplated that food preparations can be prepared from mixtures of a plurality of food items (*e*.*g*., a mixture of citrus comprising lemon, orange, and a grapefruit, a mixture of yeast comprising baker's yeast and brewer's yeast, a mixture of rice comprising a brown rice and white rice, a mixture of sugars comprising honey, malt, and cane sugar. In some embodiments, it is also contemplated that food preparations can be prepared from purified food antigens or recombinant food antigens.

As it is generally preferred that the food preparation is immobilized on a solid surface (typically in an addressable manner), it is contemplated that the step of measuring the IgG bound to the component of the food preparation is performed via an ELISA test. Exemplary solid surfaces include, but are not limited to, wells in a multiwell plate, such that each food preparation may be isolated to a separate microwell. In certain embodiments, the food preparation will be coupled to, or immobilized on, the solid surface. In other embodiments, the food preparation(s) will be coupled to a molecular tag that allows for binding to human immunoglobulins (e.g., IgG) in solution.

Thus, it should be appreciated that by having a high-confidence test system as described herein, the rate of false-positive and false negatives can be significantly reduced, and especially where the test systems and methods are gender stratified or adjusted for gender differences as shown below. Such advantages have heretofore not been realized and it is expected that the systems and methods presented herein will substantially increase the predictive power of food sensitivity tests for patients diagnosed with or suspected to have Ulcerative Colitis.

### Experiments

General Protocol for food preparation generation: Commercially available food extracts (available from Biomerica Inc., 17571 Von Karman Ave, Irvine, CA 92614) prepared from the edible portion of the respective raw foods were used to prepare ELISA plates following the manufacturer's instructions.

For some food extracts, the inventors expect that food extracts prepared with specific procedures to generate food extracts provides more superior results in detecting elevated IgG reactivity in Ulcerative Colitis patients compared to commercially available food extracts. For example, for grains and nuts, a three-step procedure of generating food extracts is preferred. The first step is a defatting step. In this step, lipids from grains and nuts are extracted by contacting the flour of grains and nuts with a non-polar solvent and collecting residue. Then, the defatted grain or nut flour are extracted by contacting the flour with elevated pH to obtain a mixture and removing the solid from the mixture to obtain the liquid extract. Once the liquid extract is generated, the liquid extract is stabilized by adding an aqueous formulation. In a preferred embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For another example, for meats and fish, a two step procedure of generating food extract is preferred. The first step is an extraction step. In this step, extracts from raw, uncooked meats or fish are generated by emulsifying the raw, uncooked meats or fish in an aqueous buffer formulation in a high impact pressure processor. Then, solid materials are removed to obtain liquid extract. Once the liquid extract is generated, the liquid extract is stabilized by adding an aqueous formulation. In a preferred embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For still another example, for fruits and vegetables, a two step procedure of generating food extract is preferred. The first step is an extraction step. In this step, liquid extracts from fruits or vegetables are generated using an extractor (e.g., masticating juicer, etc) to pulverize foods and extract juice. Then, solid materials are removed to obtain liquid extract. Once the liquid extract is generated, the liquid extract is stabilized by adding an aqueous formulation. In a preferred embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

Blocking of ELISA plates: To optimize signal to noise, plates will be blocked with a proprietary blocking buffer. In a preferred embodiment, the blocking buffer includes 20-50 mM of buffer from 4-9 pH, a protein of animal origin and a short chain alcohol. Other blocking buffers, including several commercial preparations, can be attempted but may not provide adequate signal to noise and low assay variability required.

ELISA preparation and sample testing: Food antigen preparations were immobilized onto respective microtiter wells following the manufacturer's instructions. For the assays, the food antigens were allowed to react with antibodies present in the patients' serum, and excess serum proteins were removed by a wash step. For detection of IgG antibody binding, enzyme labeled anti-IgG antibody conjugate was allowed to react with antigen-antibody complex. A color was developed by the addition of a substrate that reacts with the coupled enzyme. The color intensity was measured and is directly proportional to the concentration of IgG antibody specific to a particular food antigen.

Methodology to determine ranked food list in order of ability of ELISA signals to distinguish Ulcerative Colitis from control subjects: Out of an initial selection (e.g., 100 food items, or 150 food items, or even more), samples can be eliminated prior to analysis due to low consumption in an intended population. In addition, specific food items can be used as being representative of a larger generic food group, especially where prior testing has established a correlation among different species within a generic group (most preferably in both genders, but also suitable for correlation for a single gender). For example, green pepper could be dropped in favor of chili pepper as representative of the "pepper" food group, or sweet potato could be dropped in favor of potato as representative of the "potato" food group. In further preferred aspects, the final list foods will be shorter than 50 food items, and more preferably equal or less than of 40 food items.

Since the foods ultimately selected for the food intolerance panel will not be specific for a particular gender, a gender-neutral food list is necessary. Since the observed sample will be at least initially imbalanced by gender (e.g., Controls: 40% female, Ulcerative Colitis: 55% female), differences in ELISA signal magnitude strictly due to gender will be removed by modeling signal scores against gender using a two-sample t-test and storing the residuals for further analysis. For each of the tested foods, residual signal scores will be compared between Ulcerative Colitis and controls using a permutation test on a two-sample t-test with a relative high number of resamplings (e.g., >1,000, more preferably >10,000, even more preferably >50,000). The Satterthwaite approximation can then be used for the denominator degrees of freedom to account for lack of homogeneity of variances, and the 2-tailed permuted p-value will represent the raw p-value for each food. False Discovery Rates (FDR) among the comparisons, will be adjusted by any acceptable statistical procedures (e.g., Benjamini-Hochberg, Family-wise Error Rate (FWER), Per Comparison Error Rate (PCER), etc.).

Foods were then ranked according to their 2-tailed FDR multiplicity-adjusted p-values. Foods with adjusted p-values equal to or lower than the desired FDR threshold are deemed to have significantly higher signal scores among Ulcerative Colitis than control subjects and therefore deemed candidates for inclusion into a food intolerance panel. A typical result that is representative of the outcome of the statistical procedure is provided in **Table** 2. Here the ranking of foods is according to 2-tailed permutation T-test p-values with FDR adjustment.

Based on earlier experiments (data not shown here, see US 62/327932), the inventors contemplate that even for the same food preparation tested, the ELISA score for at least several food items will vary dramatically, and exemplary raw data are provided in **Table 3.** As should be readily appreciated, data unstratified by gender will therefore lose significant explanatory power where the same cutoff value is applied to raw data for male and female data. To overcome such disadvantage, the inventors therefore contemplate stratification of the data by gender as described below.

Statistical Method for Cutpoint Selection for each Food: The determination of what ELISA signal scores would constitute a "positive" response can be made by summarizing the distribution of signal scores among the Control subjects. For each food, Ulcerative Colitis subjects who have observed scores greater than or equal to selected quantiles of the Control subject distribution will be deemed "positive". To attenuate the influence of any one subject on cutpoint determination, each food-specific and gender-specific dataset will be bootstrap resampled 1000 times. Within each bootstrap replicate, the 90th and 95th percentiles of the Control signal scores will be determined. Each Ulcerative Colitis subject in the bootstrap sample will be compared to the 90th and 95% percentiles to determine whether he/she had a "positive" response. The final 90th and 95th percentile-based cutpoints for each food and gender will be computed as the average 90th and 95th percentiles across the 1000 samples. The number of foods for which each Ulcerative Colitis subject will be rated as "positive" was computed by pooling data across foods. Using such method, the inventors will be now able to identify cutoff values for a predetermined percentile rank that in most cases was substantially different as can be taken from **Table 4.**

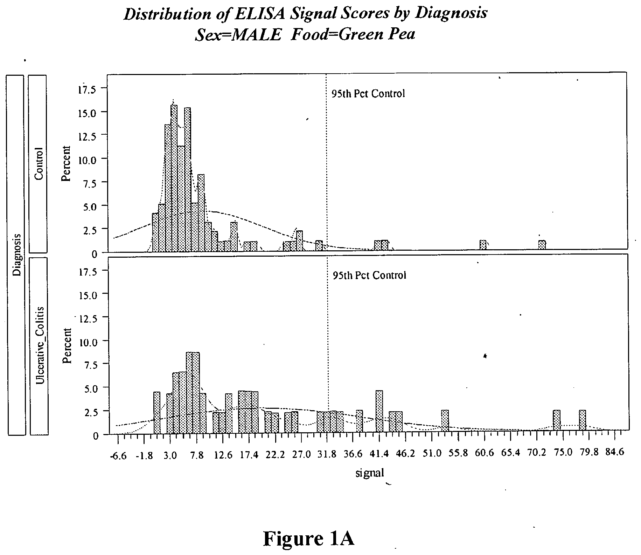

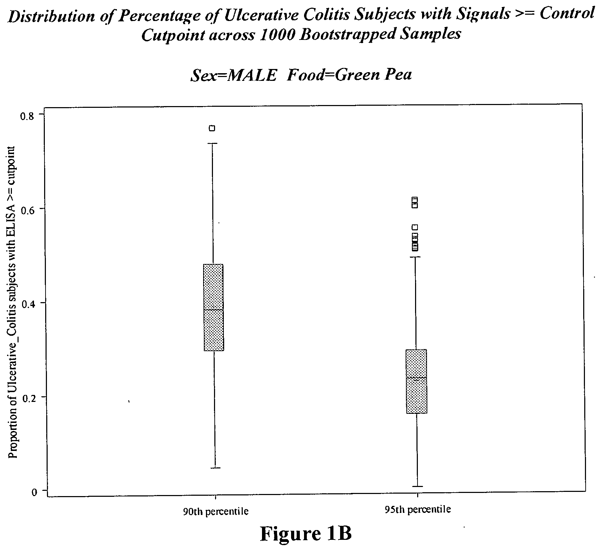

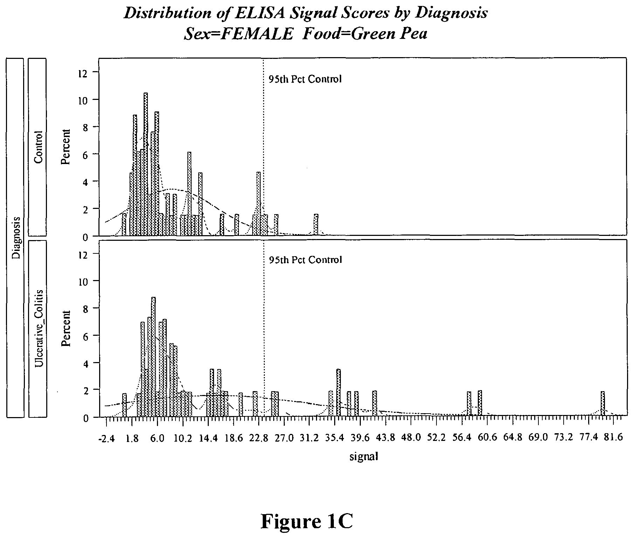

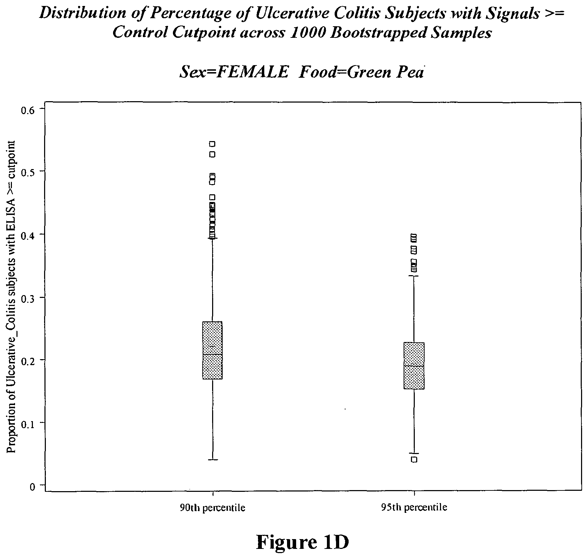

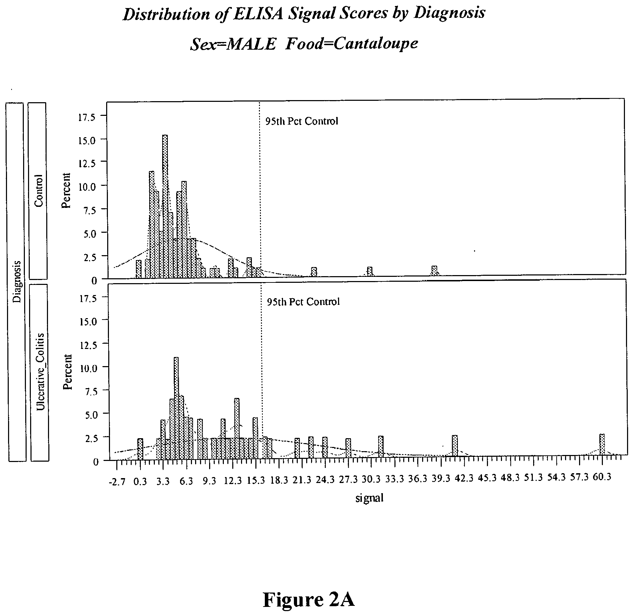

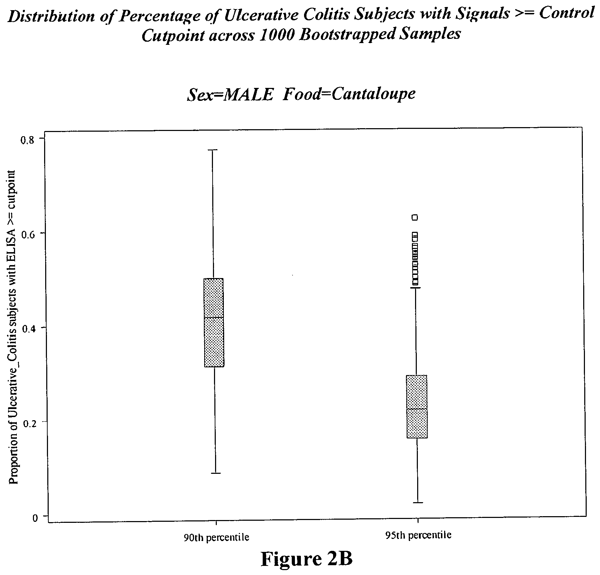

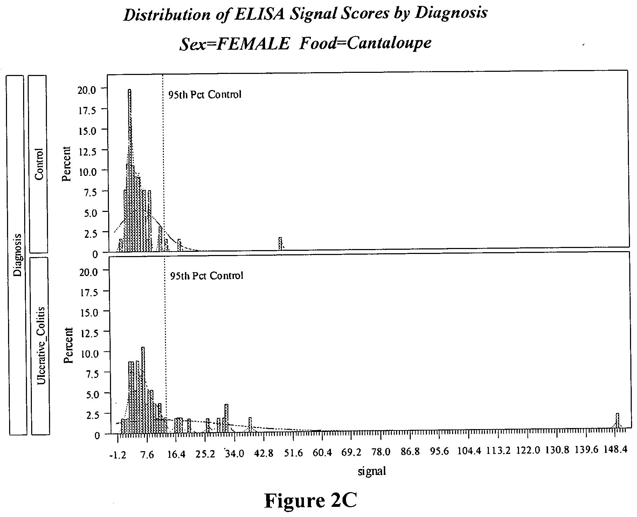

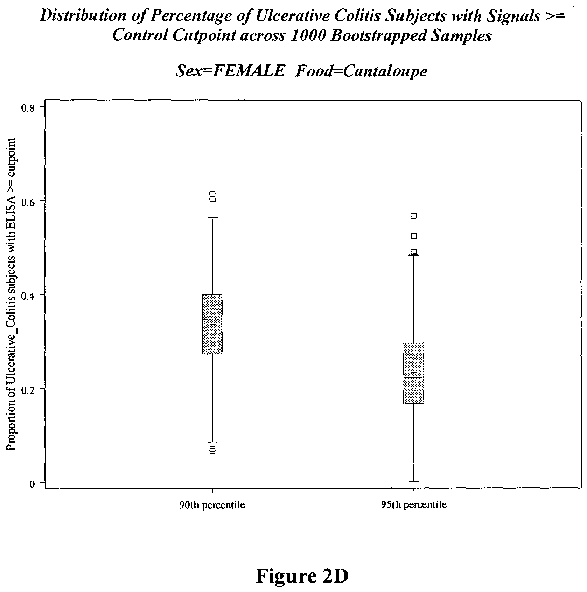

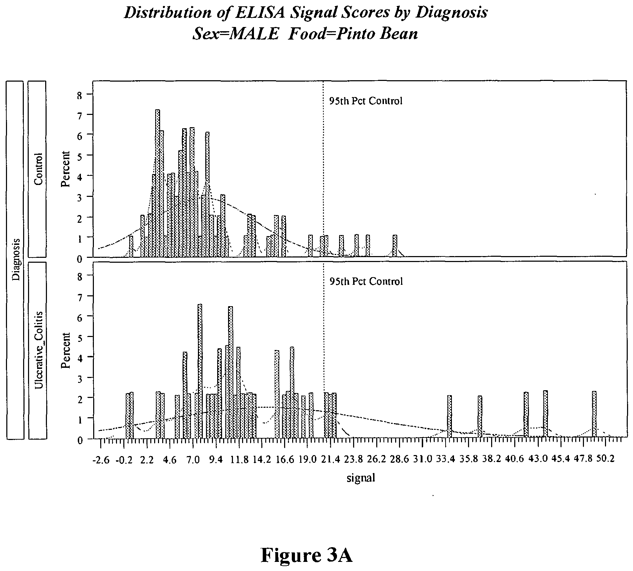

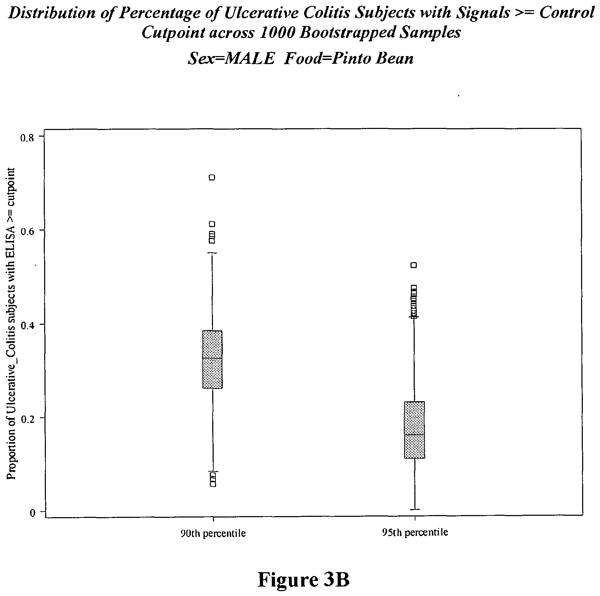

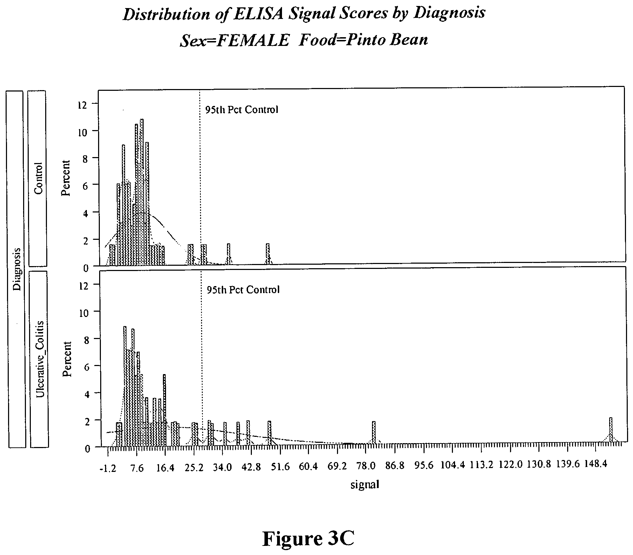

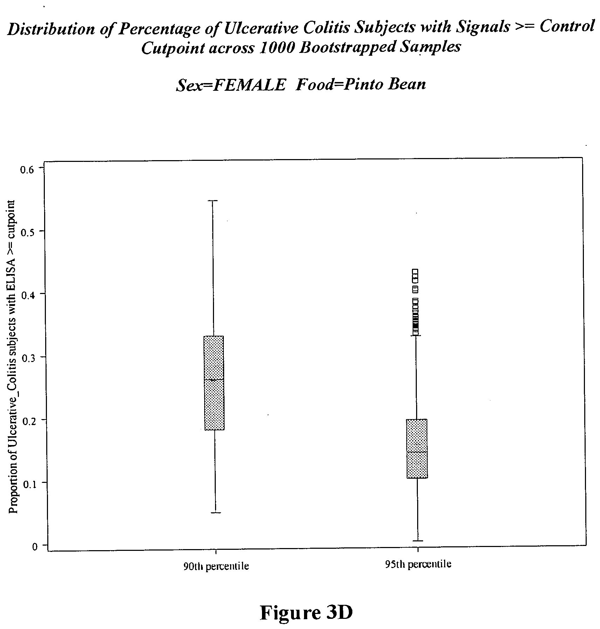

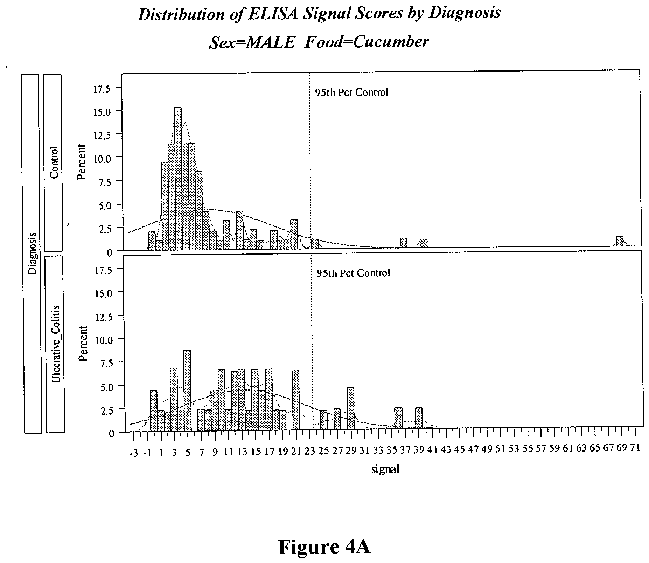

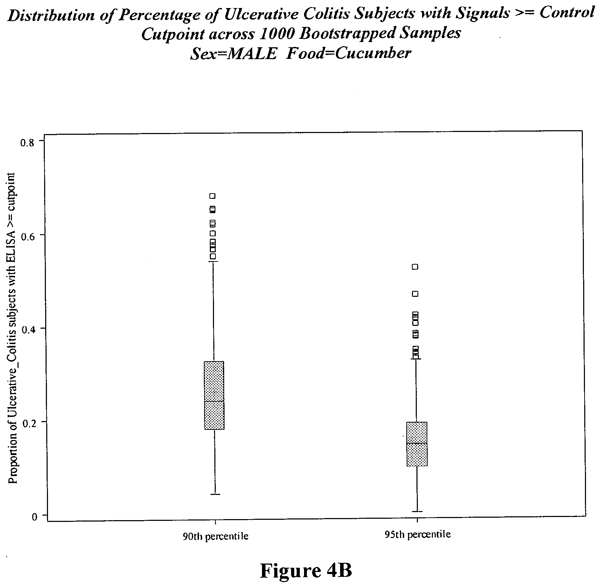

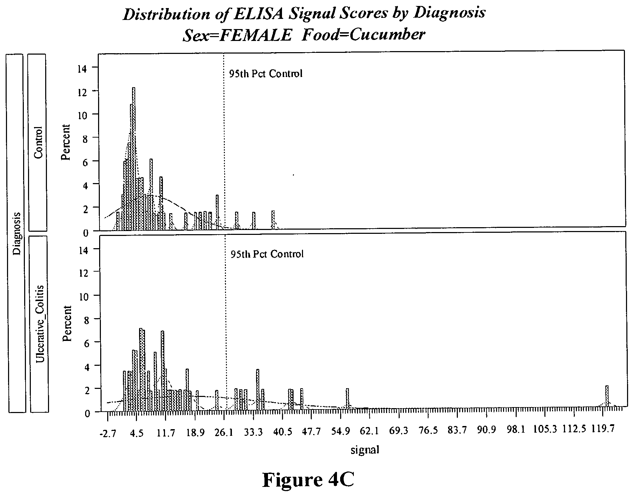

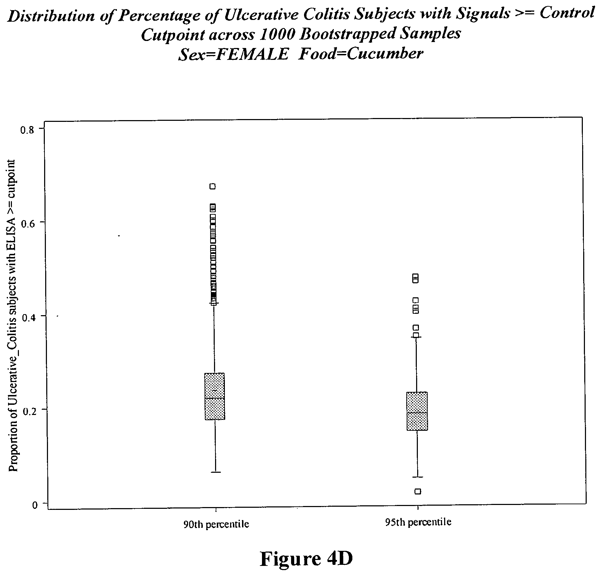

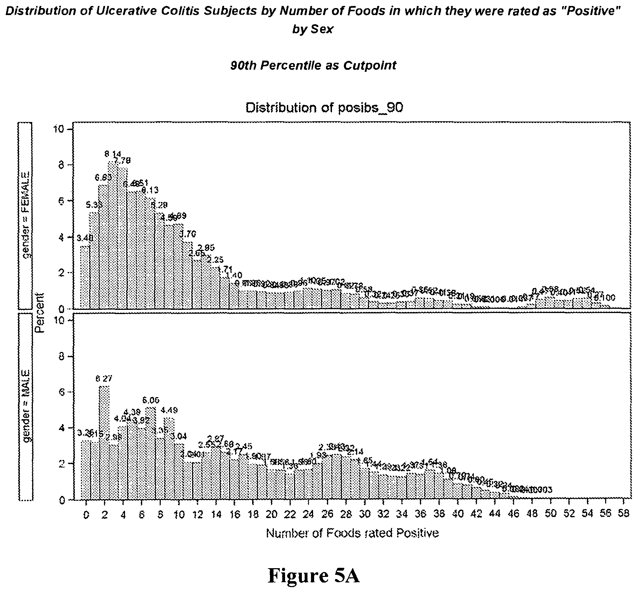

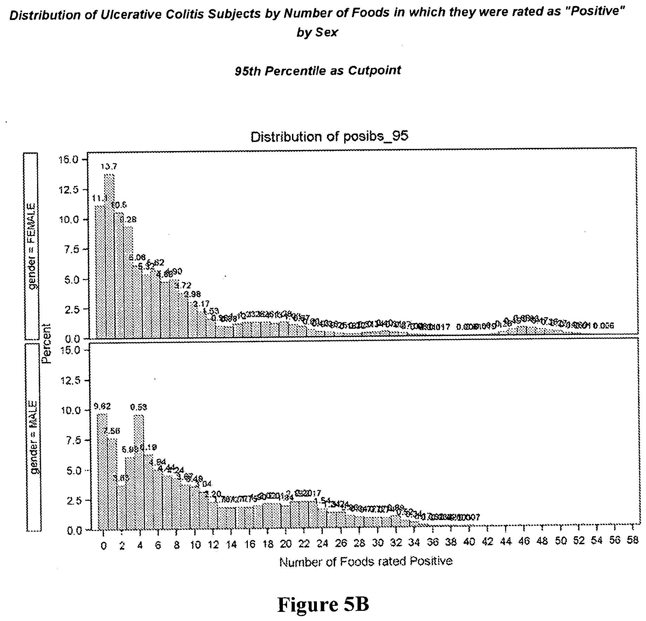

Typical examples for the gender difference in IgG response in blood with respect to green pea is shown in **Figures 1A-1D****,** where Figure 1A shows the signal distribution in men along with the 95^{th} percentile cutoff as determined from the male control population. Figure 1B shows the distribution of percentage of male Ulcerative Colitis subjects exceeding the 90^{th} and 95^{th} percentile, while Figure 1C shows the signal distribution in women along with the 95^{th} percentile cutoff as determined from the female control population. Figure 1D shows the distribution of percentage of female Ulcerative Colitis subjects exceeding the 90^{th} and 95^{th} percentile. In the same fashion, **Figures 2A-2D** exemplarily depict the differential response to cantaloupe, **Figures 3A-3D** exemplarily depict the differential response to pinto bean, and **Figures 4A-4D** exemplarily depict the differential response to cucumber. **Figures 5A-5B** show the distribution of Ulcerative Colitis subjects by number of foods that were identified as trigger foods at the 90^{th} percentile (SA) and 95^{th} percentile (5B). Inventors contemplate that regardless of the particular food items, male and female responses will be notably distinct.

It should be noted that nothing in the art have provided any predictable food groups related to Ulcerative Colitis that is gender-stratified. Thus, a discovery of food items that show distinct responses by gender is a surprising result, which could not be obviously expected in view of all previously available arts. In other words, selection of food items based on gender stratification provides an unexpected technical effect such that statistical significances for particular food items as triggering food among male or female Ulcerative Colitis patients have been significantly improved.

Normalization of IgG Response Data: While the raw data of the patient's IgG response results can be used to compare strength of response among given foods, it is also contemplated that the IgG response results of a patient are normalized and indexed to generate unit-less numbers for comparison of relative strength of response to a given food. For example, one or more of a patient's food specific IgG results (*e.g.*, IgG specific to orange and IgG specific to malt) can be normalized to the patient's total IgG. The normalized value of the patient's IgG specific to orange can be 0.1 and the normalized value of the patient's IgG specific to malt can be 0.3. In this scenario, the relative strength of the patient's response to malt is three times higher compared to orange. Then, the patient's sensitivity to malt and orange can be indexed as such.

In other examples, one or more of a patient's food specific IgG results (e.g., IgG specific to shrimp and IgG specific to pork) can be normalized to the global mean of that patient's food specific IgG results. The global means of the patient's food specific IgG can be measured by total amount of the patient's food specific IgG. In this scenario, the patient's specific IgG to shrimp can be normalized to the mean of patient's total food specific IgG (e.g., mean of IgG levels to shrimp, pork, Dungeness crab, chicken, peas, etc.). However, it is also contemplated that the global means of the patient's food specific IgG can be measured by the patient's IgG levels to a specific type of food via multiple tests. If the patient have been tested for his sensitivity to shrimp five times and to pork seven times previously, the patient's new IgG values to shrimp or to pork are normalized to the mean of five-times test results to shrimp or the mean of seven-times test results to pork. The normalized value of the patient's IgG specific to shrimp can be 6.0 and the normalized value of the patient's IgG specific to pork can be 1.0. In this scenario, the patient has six times higher sensitivity to shrimp at this time compared to his average sensitivity to shrimp, but substantially similar sensitivity to pork. Then, the patient's sensitivity to shrimp and pork can be indexed based on such comparison.

Methodology to determine the subset of Ulcerative Colitis patients with food sensitivities that underlie Ulcerative Colitis: While it is suspected that food sensitivities plays a substantial role in signs and symptoms of Ulcerative Colitis, some Ulcerative Colitis patients may not have food sensitivities that underlie Ulcerative Colitis. Those patients would not be benefit from dietary intervention to treat signs and symptoms of Ulcerative Colitis. To determine the subset of such patients, body fluid samples of Ulcerative Colitis patients and non-Ulcerative Colitis patients can be tested with ELISA test using test devices with up to 58 food samples.

**Table 5A** and **Table 5B** provide exemplary raw data. As should be readily appreciated, the data indicate number of positive results out of 58 sample foods based on 90^{th} percentile value (**Table 5A**) or 95^{th} percentile value (**Table 5B**). The first column is Ulcerative Colitis (n=103); second column is non-Ulcerative Colitis (n=163) by ICD-10 code. Average and median number of positive foods was computed for Ulcerative Colitis and non-Ulcerative Colitis patients. From the raw data shown in **Table 5A** and **Table 5B**, average and standard deviation of the number of positive foods was computed for Ulcerative Colitis and non-Ulcerative Colitis patients. Additionally, the number and percentage of patients with zero positive foods was calculated for both Ulcerative Colitis and non-Ulcerative Colitis. The number and percentage of patients with zero positive foods in the Ulcerative Colitis population is more than 6-fold lower than the percentage of patients with zero positive foods in the non-Ulcerative Colitis population (3% vs. 19%, respectively) based on 90^{th} percentile value (**Table 5A**), and the percentage of patients in the Ulcerative Colitis population with zero positive foods is also less than half of that seen in the non-Ulcerative Colitis population (12 % vs. 31%, respectively) based on 95^{th} percentile value (**Table 5B**). Thus, it can be easily appreciated that the Ulcerative Colitis patient having sensitivity to zero positive foods is unlikely to have food sensitivities underlying their signs and symptoms of Ulcerative Colitis.

**Table 6A** and **Table 7A** show exemplary statistical data summarizing the raw data of two patient populations shown in Table 5A. The statistical data includes normality, arithmetic mean, median, percentiles and 95% confidence interval (CI) for the mean and median representing number of positive foods in the Ulcerative Colitis population and the non-Ulcerative Colitis population. **Table 6B** and **Table 7B** show exemplary statistical data summarizing the raw data of two patient populations shown in Table 5B. The statistical data includes normality, arithmetic mean, median, percentiles and 95% confidence interval (CI) for the mean and median representing number of positive foods in the Ulcerative Colitis population and the non-Ulcerative Colitis population.

**Table 8A** and **Table 9A** show exemplary statistical data summarizing the raw data of two patient populations shown in Table 5A. In Tables 8A and 9A, the raw data was transformed by logarithmic transformation to improve the data interpretation. **Table 8B** and **Table 9B** show another exemplary statistical data summarizing the raw data of two patient populations shown in Table 5B. In Tables 8B and 9B, the raw data was transformed by logarithmic transformation to improve the data interpretation.

**Table 10A** and **Table 11A** show exemplary statistical data of an independent T-test (Table 10A, logarithmically transformed data) and a Mann-Whitney test (Table 11A) to compare the geometric mean number of positive foods between the Ulcerative Colitis and non-Ulcerative Colitis samples. The data shown in Table 10A and Table 11A indicate statistically significant differences in the geometric mean of positive number of foods between the Ulcerative Colitis population and the non-Ulcerative Colitis population. In both statistical tests, it is shown that the number of positive responses with 58 food samples is significantly higher in the Ulcerative Colitis population than in the non-Ulcerative Colitis population with an average discriminatory p-value of ≤ 0.0001. These statistical data is also illustrated as a box and whisker plot in **Figure 6A**, and a notched box and whisker plot in **Figure 6B****.**

**Table 10B** and **Table 11B** show exemplary statistical data of an independent T-test (Table 10A, logarithmically transformed data) and a Mann-Whitney test (Table 11B) to compare the geometric mean number of positive foods between the Ulcerative Colitis and non-Ulcerative Colitis samples. The data shown in Table 10B and Table 11B indicate statistically significant differences in the geometric mean of positive number of foods between the Ulcerative Colitis population and the non-Ulcerative Colitis population. In both statistical tests, it is shown that the number of positive responses with 58 food samples is significantly higher in the Ulcerative Colitis population than in the non-Ulcerative Colitis population with an average discriminatory p-value of ≤ 0.0001. These statistical data is also illustrated as a box and whisker plot in **Figure 6C**, and a notched box and whisker plot in **Figure 6D****.**

**Table 12A** shows exemplary statistical data of a Receiver Operating Characteristic (ROC) curve analysis of data shown in Tables 5A-11A to determine the diagnostic power of the test used in Table 5 at discriminating Ulcerative Colitis from non-Ulcerative Colitis subjects. When a cutoff criterion of more than 5 positive foods is used, the test yields a data with 66% sensitivity and 68% specificity, with an area under the curve (AUROC) of 0.720. The p-value for the ROC is significant at a p-value of <0.0001. **Figure 7A** illustrates the ROC curve corresponding to the statistical data shown in Table 12A. Because the statistical difference between the Ulcerative Colitis population and the non-Ulcerative Colitis population is significant when the test results are cut off to a positive number of 5, the number of foods for which a patient tests positive could be used as a confirmation of the primary clinical diagnosis of Ulcerative Colitis, and whether it is likely that food sensitivities underlies on the patient's signs and symptoms of Ulcerative Colitis. Therefore, the above test can be used as another 'rule in' test to add to currently available clinical criteria for diagnosis for Ulcerative Colitis.

As shown in Tables 5A-12A, and Figure 7A, based on 90^{th} percentile data, the number of positive foods seen in Ulcerative Colitis vs. non-Ulcerative Colitis subjects is significantly different whether the geometric mean or median of the data is compared. The number of positive foods that a person has is indicative of the presence of Ulcerative Colitis in subjects. The test has discriminatory power to detect Ulcerative Colitis with -66% sensitivity and -68% specificity. Additionally, the absolute number and percentage of subjects with 0 positive foods is also very different in Ulcerative Colitis vs. non-Ulcerative Colitis subjects, with a far lower percentage of Ulcerative Colitis subjects (3%) having 0 positive foods than non-Ulcerative Colitis subjects (19%). The data suggests a subset of Ulcerative Colitis patients may have Ulcerative Colitis due to other factors than diet, and may not benefit from dietary restriction.

**Table 12B** shows exemplary statistical data of a Receiver Operating Characteristic (ROC) curve analysis of data shown in Tables 5B-1 1B to determine the diagnostic power of the test used in Table 5 at discriminating Ulcerative Colitis from non-Ulcerative Colitis subjects. When a cutoff criterion of more than 3 positive foods is used, the test yields a data with 60.2% sensitivity and 75.5% specificity, with an area under the curve (AUROC) of 0.719. The p-value for the ROC is significant at a p-value of <0.0001. **Figure 7B** illustrates the ROC curve corresponding to the statistical data shown in Table 12B. Because the statistical difference between the Ulcerative Colitis population and the non-Ulcerative Colitis population is significant when the test results are cut off to positive number of >3, the number of foods that a patient tests positive could be used as a confirmation of the primary clinical diagnosis of Ulcerative Colitis, and whether it is likely that food sensitivities underlies on the patient's signs and symptoms of Ulcerative Colitis. Therefore, the above test can be used as another 'rule in' test to add to currently available clinical criteria for diagnosis for Ulcerative Colitis.

As shown in Tables 5B-12B, and Figure 7B, based on 95^{th} percentile data, the number of positive foods seen in Ulcerative Colitis vs. non-Ulcerative Colitis subjects is significantly different whether the geometric mean or median of the data is compared. The number of positive foods that a person has is indicative of the presence of Ulcerative Colitis in subjects. The test has discriminatory power to detect Ulcerative Colitis with ∼60% sensitivity and -76% specificity. Additionally, the absolute number and percentage of subjects with 0 positive foods is also very different in Ulcerative Colitis vs. non-Ulcerative Colitis subjects, with a far lower percentage of Ulcerative Colitis subjects (-19%) having 0 positive foods than non- Ulcerative Colitis subjects (-31%). The data suggests a subset of Ulcerative Colitis patients may have Ulcerative Colitis due to other factors than diet, and may not benefit from dietary restriction.

Method for determining distribution of per-person number of foods declared "positive": To determine the distribution of number of "positive" foods per person and measure the diagnostic performance, the analysis will be performed with 58 food items from Table 2, which shows most positive responses to Ulcerative Colitis patients. To attenuate the influence of any one subject on this analysis, each food-specific and gender-specific dataset will be bootstrap resampled 1000 times. Then, for each food item in the bootstrap sample, sex-specific cutpoint will be determined using the 90th and 95th percentiles of the control population. Once the sex-specific cutpoints are determined, the sex-specific cutpoints will be compared with the observed ELISA signal scores for both control and Ulcerative Colitis subjects. In this comparison, if the observed signal is equal or more than the cutpoint value, then it will be determined "positive" food, and if the observed signal is less than the cutpoint value, then it will be determined "negative" food.

Once all food items were determined either positive or negative, the results of the 116 (58 foods x 2 cutpoints) calls for each subject will be saved within each bootstrap replicate. Then, for each subject, 58 calls will be summed using 90^{th} percentile as cutpoint to get "Number of Positive Foods (90^{th})," and the rest of 58 calls will be summed using 95^{th} percentile to get "Number of Positive Foods (95^{th})." Then, within each replicate, "Number of Positive Foods (90^{th})" and "Number of Positive Foods (95^{th})" will be summarized across subjects to get descriptive statistics for each replicate as follows: 1) overall means equals to the mean of means, 2) overall standard deviation equals to the mean of standard deviations, 3) overall medial equals to the mean of medians, 4) overall minimum equals to the minimum of minimums, and 5) overall maximum equals to maximum of maximum. In this analysis, to avoid non-integer "Number of Positive Foods" when computing frequency distribution and histogram, the authors will pretend that the 1000 repetitions of the same original dataset were actually 999 sets of new subjects of the same size added to the original sample. Once the summarization of data is done, frequency distributions and histograms will be generated for both "Number of Positive Foods (90^{th})" and "Number of Positive Foods (95^{th})" for both genders and for both Ulcerative Colitis subjects and control subjects using programs "a_pos_foods.sas, a_pos_foods_by_dx.sas".

Method for measuring diagnostic performance: To measure diagnostic performance for each food items for each subject, we will use data of "Number of Positive Foods (90^{th})" and "Number of Positive Foods (95^{th})" for each subject within each bootstrap replicate described above. In this analysis, the cutpoint was set to 1. Thus, if a subject has one or more "Number of Positive Foods (90^{th})", then the subject will be called "Has Ulcerative Colitis." If a subject has less than one "Number of Positive Foods (90^{th})", then the subject will be called "Does Not Have Ulcerative Colitis." When all calls were made, the calls were compared with actual diagnosis to determine whether a call was a True Positive (TP), True Negative (TN), False Positive (FP), or False Negative (FN). The comparisons will be summarized across subjects to get the performance metrics of sensitivity, specificity, positive predictive value, and negative predictive value for both "Number of Positive Foods (90^{th})" and "Number of Positive Foods(95^{th})" when the cutpoint is set to 1 for each method. Each (sensitivity, 1-specificity) pair becomes a point on the ROC curve for this replicate.

To increase the accuracy, the analysis above will be repeated by incrementing cutpoint from 2 up to 58, and repeated for each of the 1000 bootstrap replicates. Then the performance metrics across the 1000 bootstrap replicates will be summarized by calculating averages using a program "t_pos_foods_by_dx.sas". The results of diagnostic performance for female and male are shown in **Tables 13A and 13B** (90th percentile) and **Tables 14A and 14B** (95th percentile).

**Table 1**

| | | | |
|---|---|---|---|
| Abalone | Cured Cheese | Onion | Walnut, black |
| Adlay | Cuttlefish | Orange | Watermelon |
| Almond | Duck | Oyster | Welch Onion |
| American Cheese | Durian | Papaya | Wheat |
| Apple | Eel | Paprika | Wheat bran |
| Artichoke | Egg White (separate) | Parsley | Yeast (S. cerevisiae) |
| Asparagus | Egg Yolk (separate) | Peach | Yogurt |
| Avocado | Egg, white/yolk (comb.) | Peanut | |
| Baby Bok Choy | Eggplant | Pear | **FOOD ADDITIV ES** |
| Bamboo shoots | Garlic | Pepper, Black | Arabic Gum |
| Banana | Ginger | Pineapple | Carboxymethyl Cellulose |
| Barley, whole grain | Gluten - Gliadin | Pinto bean | Carrageneenan |
| Beef | Goat's milk | Plum | FD&C Blue #1 |
| Beets | Grape, white/concord | Pork | FD&C Red #3 |
| Beta-lactoglobulin | Grapefruit | Potato | FD&C Red #40 |
| Blueberry | Grass Carp | Rabbit | FD&C Yellow #5 |
| Broccoli | Green Onion | Rice | FD&C Yellow #6 |
| Buckwheat | Green pea | Roquefort Cheese | Gelatin |
| Butter | Green pepper | Rye | Guar Gum |
| Cabbage | Guava | Saccharine | Maltodextrin |
| Cane sugar | Hair Tail | Safflower seed | Pectin |
| Cantaloupe | Hake | Salmon | Whey |
| Caraway | Halibut | Sardine | Xanthan Gum |
| Carrot | Hazelnut | Scallop | |
| Casein | Honey | Sesame | |
| Cashew | Kelp | Shark fin | |
| Cauliflower | Kidney bean | Sheep's milk | |
| Celery | Kiwi Fruit | Shrimp | |
| Chard | Lamb | Sole | |
| Cheddar Cheese | Leek | Soybean | |
| Chick Peas | Lemon | Spinach | |
| Chicken | Lentils | Squashes | |
| Chili pepper | Lettuce, Iceberg | Squid | |
| Chocolate | Lima bean | Strawberry | |
| Cinnamon | Lobster | String bean | |
| Clam | Longan | Sunflower seed | |
| Cocoa Bean | Mackerel | Sweet potato | |
| Coconut | Malt | Swiss cheese | |
| Codfish | Mango | Taro | |
| Coffee | Marjoram | Tea, black | |
| Cola nut | Millet | Tobacco | |
| Corn | Mung bean | Tomato | |
| Cottage cheese | Mushroom | Trout | |
| Cow's milk | Mustard seed | Tuna | |
| Crab | Oat | Turkey | |
| Cucumber | Olive | Vanilla | |

### Ranking of Foods according to 2-tailed Permutation T-test p-values with FDR adjustment

**Table 2**

| *Rank* | *Food* | *Raw p-value* | *FOR Multiplicity-adj p-value* |
|---|---|---|---|
| 1 | Green_Pea | 0.0000 | 0.0000 |
| 2 | Cantaloupe | 0.0000 | 0.0009 |
| 3 | Pinto_Bean | 0.0001 | 0.0021 |
| 4 | Cucumber | 0.0001 | 0.0021 |
| 5 | Green_Pepper | 0.0001 | 0.0021 |
| 6 | Grapefruit | 0.0002 | 0.0021 |
| 7 | Carrot | 0.0002 | 0.0021 |
| 8 | Orange | 0.0002 | 0.0021 |
| 9 | Almond | 0.0002 | 0.0021 |
| 10 | Sardine | 0.0003 | 0.0021 |
| 11 | Sweet_Pot_ | 0.0003 | 0.0021 |
| 12 | Broccoli | 0.0003 | 0.0021 |
| 13 | Garlic | 0.0003 | 0.0021 |
| 14 | Lima_Bean | 0.0003 | 0.0021 |
| 15 | Squashes | 0.0004 | 0.0024 |
| 16 | Celery | 0.0004 | 0.0025 |
| 17 | String_Bean | 0.0006 | 0.0030 |
| 18 | Tomato | 0.0008 | 0.0040 |
| 19 | Cauliflower | 0.0009 | 0.0041 |
| 20 | Walnut_Blk | 0.0010 | 0.0046 |
| 21 | Sunflower_Sd | 0.0012 | 0.0051 |
| 22 | Cane_Sugar | 0.0012 | 0.0051 |
| 23 | Buck_Wheat | 0.0028 | 0.0106 |
| 24 | Soybean | 0.0028 | 0.0106 |
| 25 | Lemon | 0.0030 | 0.0108 |
| 26 | Barley | 0.0047 | 0.0163 |
| 27 | Oat | 0.0051 | 0.0170 |
| 28 | Oyster | 0.0055 | 0.0173 |
| 29 | Mustard | 0.0056 | 0.0173 |
| 30 | Rye | 0.0058 | 0.0173 |
| 31 | Peach | 0.0068 | 0.0196 |
| 32 | Chili_Pepper | 0.0072 | 0.0201 |
| 33 | Spinach | 0.0082 | 0.0222 |
| 34 | Peanut | 0.0084 | 0.0222 |
| 35 | Avocado | 0.0088 | 0.0226 |
| 36 | Shrimp | 0.0094 | 0.0236 |
| 37 | Pineapple | 0.0098 | 0.0239 |
| 38 | Cola_Nut | 0.0118 | 0.0275 |
| 39 | Rice | 0.0119 | 0.0275 |
| 40 | Cabbage | 0.0131 | 0.0294 |
| 41 | Butter | 0.0150 | 0.0330 |
| 42 | Eggplant | 0.0156 | 0.0330 |
| 43 | Apple | 0.0158 | 0.0330 |
| 44 | Egg | 0.0176 | 0.0359 |
| 45 | Wheat | 0.0215 | 0.0419 |
| 46 | Cottage_Ch_ | 0.0219 | 0.0419 |
| 47 | Sole | 0.0219 | 0.0419 |
| 48 | Cashew | 0.0238 | 0.0446 |
| 49 | Olive | 0.0259 | 0.0476 |
| 50 | Parsley | 0.0276 | 0.0496 |
| 51 | Corn | 0.0340 | 0.0578 |
| 52 | Honey | 0.0340 | 0.0578 |
| 53 | Chocolate | 0.0345 | 0.0578 |
| 54 | Cow_Milk | 0.0347 | 0.0578 |
| 55 | Potato | 0.0359 | 0.0587 |
| 56 | Onion | 0.0467 | 0.0750 |
| 57 | Tea | 0.0506 | 0.0799 |
| 58 | Tobacco | 0.0625 | 0.0970 |
| 59 | Banana | 0.0706 | 0.1078 |
| 60 | Strawberry | 0.0751 | 0.1127 |
| 61 | Coffee | 0.0771 | 0.1138 |
| 62 | Malt | 0.0823 | 0.1195 |
| 63 | Scallop | 0.0887 | 0.1268 |
| 64 | Chicken | 0.0987 | 0.1388 |
| 65 | Yeast_Baker | 0.1152 | 0.1595 |
| 66 | Millet | 0.1171 | 0.1597 |
| 67 | Swiss_Ch_ | 0.1770 | 0.2378 |
| 68 | Turkey | 0.1806 | 0.2381 |
| 69 | Cheddar_Ch_ | 0.1826 | 0.2381 |
| 70 | Yeast_Brewer | 0.2178 | 0.2801 |
| 71 | Yogurt | 0.2255 | 0.2859 |
| 72 | Cinnamon | 0.2600 | 0.3250 |
| 73 | Clam | 0.2998 | 0.3696 |
| 74 | Tuna | 0.3102 | 0.3762 |
| 75 | Beef | 0.3135 | 0.3762 |
| 76 | Lettuce | 0.3266 | 0.3868 |
| 77 | Trout | 0.3672 | 0.4292 |
| 78 | Safflower | 0.4487 | 0.5178 |
| 79 | Codfish | 0.4712 | 0.5368 |
| 80 | Salmon | 0.5076 | 0.5711 |
| 81 | Mushroom | 0.5634 | 0.6260 |
| 82 | Grape | 0.5825 | 0.6389 |
| 83 | Blueberry | 0.5892 | 0.6389 |
| 84 | Pork | 0.7160 | 0.7667 |
| 85 | Sesame | 0.7241 | 0.7667 |
| 86 | Amer_Cheese | 0.7739 | 0.8099 |
| 87 | Lobster | 0.7946 | 0.8220 |
| 88 | Halibut | 0.8497 | 0.8690 |
| 89 | Goat_Milk | 0.9112 | 0.9215 |
| 90 | Crab | 0.9888 | 0.9888 |

### Basic Descriptive Statistics of ELISA Score by Food and Gender Comparing Ulcerative Colitis to Control

**Table 3**

| *Sex* | *Food* | *Diagnosis* | *ELISA Score* | | | | |
|---|---|---|---|---|---|---|---|
| | | | *N* | *Mean* | *SD* | *Min* | *Max* |
| FEMALE | Almond | Ulcerative_Colitis | 57 | 10.079 | 25.036 | 0.439 | 158.47 |
| | | Control | 66 | 4.034 | 2.187 | 0.100 | 13.068 |
| | | Diff (1-2) | _ | 6.045 | 17.107 | _ | _ |
| | Amer_Cheese | Ulcerative_Colitis | 57 | 21.630 | 31.036 | 1.602 | 140.07 |
| | | Control | 66 | 23.434 | 52.616 | 0.100 | 400.00 |
| | | Diff (1-2) | _ | -1.804 | 43.965 | _ | _ |
| | Apple | Ulcerative_Colitis | 57 | 5.340 | 4.304 | 0.493 | 28.693 |
| | | Control | 66 | 4.432 | 3.291 | 0.100 | 15.890 |
| | | Diff (1-2) | _ | 0.908 | 3.793 | _ | _ |
| | Avocado | Ulcerative_Colitis | 57 | 3.858 | 3.507 | 0.100 | 21.077 |
| | | Control | 66 | 2.930 | 2.339 | 0.100 | 14.256 |
| | | Diff (1-2) | _ | 0.927 | 2.938 | _ | _ |
| | Banana | Ulcerative_Colitis | 57 | 19.827 | 46.868 | 0.100 | 256.94 |
| | | Control | 66 | 8.063 | 14.962 | 0.100 | 83.654 |
| | | Diff (1-2) | _ | 11.765 | 33.717 | _ | _ |
| | Barley | Ulcerative_Colitis | 57 | 25.942 | 30.538 | 1.974 | 165.95 |
| | | Control | 66 | 19.090 | 12.984 | 3.026 | 64.831 |
| | | Diff (1-2) | _ | 6.851 | 22.851 | _ | _ |
| | Beef | Ulcerative_Colitis | 57 | 11.027 | 14.479 | 1.479 | 83.266 |
| | | Control | 66 | 10.288 | 13.960 | 3.026 | 104.76 |
| | | Diff (1-2) | _ | 0.739 | 14.202 | _ | _ |
| | Blueberry | Ulcerative_Colitis | 57 | 5.142 | 3.166 | 1.206 | 17.780 |
| | | Control | 66 | 5.440 | 3.773 | 0.100 | 26.772 |
| | | Diff (1-2) | _ | -0.298 | 3.505 | _ | _ |
| | Broccoli | Ulcerative_Colitis | 57 | 11.435 | 15.944 | 1.355 | 99.132 |
| | | Control | 66 | 6.280 | 5.292 | 0.100 | 36.378 |
| | | Diff (1-2) | _ | 5.154 | 11.520 | _ | _ |
| | Buck_Wheat | Ulcerative_Colitis | 57 | 12.377 | 18.040 | 1.848 | 104.34 |
| | | Control | 66 | 8.034 | 4.990 | 1.316 | 29.397 |
| | | Diff (1-2) | _ | 4.342 | 12.806 | _ | _ |
| | Butter | Ulcerative_Colitis | 57 | 25.891 | 26.436 | 3.865 | 154.85 |
| | | Control | 66 | 21.874 | 29.162 | 0.100 | 204.33 |
| | | Diff (1-2) | _ | 4.017 | 27.933 | _ | _ |
| | Cabbage | Ulcerative_Colitis | 57 | 13.302 | 23.916 | 0.123 | 135.74 |
| | | Control | 66 | 7.362 | 10.123 | 0.100 | 56.932 |
| | | Diff (1-2) | _ | 5.940 | 17.882 | _ | _ |
| | Cane_Sugar | Ulcerative_Colitis | 57 | 32.174 | 30.535 | 8.009 | 178.78 |
| | | Control | 66 | 18.288 | 9.172 | 2.632 | 43.466 |
| | | Diff (1-2) | _ | 13.885 | 21.833 | _ | _ |
| | Cantaloupe | Ulcerative_Colitis | 57 | 12.200 | 20.373 | 0.751 | 149.18 |
| | | Control | 66 | 6.154 | 6.160 | 0.100 | 48.752 |
| | | Diff (1-2) | _ | 6.046 | 14.576 | _ | _ |
| | Carrot | Ulcerative_Colitis | 57 | 6.467 | 6.804 | 0.987 | 47.767 |
| | | Control | 66 | 4.813 | 3.705 | 0.100 | 24.141 |
| | | Diff (1-2) | _ | 1.654 | 5.367 | _ | _ |
| | Cashew | Ulcerative_Colitis | 57 | 12.920 | 21.204 | 0.966 | 98.745 |
| | | Control | 66 | 9.924 | 16.382 | 0.100 | 94.907 |
| | | Diff (1-2) | _ | 2.996 | 18.768 | _ | _ |
| | Cauliflower | Ulcerative_Colitis | 57 | 9.756 | 18.230 | 0.100 | 131.25 |
| | | Control | 66 | 5.977 | 8.336 | 0.100 | 58.808 |
| | | Diff (1-2) | _ | 3.778 | 13.825 | _ | _ |
| | Celery | Ulcerative_Colitis | 57 | 12.601 | 15.076 | 3.080 | 107.65 |
| | | Control | 66 | 9.634 | 5.975 | 0.395 | 32.141 |
| | | Diff (1-2) | _ | 2.967 | 11.152 | _ | _ |
| | Cheddar_Ch_ | Ulcerative_Colitis | 57 | 32.153 | 50.450 | 1.833 | 266.75 |
| | | Control | 66 | 26.852 | 55.697 | 0.100 | 400.00 |
| | | Diff (1-2) | _ | 5.302 | 53.333 | _ | _ |
| | Chicken | Ulcerative_Colitis | 57 | 21.024 | 19.326 | 3.865 | 106.76 |
| | | Control | 66 | 18.303 | 10.514 | 4.743 | 61.887 |
| | | Diff (1-2) | _ | 2.721 | 15.240 | _ | _ |
| | Chili_Pepper | Ulcerative_Colitis | 57 | 9.931 | 9.801 | 1.517 | 56.432 |
| | | Control | 66 | 8.577 | 7.784 | 0.100 | 42.583 |
| | | Diff (1-2) | _ | 1.355 | 8.775 | _ | _ |
| | Chocolate | Ulcerative_Colitis | 57 | 18.043 | 15.319 | 3.510 | 71.901 |
| | | Control | 66 | 14.350 | 6.578 | 3.006 | 35.317 |
| | | Diff (1-2) | _ | 3.693 | 11.483 | _ | _ |
| | Cinnamon | Ulcerative_Colitis | 57 | 34.013 | 22.107 | 5.090 | 119.22 |
| | | Control | 66 | 32.170 | 24.180 | 5.374 | 132.49 |
| | | Diff (1-2) | _ | 1.843 | 23.244 | _ | _ |
| | Clam | Ulcerative_Colitis | 57 | 39.841 | 37.147 | 9.968 | 197.01 |
| | | Control | 66 | 52.166 | 58.253 | 7.819 | 400.00 |
| | | Diff (1-2) | _ | -12.324 | 49.614 | _ | _ |
| | Codfish | Ulcerative_Colitis | 57 | 17.321 | 10.395 | 3.450 | 50.000 |
| | | Control | 66 | 29.652 | 31.720 | 6.200 | 168.28 |
| | | Diff (1-2) | _ | -12.330 | 24.300 | _ | _ |
| | Coffee | Ulcerative_Colitis | 57 | 38.327 | 69.479 | 2.523 | 400.00 |
| | | Control | 66 | 29.631 | 46.880 | 5.215 | 346.81 |
| | | Diff (1-2) | _ | 8.696 | 58.436 | _ | _ |
| | Cola_Nut | Ulcerative_Colitis | 57 | 35.111 | 16.941 | 14.321 | 94.417 |
| | | Control | 66 | 29.138 | 12.588 | 8.723 | 58.129 |
| | | Diff (1-2) | _ | 5.972 | 14.763 | _ | _ |
| | Corn | Ulcerative_Colitis | 57 | 21.320 | 39.276 | 1.426 | 231.14 |
| | | Control | 66 | 11.407 | 23.137 | 0.100 | 187.68 |
| | | Diff (1-2) | _ | 9.913 | 31.646 | _ | _ |
| | Cottage_Ch_ | Ulcerative_Colitis | 57 | 93.700 | 117.494 | 2.594 | 400.00 |
| | | Control | 66 | 76.158 | 92.333 | 0.100 | 400.00 |
| | | Diff (1-2) | _ | 17.543 | 104.732 | _ | _ |
| | Cow_Milk | Ulcerative_Colitis | 57 | 85.720 | 104.244 | 0.682 | 400.00 |
| | | Control | 66 | 75.882 | 86.959 | 0.100 | 400.00 |
| | | Diff (1-2) | _ | 9.838 | 95.349 | _ | _ |
| | Crab | Ulcerative_Colitis | 57 | 19.921 | 13.939 | 4.440 | 70.735 |
| | | Control | 66 | 23.583 | 17.654 | 3.803 | 93.236 |
| | | Diff (1-2) | _ | -3.661 | 16.042 | _ | _ |
| | Cucumber | Ulcerative_Colitis | 57 | 16.195 | 18.948 | 1.232 | 120.91 |
| | | Control | 66 | 8.461 | 8.149 | 0.100 | 38.939 |
| | | Diff (1-2) | _ | 7.735 | 14.207 | _ | _ |
| | Egg | Ulcerative_Colitis | 57 | 85.576 | 122.235 | 2.451 | 400.00 |
| | | Control | 66 | 55.102 | 89.966 | 0.100 | 400.00 |
| | | Diff (1-2) | _ | 30.475 | 106.127 | _ | _ |
| | Eggplant | Ulcerative_Colitis | 57 | 9.361 | 12.488 | 0.100 | 69.989 |
| | | Control | 66 | 5.732 | 5.993 | 0.100 | 31.330 |
| | | Diff (1-2) | _ | 3.628 | 9.564 | _ | _ |
| | Garlic | Ulcerative_Colitis | 57 | 20.485 | 17.805 | 2.413 | 90.456 |
| | | Control | 66 | 11.174 | 5.779 | 3.380 | 28.482 |
| | | Diff (1-2) | _ | 9.310 | 12.832 | _ | _ |
| | Goat_Milk | Ulcerative_Colitis | 57 | 13.970 | 15.091 | 1.146 | 78.345 |
| | | Control | 66 | 15.413 | 28.452 | 0.100 | 180.08 |
| | | Diff (1-2) | _ | -1.443 | 23.243 | _ | _ |
| | Grape | Ulcerative_Colitis | 57 | 20.135 | 11.537 | 4.169 | 78.950 |
| | | Control | 66 | 20.276 | 6.827 | 10.650 | 47.817 |
| | | Diff (1-2) | _ | -0.141 | 9.308 | _ | _ |
| | Grapefruit | Ulcerative_Colitis | 57 | 5.675 | 9.301 | 0.100 | 68.905 |
| | | Control | 66 | 3.278 | 2.446 | 0.100 | 14.364 |
| | | Diff (1-2) | _ | 2.397 | 6.576 | _ | _ |
| | Green_Pea | Ulcerative_Colitis | 57 | 15.251 | 15.940 | 0.658 | 79.774 |
| | | Control | 66 | 8.631 | 7.160 | 0.496 | 32.502 |
| | | Diff (1-2) | _ | 6.620 | 12.047 | _ | _ |
| | Green_Pepper | Ulcerative_Colitis | 57 | 7.641 | 14.196 | 0.100 | 107.26 |
| | | Control | 66 | 4.149 | 2.875 | 0.100 | 14.364 |
| | | Diff (1-2) | _ | 3.492 | 9.885 | _ | _ |
| | Halibut | Ulcerative_Colitis | 57 | 10.765 | 5.076 | 2.587 | 27.746 |
| | | Control | 66 | 11.119 | 7.129 | 2.729 | 44.884 |
| | | Diff (1-2) | _ | -0.354 | 6.263 | _ | _ |
| | Honey | Ulcerative_Colitis | 57 | 12.330 | 7.625 | 2.742 | 37.290 |
| | | Control | 66 | 10.185 | 4.203 | 4.227 | 19.876 |
| | | Diff (1-2) | _ | 2.145 | 6.033 | _ | _ |
| | Lemon | Ulcerative_Colitis | 57 | 3.296 | 3.105 | 0.100 | 22.003 |
| | | Control | 66 | 2.482 | 2.159 | 0.100 | 14.688 |
| | | Diff (1-2) | _ | 0.814 | 2.639 | _ | _ |
| | Lettuce | Ulcerative_Colitis | 57 | 11.835 | 9.147 | 2.711 | 59.964 |
| | | Control | 66 | 11.368 | 6.472 | 0.921 | 29.851 |
| | | Diff (1-2) | _ | 0.467 | 7.825 | _ | _ |
| | Lima_Bean | Ulcerative_Colitis | 57 | 10.268 | 8.919 | 0.329 | 39.575 |
| | | Control | 66 | 6.624 | 8.761 | 0.100 | 65.634 |
| | | Diff (1-2) | _ | 3.643 | 8.835 | _ | _ |
| | Lobster | Ulcerative_Colitis | 57 | 12.931 | 10.997 | 1.181 | 62.481 |
| | | Control | 66 | 13.398 | 8.359 | 3.938 | 46.560 |
| | | Diff (1-2) | _ | -0.468 | 9.670 | _ | _ |
| | Malt | Ulcerative_Colitis | 57 | 23.676 | 17.406 | 5.814 | 105.68 |
| | | Control | 66 | 21.743 | 11.326 | 3.684 | 57.151 |
| | | Diff (1-2) | _ | 1.933 | 14.461 | _ | _ |
| | Millet | Ulcerative_Colitis | 57 | 5.424 | 5.233 | 0.487 | 27.187 |
| | | Control | 66 | 4.889 | 7.091 | 0.100 | 46.663 |
| | | Diff (1-2) | _ | 0.535 | 6.299 | _ | _ |
| | Mushroom | Ulcerative_Colitis | 57 | 9.754 | 12.339 | 0.100 | 69.107 |
| | | Control | 66 | 13.174 | 12.549 | 1.117 | 49.656 |
| | | Diff (1-2) | _ | -3.419 | 12.452 | _ | _ |
| | Mustard | Ulcerative_Colitis | 57 | 11.854 | 15.378 | 2.545 | 98.146 |
| | | Control | 66 | 8.842 | 5.224 | 0.100 | 23.452 |
| | | Diff (1-2) | _ | 3.011 | 11.140 | _ | _ |
| | Oat | Ulcerative_Colitis | 57 | 40.965 | 76.954 | 0.768 | 400.00 |
| | | Control | 66 | 16.237 | 14.506 | 0.100 | 76.165 |
| | | Diff (1-2) | _ | 24.727 | 53.421 | _ | _ |
| | Olive | Ulcerative_Colitis | 57 | 31.615 | 30.330 | 3.573 | 180.11 |
| | | Control | 66 | 23.704 | 14.281 | 5.272 | 59.488 |
| | | Diff (1-2) | _ | 7.911 | 23.137 | _ | _ |
| | Onion | Ulcerative_Colitis | 57 | 17.905 | 24.231 | 0.438 | 119.13 |
| | | Control | 66 | 11.329 | 16.935 | 1.184 | 114.37 |
| | | Diff (1-2) | _ | 6.576 | 20.635 | _ | _ |
| | Orange | Ulcerative_Colitis | 57 | 26.028 | 25.192 | 1.206 | 112.32 |
| | | Control | 66 | 15.289 | 11.608 | 1.489 | 47.125 |
| | | Diff (1-2) | _ | 10.738 | 19.134 | _ | _ |
| | Oyster | Ulcerative_Colitis | 57 | 63.062 | 63.526 | 4.608 | 372.89 |
| | | Control | 66 | 42.674 | 33.485 | 5.656 | 168.59 |
| | | Diff (1-2) | _ | 20.388 | 49.699 | _ | _ |
| | Parsley | Ulcerative_Colitis | 57 | 6.938 | 11.992 | 0.100 | 70.169 |
| | | Control | 66 | 5.005 | 6.541 | 0.100 | 34.932 |
| | | Diff (1-2) | _ | 1.933 | 9.462 | _ | _ |
| | Peach | Ulcerative_Colitis | 57 | 13.457 | 20.732 | 0.123 | 124.35 |
| | | Control | 66 | 7.145 | 7.742 | 0.100 | 33.820 |
| | | Diff (1-2) | _ | 6.312 | 15.203 | _ | _ |
| | Peanut | Ulcerative_Colitis | 57 | 14.262 | 48.433 | 0.219 | 349.73 |
| | | Control | 66 | 5.563 | 4.941 | 0.100 | 26.567 |
| | | Diff (1-2) | _ | 8.699 | 33.147 | _ | _ |
| | Pineapple | Ulcerative_Colitis | 57 | 53.335 | 86.808 | 0.329 | 400.00 |
| | | Control | 66 | 23.710 | 46.114 | 0.100 | 278.44 |
| | | Diff (1-2) | _ | 29.626 | 68.044 | _ | _ |
| | Pinto_Bean | Ulcerative_Colitis | 57 | 16.597 | 22.820 | 2.254 | 152.98 |
| | | Control | 66 | 10.138 | 8.167 | 0.100 | 48.623 |
| | | Diff (1-2) | _ | 6.459 | 16.639 | _ | _ |
| | Pork | Ulcerative_Colitis | 57 | 15.004 | 15.800 | 2.962 | 80.448 |
| | | Control | 66 | 15.347 | 10.345 | 4.339 | 65.759 |
| | | Diff (1-2) | _ | -0.343 | 13.154 | _ | _ |
| | Potato | Ulcerative_Colitis | 57 | 17.934 | 24.208 | 4.278 | 183.78 |
| | | Control | 66 | 13.615 | 6.063 | 6.200 | 40.802 |
| | | Diff (1-2) | _ | 4.318 | 17.058 | _ | _ |
| | Rice | Ulcerative_Colitis | 57 | 31.549 | 49.019 | 6.184 | 362.21 |
| | | Control | 66 | 21.551 | 16.950 | 3.350 | 92.642 |
| | | Diff (1-2) | _ | 9.998 | 35.587 | _ | _ |
| | Rye | Ulcerative_Colitis | 57 | 6.931 | 12.152 | 1.338 | 92.310 |
| | | Control | 66 | 5.237 | 3.633 | 0.100 | 22.824 |
| | | Diff (1-2) | _ | 1.694 | 8.685 | _ | _ |
| | Safflower | Ulcerative_Colitis | 57 | 8.917 | 6.880 | 2.531 | 41.242 |
| | | Control | 66 | 8.776 | 8.189 | 1.722 | 48.833 |
| | | Diff (1-2) | _ | 0.140 | 7.611 | _ | _ |
| | Salmon | Ulcerative_Colitis | 57 | 9.369 | 6.906 | 2.413 | 44.560 |
| | | Control | 66 | 9.377 | 7.261 | 2.862 | 56.530 |
| | | Diff (1-2) | _ | -0.008 | 7.099 | _ | _ |
| | Sardine | Ulcerative_Colitis | 57 | 44.148 | 20.802 | 12.069 | 102.96 |
| | | Control | 66 | 37.084 | 16.695 | 7.190 | 88.964 |
| | | Diff (1-2) | _ | 7.064 | 18.708 | _ | _ |
| | Scallop | Ulcerative_Colitis | 57 | 61.726 | 39.681 | 14.451 | 165.26 |
| | | Control | 66 | 64.291 | 29.551 | 18.605 | 148.58 |
| | | Diff (1-2) | _ | -2.565 | 34.610 | _ | _ |
| | Sesame | Ulcerative_Colitis | 57 | 73.122 | 118.220 | 0.100 | 400.00 |
| | | Control | 66 | 80.704 | 93.902 | 5.984 | 400.00 |
| | | Diff (1-2) | _ | -7.582 | 105.854 | _ | _ |
| | Shrimp | Ulcerative_Colitis | 57 | 21.492 | 22.231 | 1.717 | 137.49 |
| | | Control | 66 | 33.150 | 27.875 | 6.607 | 113.66 |
| | | Diff (1-2) | _ | -11.658 | 25.419 | _ | _ |
| | Sole | Ulcerative_Colitis | 57 | 6.020 | 3.293 | 1.316 | 20.885 |
| | | Control | 66 | 6.440 | 6.960 | 0.100 | 54.883 |
| | | Diff (1-2) | _ | -0.419 | 5.571 | _ | _ |
| | Soybean | Ulcerative_Colitis | 57 | 21.445 | 26.605 | 4.187 | 187.77 |
| | | Control | 66 | 15.294 | 9.373 | 2.481 | 49.071 |
| | | Diff (1-2) | _ | 6.151 | 19.360 | _ | _ |
| | Spinach | Ulcerative_Colitis | 57 | 26.961 | 49.539 | 6.802 | 367.99 |
| | | Control | 66 | 20.485 | 13.172 | 6.051 | 66.626 |
| | | Diff (1-2) | _ | 6.476 | 35.057 | _ | _ |
| | Squashes | Ulcerative_Colitis | 57 | 17.555 | 11.532 | 4.059 | 53.553 |
| | | Control | 66 | 13.415 | 11.597 | 1.842 | 74.279 |
| | | Diff (1-2) | _ | 4.140 | 11.567 | _ | _ |
| | Strawberry | Ulcerative_Colitis | 57 | 6.064 | 5.341 | 0.100 | 28.233 |
| | | Control | 66 | 5.563 | 5.305 | 0.100 | 35.745 |
| | | Diff (1-2) | _ | 0.501 | 5.321 | _ | _ |
| | String_Bean | Ulcerative_Colitis | 57 | 54.019 | 30.799 | 7.680 | 149.68 |
| | | Control | 66 | 41.957 | 22.678 | 9.539 | 125.69 |
| | | Diff (1-2) | _ | 12.063 | 26.744 | _ | _ |
| | Sunflower_Sd | Ulcerative_Colitis | 57 | 15.717 | 21.185 | 2.084 | 103.84 |
| | | Control | 66 | 9.948 | 6.094 | 2.632 | 33.347 |
| | | Diff (1-2) | _ | 5.769 | 15.089 | _ | _ |
| | Sweet_Pot_ | Ulcerative_Colitis | 57 | 13.118 | 18.306 | 2.218 | 138.11 |
| | | Control | 66 | 8.592 | 4.479 | 0.395 | 25.009 |
| | | Diff (1-2) | _ | 4.525 | 12.879 | _ | _ |
| | Swiss_Ch_ | Ulcerative_Colitis | 57 | 49.090 | 77.461 | 2.316 | 400.00 |
| | | Control | 66 | 39.219 | 73.725 | 0.100 | 400.00 |
| | | Diff (1-2) | _ | 9.871 | 75.477 | _ | _ |
| | Tea | Ulcerative_Colitis | 57 | 35.381 | 24.818 | 12.508 | 160.22 |
| | | Control | 66 | 29.771 | 12.014 | 11.634 | 64.535 |
| | | Diff (1-2) | _ | 5.610 | 19.042 | _ | _ |
| | Tobacco | Ulcerative_Colitis | 57 | 39.527 | 26.849 | 10.906 | 135.98 |
| | | Control | 66 | 33.566 | 16.789 | 7.809 | 82.097 |
| | | Diff (1-2) | _ | 5.961 | 22.024 | _ | _ |
| | Tomato | Ulcerative_Colitis | 57 | 15.238 | 16.813 | 2.218 | 107.39 |
| | | Control | 66 | 9.066 | 7.694 | 0.100 | 42.078 |
| | | Diff (1-2) | _ | 6.172 | 12.753 | _ | _ |
| | Trout | Ulcerative_Colitis | 57 | 13.805 | 8.087 | 3.749 | 47.896 |
| | | Control | 66 | 16.138 | 10.667 | 5.596 | 76.221 |
| | | Diff (1-2) | _ | -2.333 | 9.560 | _ | _ |
| | Tuna | Ulcerative_Colitis | 57 | 15.838 | 10.358 | 2.254 | 56.001 |
| | | Control | 66 | 18.092 | 12.707 | 3.873 | 64.090 |
| | | Diff (1-2) | _ | -2.253 | 11.679 | _ | _ |
| | Turkey | Ulcerative_Colitis | 57 | 16.023 | 14.275 | 3.006 | 95.919 |
| | | Control | 66 | 14.461 | 6.976 | 4.094 | 32.151 |
| | | Diff (1-2) | _ | 1.561 | 10.975 | _ | _ |
| | Walnut_Blk | Ulcerative_Colitis | 57 | 40.389 | 58.256 | 8.009 | 400.00 |
| | | Control | 66 | 25.386 | 17.254 | 6.943 | 117.46 |
| | | Diff (1-2) | _ | 15.003 | 41.601 | _ | _ |
| | Wheat | Ulcerative_Colitis | 57 | 25.837 | 67.552 | 2.304 | 400.00 |
| | | Control | 66 | 18.402 | 29.364 | 0.790 | 209.95 |
| | | Diff (1-2) | _ | 7.435 | 50.746 | _ | _ |
| | Yeast_Baker | Ulcerative_Colitis | 57 | 12.519 | 30.904 | 1.316 | 223.99 |
| | | Control | 66 | 5.545 | 3.349 | 0.526 | 18.811 |
| | | Diff (1-2) | _ | 6.974 | 21.167 | _ | _ |
| | Yeast_Brewer | Ulcerative_Colitis | 57 | 25.350 | 61.479 | 2.194 | 400.00 |
| | | Control | 66 | 10.847 | 7.818 | 0.100 | 43.887 |
| | | Diff (1-2) | _ | 14.503 | 42.215 | _ | _ |
| | Yogurt | Ulcerative_Colitis | 57 | 21.430 | 20.338 | 4.240 | 101.82 |
| | | Control | 66 | 22.930 | 30.973 | 0.100 | 215.73 |
| | | Diff (1-2) | _ | -1.500 | 26.585 | _ | _ |
| MALE | Almond | Ulcerative_Colitis | 46 | 9.713 | 10.631 | 0.100 | 48.413 |
| | | Control | 97 | 4.049 | 2.231 | 0.100 | 12.591 |
| | | Diff (1-2) | _ | 5.664 | 6.282 | _ | _ |
| | Amer_Cheese | Ulcerative_Colitis | 46 | 27.588 | 27.243 | 0.100 | 105.40 |
| | | Control | 97 | 22.619 | 34.069 | 0.468 | 197.38 |
| | | Diff (1-2) | _ | 4.969 | 32.049 | _ | _ |
| | Apple | Ulcerative_Colitis | 46 | 5.840 | 4.036 | 0.100 | 20.284 |
| | | Control | 97 | 4.383 | 2.900 | 0.100 | 13.795 |
| | | Diff (1-2) | _ | 1.457 | 3.305 | _ | _ |
| | Avocado | Ulcerative_Colitis | 46 | 3.569 | 2.010 | 0.100 | 11.275 |
| | | Control | 97 | 2.720 | 2.992 | 0.100 | 28.693 |
| | | Diff (1-2) | _ | 0.849 | 2.717 | _ | _ |
| | Banana | Ulcerative_Colitis | 46 | 11.987 | 18.952 | 0.100 | 96.512 |
| | | Control | 97 | 8.576 | 36.151 | 0.100 | 350.69 |
| | | Diff (1-2) | _ | 3.411 | 31.693 | _ | _ |
| | Barley | Ulcerative_Colitis | 46 | 37.135 | 58.378 | 0.100 | 400.00 |
| | | Control | 97 | 19.214 | 11.923 | 4.612 | 58.865 |
| | | Diff (1-2) | _ | 17.921 | 34.416 | _ | _ |
| | Beef | Ulcerative_Colitis | 46 | 12.163 | 15.192 | 0.100 | 89.210 |
| | | Control | 97 | 9.327 | 11.981 | 2.059 | 93.494 |
| | | Diff (1-2) | _ | 2.836 | 13.092 | _ | _ |
| | Blueberry | Ulcerative_Colitis | 46 | 6.305 | 4.453 | 0.100 | 26.859 |
| | | Control | 97 | 5.393 | 2.868 | 0.100 | 19.410 |
| | | Diff (1-2) | _ | 0.911 | 3.454 | _ | _ |
| | Broccoli | Ulcerative_Colitis | 46 | 10.771 | 6.468 | 0.100 | 29.342 |
| | | Control | 97 | 6.790 | 8.012 | 0.131 | 72.543 |
| | | Diff (1-2) | _ | 3.981 | 7.554 | _ | _ |
| | Buck_Wheat | Ulcerative_Colitis | 46 | 9.904 | 5.030 | 0.100 | 23.189 |
| | | Control | 97 | 6.978 | 3.384 | 2.656 | 24.338 |
| | | Diff (1-2) | _ | 2.926 | 3.984 | _ | _ |
| | Butter | Ulcerative_Colitis | 46 | 28.310 | 23.146 | 2.104 | 87.745 |
| | | Control | 97 | 17.846 | 20.091 | | 1.490 131.60 |
| | | Diff (1-2) | _ | 10.464 | 21.114 | _ | _ |
| | Cabbage | Ulcerative_Colitis | 46 | 11.079 | 9.922 | 0.100 | 41.324 |
| | | Control | 97 | 6.540 | 18.133 | 0.100 | 174.96 |
| | | Diff (1-2) | _ | 4.539 | 15.977 | _ | _ |
| | Cane_Sugar | Ulcerative_Colitis | 46 | 28.481 | 24.975 | 2.955 | 147.61 |
| | | Control | 97 | 22.356 | 18.718 | 2.789 | 100.82 |
| | | Diff (1-2) | _ | 6.125 | 20.919 | _ | _ |
| | Cantaloupe | Ulcerative_Colitis | 46 | 12.177 | 10.882 | 0.100 | 60.013 |
| | | Control | 97 | 6.052 | 5.569 | 0.468 | 38.706 |
| | | Diff (1-2) | _ | 6.126 | 7.675 | _ | _ |
| | Carrot | Ulcerative_Colitis | 46 | 9.182 | 8.539 | 0.100 | 50.970 |
| | | Control | 97 | 4.684 | 3.636 | 0.468 | 28.593 |
| | | Diff (1-2) | _ | 4.498 | 5.681 | _ | _ |
| | Cashew | Ulcerative_Colitis | 46 | 17.599 | 28.317 | 0.100 | 167.72 |
| | | Control | 97 | 8.362 | 10.271 | 0.100 | 55.749 |
| | | Diff (1-2) | _ | 9.237 | 18.103 | _ | _ |
| | Cauliflower | Ulcerative_Colitis | 46 | 9.803 | 9.337 | 0.100 | 42.378 |
| | | Control | 97 | 4.385 | 4.396 | 0.100 | 36.593 |
| | | Diff (1-2) | _ | 5.418 | 6.402 | _ | _ |
| | Celery | Ulcerative_Colitis | 46 | 16.290 | 11.968 | 0.100 | 52.534 |
| | | Control | 97 | 8.930 | 4.985 | 2.394 | 26.982 |
| | | Diff (1-2) | _ | 7.360 | 7.914 | _ | _ |
| | Cheddar_Ch_ | Ulcerative_Colitis | 46 | 41.438 | 45.998 | 0.100 | 208.47 |
| | | Control | 97 | 28.479 | 49.022 | 1.169 | 298.91 |
| | | Diff (1-2) | _ | 12.959 | 48.077 | _ | _ |
| | Chicken | Ulcerative_Colitis | 46 | 21.425 | 15.312 | 0.100 | 71.379 |
| | | Control | 97 | 17.778 | 11.456 | 5.137 | 69.503 |
| | | Diff (1-2) | _ | 3.646 | 12.813 | _ | _ |
| | Chili_Pepper | Ulcerative_Colitis | 46 | 13.087 | 11.692 | 0.100 | 61.496 |
| | | Control | 97 | 7.802 | 5.945 | 1.591 | 31.070 |
| | | Diff (1-2) | _ | 5.286 | 8.227 | _ | _ |
| | Chocolate | Ulcerative_Colitis | 46 | 20.511 | 13.811 | 0.100 | 69.232 |
| | | Control | 97 | 16.536 | 11.276 | 1.726 | 63.673 |
| | | Diff (1-2) | _ | 3.975 | 12.143 | _ | _ |
| | Cinnamon | Ulcerative_Colitis | 46 | 43.331 | 30.200 | 7.718 | 117.58 |
| | | Control | 97 | 35.928 | 28.520 | 3.136 | 146.95 |
| | | Diff (1-2) | _ | 7.403 | 29.067 | _ | _ |
| | Clam | Ulcerative_Colitis | 46 | 38.009 | 28.872 | 3.421 | 121.47 |
| | | Control | 97 | 38.293 | 21.598 | 6.370 | 103.47 |
| | | Diff (1-2) | _ | -0.284 | 24.159 | _ | _ |
| | Codfish | Ulcerative_Colitis | 46 | 26.039 | 20.205 | 0.100 | 86.059 |
| | | Control | 97 | 22.538 | 29.644 | 4.176 | 269.16 |
| | | Diff (1-2) | _ | 3.501 | 26.992 | _ | _ |
| | Coffee | Ulcerative_Colitis | 46 | 34.715 | 62.443 | 3.884 | 400.00 |
| | | Control | 97 | 20.037 | 24.002 | 2.705 | 192.24 |
| | | Diff (1-2) | _ | 14.679 | 40.455 | _ | _ |
| | Cola_Nut | Ulcerative_Colitis | 46 | 38.888 | 16.023 | 11.891 | 84.315 |
| | | Control | 97 | 32.919 | 20.025 | 3.851 | 112.10 |
| | | Diff (1-2) | _ | 5.969 | 18.840 | _ | _ |
| | Corn | Ulcerative_Colitis | 46 | 13.329 | 9.353 | 0.100 | 53.955 |
| | | Control | 97 | 10.126 | 15.048 | | 1.520 117.90 |
| | | Diff (1-2) | _ | 3.203 | 13.494 | _ | _ |
| | Cottage_Ch_ | Ulcerative_Colitis | 46 | 127.105 | 127.624 | 1.867 | 400.00 |
| | | Control | 97 | 74.814 | 101.386 | 1.446 | 400.00 |
| | | Diff (1-2) | _ | 52.292 | 110.439 | _ | _ |
| | Cow_Milk | Ulcerative_Colitis | 46 | 115.427 | 111.909 | 2.595 | 400.00 |
| | | Control | 97 | 68.606 | 94.032 | 1.343 | 400.00 |
| | | Diff (1-2) | _ | 46.821 | 100.085 | _ | _ |
| | Crab | Ulcerative_Colitis | 46 | 29.571 | 61.851 | 2.104 | 400.00 |
| | | Control | 97 | 24.550 | 29.311 | 3.108 | 252.41 |
| | | Diff (1-2) | _ | 5.021 | 42.496 | _ | _ |
| | Cucumber | Ulcerative_Colitis | 46 | 13.314 | 9.189 | 0.100 | 39.378 |
| | | Control | 97 | 8.320 | 9.298 | 0.234 | 69.188 |
| | | Diff (1-2) | _ | 4.994 | 9.263 | _ | _ |
| | Egg | Ulcerative_Colitis | 46 | 71.044 | 98.867 | 0.935 | 400.00 |
| | | Control | 97 | 44.335 | 66.828 | 0.100 | 400.00 |
| | | Diff (1-2) | _ | 26.709 | 78.487 | _ | _ |
| | Eggplant | Ulcerative_Colitis | 46 | 8.891 | 11.349 | 0.100 | 74.721 |
| | | Control | 97 | 5.856 | 10.455 | 0.100 | 92.376 |
| | | Diff (1-2) | _ | 3.035 | 10.749 | _ | _ |
| | Garlic | Ulcerative_Colitis | 46 | 17.749 | 14.628 | 0.100 | 72.515 |
| | | Control | 97 | 13.476 | 12.122 | 3.097 | 70.591 |
| | | Diff (1-2) | _ | 4.274 | 12.975 | _ | _ |
| | Goat_Milk | Ulcerative_Colitis | 46 | 21.482 | 21.250 | 0.100 | 81.830 |
| | | Control | 97 | 17.999 | 36.202 | 0.100 | 275.19 |
| | | Diff (1-2) | _ | 3.483 | 32.194 | _ | _ |
| | Grape | Ulcerative_Colitis | 46 | 22.888 | 11.749 | 0.100 | 71.188 |
| | | Control | 97 | 23.308 | 7.422 | 11.900 | 41.654 |
| | | Diff (1-2) | _ | -0.420 | 9.031 | _ | _ |
| | Grapefruit | Ulcerative_Colitis | 46 | 5.464 | 4.181 | 0.100 | 20.502 |
| | | Control | 97 | 3.049 | 2.306 | 0.100 | 14.648 |
| | | Diff (1-2) | _ | 2.415 | 3.033 | _ | _ |
| | Green_Pea | Ulcerative_Colitis | 46 | 19.698 | 18.404 | 0.100 | 78.678 |
| | | Control | 97 | 9.229 | 11.366 | 0.100 | 71.765 |
| | | Diff (1-2) | _ | 10.469 | 14.002 | _ | _ |
| | Green_Pepper | Ulcerative_Colitis | 46 | 7.397 | 6.122 | 0.100 | 27.348 |
| | | Control | 97 | 3.972 | 2.664 | 0.100 | 15.744 |
| | | Diff (1-2) | _ | 3.425 | 4.098 | _ | _ |
| | Halibut | Ulcerative_Colitis | 46 | 14.268 | 13.472 | 0.100 | 81.343 |
| | | Control | 97 | 12.657 | 15.451 | 0.818 | 142.09 |
| | | Diff (1-2) | _ | 1.611 | 14.848 | _ | _ |
| | Honey | Ulcerative_Colitis | 46 | 12.703 | 6.605 | 0.100 | 33.490 |
| | | Control | 97 | 11.082 | 6.215 | 2.434 | 31.202 |
| | | Diff (1-2) | _ | 1.620 | 6.343 | _ | _ |
| | Lemon | Ulcerative_Colitis | 46 | 3.113 | 1.709 | 0.100 | 7.749 |
| | | Control | 97 | 2.310 | 1.436 | 0.100 | 8.383 |
| | | Diff (1-2) | _ | 0.803 | 1.528 | _ | _ |
| | Lettuce | Ulcerative_Colitis | 46 | 12.892 | 7.188 | 0.100 | 29.846 |
| | | Control | 97 | 11.271 | 8.295 | 2.871 | 52.209 |
| | | Diff (1-2) | _ | 1.621 | 7.958 | _ | _ |
| | Lima_Bean | Ulcerative_Colitis | 46 | 8.928 | 5.835 | 0.100 | 29.759 |
| | | Control | 97 | 5.994 | 5.650 | 0.100 | 37.640 |
| | | Diff (1-2) | _ | 2.934 | 5.710 | _ | _ |
| | Lobster | Ulcerative_Colitis | 46 | 11.944 | 7.361 | 0.117 | 37.739 |
| | | Control | 97 | 15.678 | 11.555 | 0.468 | 61.064 |
| | | Diff (1-2) | _ | -3.734 | 10.402 | _ | _ |
| | Malt | Ulcerative_Colitis | 46 | 26.092 | 17.394 | 0.100 | 105.54 |
| | | Control | 97 | 21.137 | 12.373 | 3.182 | 58.638 |
| | | Diff (1-2) | _ | 4.955 | 14.170 | _ | _ |
| | Millet | Ulcerative_Colitis | 46 | 5.919 | 7.006 | 0.100 | 42.933 |
| | | Control | 97 | 4.006 | 6.783 | 0.100 | 67.831 |
| | | Diff (1-2) | _ | 1.913 | 6.855 | _ | _ |
| | Mushroom | Ulcerative_Colitis | 46 | 14.755 | 16.831 | 0.100 | 68.603 |
| | | Control | 97 | 12.883 | 12.397 | 1.350 | 59.949 |
| | | Diff (1-2) | _ | 1.873 | 13.966 | _ | _ |
| | Mustard | Ulcerative_Colitis | 46 | 17.526 | 26.970 | 1.089 | 183.13 |
| | | Control | 97 | 9.168 | 5.413 | 1.044 | 28.538 |
| | | Diff (1-2) | _ | 8.358 | 15.878 | _ | _ |
| | Oat | Ulcerative_Colitis | 46 | 29.789 | 33.374 | 0.100 | 193.73 |
| | | Control | 97 | 20.964 | 22.946 | 1.461 | 107.25 |
| | | Diff (1-2) | _ | 8.825 | 26.720 | _ | _ |
| | Olive | Ulcerative_Colitis | 46 | 30.506 | 20.247 | 0.139 | 118.07 |
| | | Control | 97 | 24.794 | 22.708 | 5.137 | 160.63 |
| | | Diff (1-2) | _ | 5.711 | 21.952 | _ | _ |
| | Onion | Ulcerative_Colitis | 46 | 14.182 | 12.107 | 0.100 | 50.545 |
| | | Control | 97 | 11.600 | 17.551 | 1.175 | 158.57 |
| | | Diff (1-2) | _ | 2.583 | 16.016 | _ | _ |
| | Orange | Ulcerative_Colitis | 46 | 28.800 | 21.379 | 0.100 | 110.43 |
| | | Control | 97 | 17.767 | 16.361 | 2.146 | 79.419 |
| | | Diff (1-2) | _ | 11.034 | 18.114 | _ | _ |
| | Oyster | Ulcerative_Colitis | 46 | 63.323 | 74.746 | 6.369 | 357.39 |
| | | Control | 97 | 43.016 | 35.689 | 5.069 | 216.58 |
| | | Diff (1-2) | _ | 20.306 | 51.481 | _ | _ |
| | Parsley | Ulcerative_Colitis | 46 | 9.862 | 16.304 | 0.100 | 74.199 |
| | | Control | 97 | 4.867 | 7.352 | 0.100 | 58.674 |
| | | Diff (1-2) | _ | 4.995 | 11.029 | _ | _ |
| | Peach | Ulcerative_Colitis | 46 | 16.604 | 35.101 | 0.100 | 236.47 |
| | | Control | 97 | 8.390 | 8.373 | 0.100 | 50.444 |
| | | Diff (1-2) | _ | 8.214 | 20.999 | _ | _ |
| | Peanut | Ulcerative_Colitis | 46 | 8.452 | 9.914 | 0.100 | 51.491 |
| | | Control | 97 | 4.241 | 4.514 | 0.855 | 41.070 |
| | | Diff (1-2) | _ | 4.211 | 6.726 | _ | _ |
| | Pineapple | Ulcerative_Colitis | 46 | 34.321 | 47.506 | 0.100 | 207.41 |
| | | Control | 97 | 23.259 | 48.769 | 0.100 | 400.00 |
| | | Diff (1-2) | _ | 11.061 | 48.370 | _ | _ |
| | Pinto_Bean | Ulcerative_Colitis | 46 | 14.680 | 10.767 | 0.100 | 49.004 |
| | | Control | 97 | 8.132 | 5.524 | 0.664 | 28.288 |
| | | Diff (1-2) | _ | 6.548 | 7.601 | _ | _ |
| | Pork | Ulcerative_Colitis | 46 | 14.508 | 12.409 | 0.100 | 73.385 |
| | | Control | 97 | 13.403 | 10.218 | 1.637 | 57.274 |
| | | Diff (1-2) | _ | 1.106 | 10.965 | _ | _ |
| | Potato | Ulcerative_Colitis | 46 | 18.153 | 11.266 | 0.100 | 55.737 |
| | | Control | 97 | 14.555 | 5.951 | 5.259 | 49.002 |
| | | Diff (1-2) | _ | 3.598 | 8.039 | _ | _ |
| | Rice | Ulcerative_Colitis | 46 | 43.673 | 60.315 | 1.867 | 400.00 |
| | | Control | 97 | 25.220 | 18.948 | 5.149 | 118.12 |
| | | Diff (1-2) | _ | 18.453 | 37.490 | _ | _ |
| | Rye | Ulcerative_Colitis | 46 | 11.156 | 18.678 | 0.100 | 113.72 |
| | | Control | 97 | 4.801 | 2.690 | 0.653 | 15.288 |
| | | Diff (1-2) | _ | 6.355 | 10.783 | _ | _ |
| | Safflower | Ulcerative_Colitis | 46 | 9.950 | 6.790 | 0.100 | 33.143 |
| | | Control | 97 | 8.672 | 6.177 | 1.958 | 38.914 |
| | | Diff (1-2) | _ | 1.278 | 6.379 | _ | _ |
| | Salmon | Ulcerative_Colitis | 46 | 9.627 | 5.825 | 0.100 | 28.441 |
| | | Control | 97 | 10.920 | 13.350 | 0.100 | 125.74 |
| | | Diff (1-2) | _ | -1.293 | 11.496 | _ | _ |
| | Sardine | Ulcerative_Colitis | 46 | 48.386 | 21.967 | 10.375 | 121.32 |
| | | Control | 97 | 37.035 | 15.979 | 7.037 | 90.406 |
| | | Diff (1-2) | _ | 11.351 | 18.106 | _ | _ |
| | Scallop | Ulcerative_Colitis | 46 | 81.379 | 44.060 | 12.717 | 186.86 |
| | | Control | 97 | 60.721 | 32.618 | 8.942 | 167.75 |
| | | Diff (1-2) | _ | 20.658 | 36.660 | _ | _ |
| | Sesame | Ulcerative_Colitis | 46 | 72.997 | 95.118 | 0.100 | 400.00 |
| | | Control | 97 | 60.406 | 79.861 | 2.115 | 400.00 |
| | | Diff (1-2) | _ | 12.592 | 85.028 | _ | _ |
| | Shrimp | Ulcerative_Colitis | 46 | 22.090 | 14.510 | 2.955 | 63.471 |
| | | Control | 97 | 34.490 | 42.689 | 2.663 | 342.67 |
| | | Diff (1-2) | _ | -12.400 | 36.165 | _ | _ |
| | Sole | Ulcerative_Colitis | 46 | 7.515 | 4.149 | 0.100 | 20.953 |
| | | Control | 97 | 4.912 | 2.238 | 0.100 | 14.303 |
| | | Diff (1-2) | _ | 2.603 | 2.984 | _ | _ |
| | Soybean | Ulcerative_Colitis | 46 | 26.364 | 27.186 | 0.778 | 141.84 |
| | | Control | 97 | 15.880 | 9.273 | 4.912 | 71.264 |
| | | Diff (1-2) | _ | 10.484 | 17.159 | _ | _ |
| | Spinach | Ulcerative_Colitis | 46 | 24.393 | 17.724 | 2.770 | 95.908 |
| | | Control | 97 | 14.656 | 7.304 | 3.054 | 39.867 |
| | | Diff (1-2) | _ | 9.737 | 11.687 | _ | _ |
| | Squashes | Ulcerative_Colitis | 46 | 18.247 | 11.663 | 0.100 | 50.213 |
| | | Control | 97 | 12.688 | 7.539 | 1.637 | 49.775 |
| | | Diff (1-2) | _ | 5.558 | 9.062 | _ | _ |
| | Strawberry | Ulcerative_Colitis | 46 | 6.490 | 5.578 | 0.100 | 34.770 |
| | | Control | 97 | 4.767 | 4.446 | 0.100 | 30.664 |
| | | Diff (1-2) | _ | 1.724 | 4.836 | _ | _ |
| | String_Bean | Ulcerative_Colitis | 46 | 59.790 | 51.398 | 4.432 | 325.08 |
| | | Control | 97 | 40.720 | 22.088 | 5.609 | 141.76 |
| | | Diff (1-2) | _ | 19.070 | 34.283 | _ | _ |
| | Sunflower_Sd | Ulcerative_Colitis | 46 | 21.265 | 47.116 | 0.100 | 326.78 |
| | | Control | 97 | 9.071 | 5.842 | 2.523 | 46.948 |
| | | Diff (1-2) | | 12.193 | 27.050 | _ | _ |
| | Sweet_Pot_ | Ulcerative_Colitis | _ 46 | 13.540 | 9.152 | 0.100 | 38.861 |
| | | Control | 97 | 8.456 | 4.878 | 0.100 | 30.052 |
| | | Diff (1-2) | _ | 5.084 | 6.552 | _ | _ |
| | Swiss_Ch_ | Ulcerative_Colitis | 46 | 62.321 | 76.987 | 0.100 | 353.99 |
| | | Control | 97 | 43.413 | 79.791 | 0.100 | 400.00 |
| | | Diff (1-2) | _ | 18.908 | 78.907 | _ | _ |
| | Tea | Ulcerative_Colitis | 46 | 34.993 | 14.697 | 8.857 | 76.433 |
| | | Control | 97 | 31.353 | 13.716 | 8.890 | 70.271 |
| | | Diff (1-2) | _ | 3.640 | 14.036 | _ | _ |
| | Tobacco | Ulcerative_Colitis | 46 | 52.669 | 54.079 | 10.677 | 354.77 |
| | | Control | 97 | 39.354 | 26.787 | 6.106 | 134.30 |
| | | Diff (1-2) | _ | 13.315 | 37.708 | _ | _ |
| | Tomato | Ulcerative_Colitis | 46 | 19.627 | 43.625 | 0.100 | 301.96 |
| | | Control | 97 | 9.088 | 7.957 | 0.100 | 48.338 |
| | | Diff (1-2) | _ | 10.539 | 25.504 | _ | _ |
| | Trout | Ulcerative_Colitis | 46 | 17.035 | 10.017 | 0.100 | 57.313 |
| | | Control | 97 | 16.891 | 15.673 | 0.100 | 144.46 |
| | | Diff (1-2) | _ | 0.144 | 14.116 | _ | _ |
| | Tuna | Ulcerative_Colitis | 46 | 17.635 | 11.232 | 0.100 | 48.815 |
| | | Control | 97 | 18.392 | 16.755 | 3.156 | 110.69 |
| | | Diff (1-2) | _ | -0.757 | 15.211 | _ | _ |
| | Turkey | Ulcerative_Colitis | 46 | 17.700 | 13.152 | 0.100 | 60.557 |
| | | Control | 97 | 14.840 | 10.829 | 2.789 | 69.572 |
| | | Diff (1-2) | _ | 2.860 | 11.621 | _ | _ |
| | Walnut_Blk | Ulcerative_Colitis | 46 | 41.473 | 31.581 | 2.178 | 146.59 |
| | | Control | 97 | 25.520 | 14.492 | 4.249 | 71.927 |
| | | Diff (1-2) | _ | 15.952 | 21.478 | _ | _ |
| | Wheat | Ulcerative_Colitis | 46 | 46.983 | 93.083 | 0.100 | 400.00 |
| | | Control | 97 | 14.494 | 12.413 | 2.741 | 90.037 |
| | | Diff (1-2) | _ | 32.489 | 53.574 | _ | _ |
| | Yeast_Baker | Ulcerative_Colitis | 46 | 11.891 | 14.388 | 0.100 | 81.470 |
| | | Control | 97 | 9.617 | 17.250 | 1.305 | 116.43 |
| | | Diff (1-2) | _ | 2.273 | 16.391 | _ | _ |
| | Yeast_Brewer | Ulcerative_Colitis | 46 | 25.256 | 36.449 | 0.100 | 190.55 |
| | | Control | 97 | 22.646 | 47.630 | 1.931 | 308.34 |
| | | Diff (1-2) | _ | 2.611 | 44.369 | _ | _ |
| | Yogurt | Ulcerative_Colitis | 46 | 27.628 | 20.117 | 0.100 | 77.470 |
| | | Control | 97 | 19.210 | 20.751 | 0.234 | 120.51 |
| | | Diff (1-2) | _ | 8.418 | 20.551 | _ | _ |

### Upper Quantiles of ELISA Signal Scores among Control Subjects as Candidates for Test Cutpoints in Determining "Positive" or "Negative" Top 58 Foods Ranked by Descending order of Discriminatory Ability using Permutation Test Ulcerative_Colitis Subjects vs. Controls

**Table 4**

| *Food Ranking* | *Food* | *Sex* | *Cutpoint* | |
|---|---|---|---|---|
| | | | *90th percentile* | *95th percentile* |
| 1 | Green_Pea | FEMALE | 20.814 | 23.684 |
| | | MALE | 19.788 | 32.100 |
| 2 | Cantaloupe | FEMALE | 9.672 | 13.552 |
| | | MALE | 11.337 | 16.219 |
| 3 | Pinto_Bean | FEMALE | 18.863 | 27.923 |
| | | MALE | 16.119 | 20.774 |
| 4 | Cucumber | FEMALE | 20.944 | 26.779 |
| | | MALE | 17.891 | 23.472 |
| 5 | Green_Pepper | FEMALE | 8.275 | 10.402 |
| | | MALE | 7.054 | 9.712 |
| 6 | Grapefruit | FEMALE | 6.215 | 7.611 |
| | | MALE | 5.330 | 7.738 |
| 7 | Carrot | FEMALE | 9.212 | 11.448 |
| | | MALE | 7.807 | 10.836 |
| 8 | Orange | FEMALE | 33.707 | 40.739 |
| | | MALE | 37.082 | 56.031 |
| 9 | Almond | FEMALE | 6.751 | 8.235 |
| | | MALE | 7.259 | 8.824 |
| 10 | Sardine | FEMALE | 58.683 | 73.442 |
| | | MALE | 57.359 | 64.811 |
| 11 | Sweet_Pot_ | FEMALE | 14.644 | 17.301 |
| | | MALE | 13.894 | 18.378 |
| 12 | Broccoli | FEMALE | 11.826 | 14.843 |
| | | MALE | 13.203 | 15.982 |
| 13 | Garlic | FEMALE | 19.323 | 22.695 |
| | | MALE | 27.228 | 41.008 |
| 14 | Lima_Bean | FEMALE | 12.667 | 18.798 |
| | | MALE | 10.738 | 14.912 |
| 15 | Squashes | FEMALE | 22.217 | 32.815 |
| | | MALE | 22.931 | 26.147 |
| 16 | Celery | FEMALE | 17.085 | 22.342 |
| | | MALE | 15.101 | 19.687 |
| 17 | String_Bean | FEMALE | 68.618 | 84.869 |
| | | MALE | 65.384 | 83.179 |
| 18 | Tomato | FEMALE | 17.721 | 23.905 |
| | | MALE | 18.818 | 26.329 |
| 19 | Cauliflower | FEMALE | 11.527 | 17.829 |
| | | MALE | 8.004 | 11.222 |
| 20 | Walnut_Blk | FEMALE | 45.008 | 56.778 |
| | | MALE | 45.356 | 56.848 |
| 21 | Sunflower_Sd | FEMALE | 16.611 | 22.529 |
| | | MALE | 14.239 | 18.733 |
| 22 | Cane_Sugar | FEMALE | 29.824 | 36.249 |
| | | MALE | 45.468 | 64.941 |
| 23 | Buck_Wheat | FEMALE | 14.739 | 18.482 |
| | | MALE | 11.356 | 12.773 |
| 24 | Soybean | FEMALE | 30.770 | 34.674 |
| | | MALE | 26.301 | 31.395 |
| 25 | Lemon | FEMALE | 4.556 | 5.959 |
| | | MALE | 4.179 | 5.210 |
| 26 | Barley | FEMALE | 35.136 | 46.859 |
| | | MALE | 36.197 | 45.928 |
| 27 | Oat | FEMALE | 33.278 | 44.414 |
| | | MALE | 55.311 | 72.680 |
| 28 | Oyster | FEMALE | 86.278 | 114.96 |
| | | MALE | 82.294 | 119.88 |
| 29 | Mustard | FEMALE | 17.479 | 19.400 |
| | | MALE | 16.227 | 20.884 |
| 30 | Rye | FEMALE | 8.475 | 12.141 |
| | | MALE | 8.360 | 10.635 |
| 31 | Peach | FEMALE | 17.987 | 26.936 |
| | | MALE | 17.616 | 26.755 |
| 32 | Chili_Pepper | FEMALE | 16.296 | 25.191 |
| | | MALE | 14.040 | 21.503 |
| 33 | Spinach | FEMALE | 37.895 | 48.052 |
| | | MALE | 24.957 | 28.650 |
| 34 | Peanut | FEMALE | 11.190 | 16.279 |
| | | MALE | 6.920 | 9.159 |
| 35 | Avocado | FEMALE | 5.397 | 7.247 |
| | | MALE | 4.483 | 5.566 |
| 36 | Shrimp | FEMALE | 81.870 | 98.743 |
| | | MALE | 69.799 | 101.18 |
| 37 | Pineapple | FEMALE | 65.230 | 122.14 |
| | | MALE | 65.661 | 106.68 |
| 38 | Cola_Nut | FEMALE | 48.288 | 53.448 |
| | | MALE | 59.969 | 72.288 |
| 39 | Rice | FEMALE | 40.837 | 58.139 |
| | | MALE | 52.100 | 63.388 |
| 40 | Cabbage | FEMALE | 18.343 | 28.722 |
| | | MALE | 9.730 | 18.345 |
| 41 | Butter | FEMALE | 47.381 | 71.040 |
| | | MALE | 44.178 | 58.044 |
| 42 | Eggplant | FEMALE | 12.557 | 18.816 |
| | | MALE | 9.359 | 14.446 |
| 43 | Apple | FEMALE | 9.017 | 11.837 |
| | | MALE | 8.631 | 10.597 |
| 44 | Egg | FEMALE | 144.38 | 28.0.18 |
| | | MALE | 106.91 | 197.02 |
| 45 | Wheat | FEMALE | 30.663 | 56.824 |
| | | MALE | 27.355 | 37.901 |
| 46 | Cottage_Ch_ | FEMALE | 200.80 | 287.02 |
| | | MALE | 220.78 | 348.31 |
| 47 | Sole | FEMALE | 9.355 | 14.730 |
| | | MALE | 7.466 | 9.176 |
| 48 | Cashew | FEMALE | 23.551 | 44.896 |
| | | MALE | 17.371 | 32.259 |
| 49 | Olive | FEMALE | 48.012 | 55.113 |
| | | MALE | 42.612 | 61.277 |
| 50 | Parsley | FEMALE | 11.123 | 19.965 |
| | | MALE | 8.545 | 17.265 |
| 51 | Corn | FEMALE | 20.036 | 31.057 |
| | | MALE | 19.953 | 30.126 |
| 52 | Honey | FEMALE | 16.276 | 17.419 |
| | | MALE | 19.199 | 24.877 |
| 53 | Chocolate | FEMALE | 23.555 | 25.869 |
| | | MALE | 32.644 | 37.625 |
| 54 | Cow_Milk | FEMALE | 199.39 | 248.98 |
| | | MALE | 181.23 | 316.72 |
| 55 | Potato | FEMALE | 20.155 | 25.293 |
| | | MALE | 21.203 | 24.281 |
| 56 | Onion | FEMALE | 20.204 | 37.487 |
| | | MALE | 25.719 | 33.230 |
| 57 | Tea | FEMALE | 46.116 | 53.257 |
| | | MALE | 49.893 | 56.701 |
| 58 | Tobacco | FEMALE | 57.943 | 64.379 |
| | | MALE | 73.610 | 101.38 |

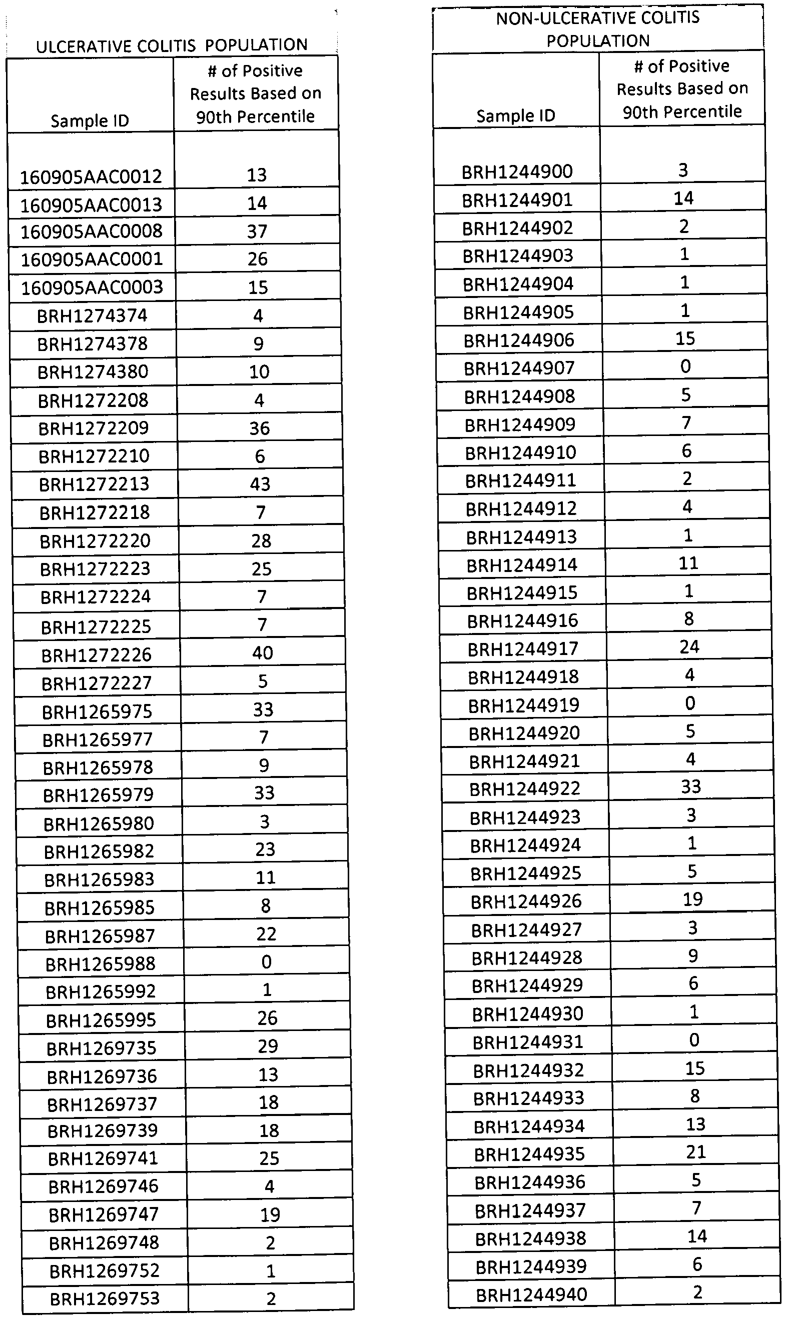

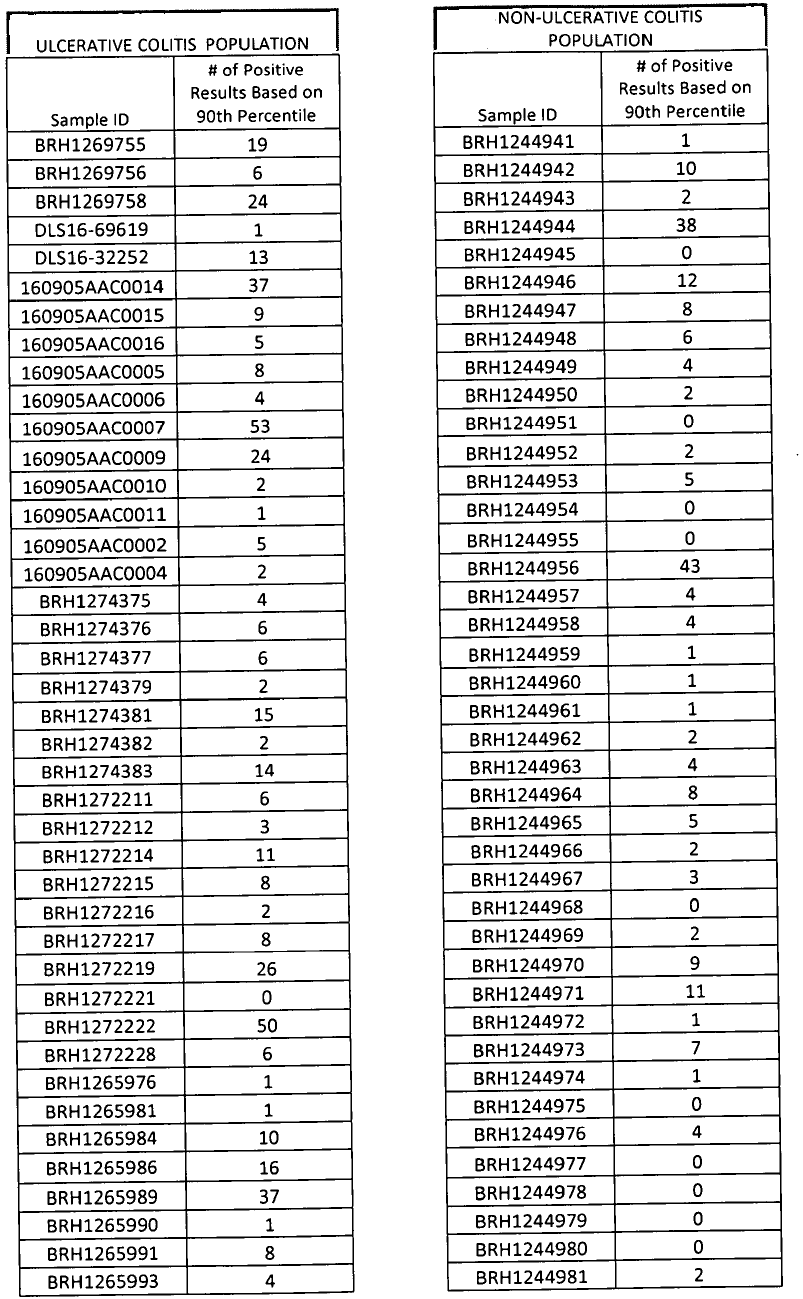

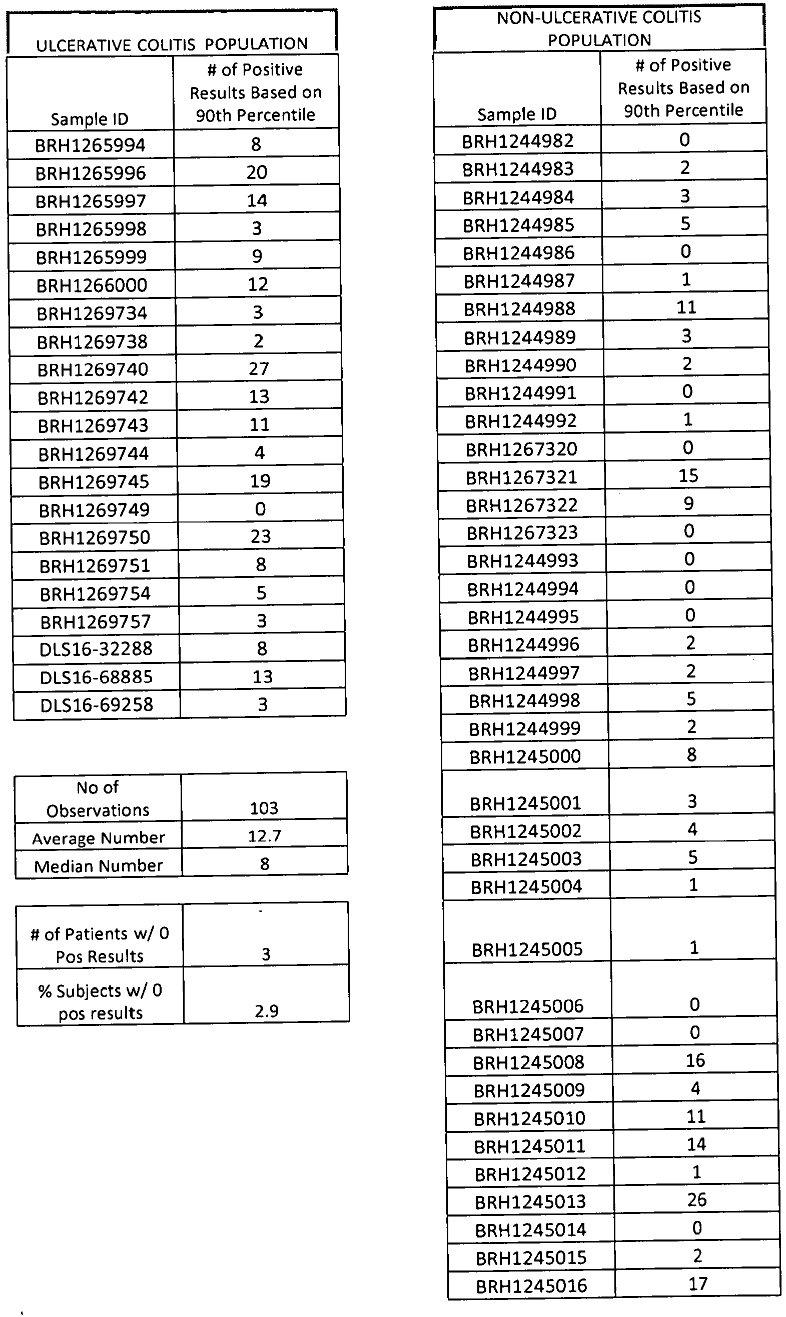

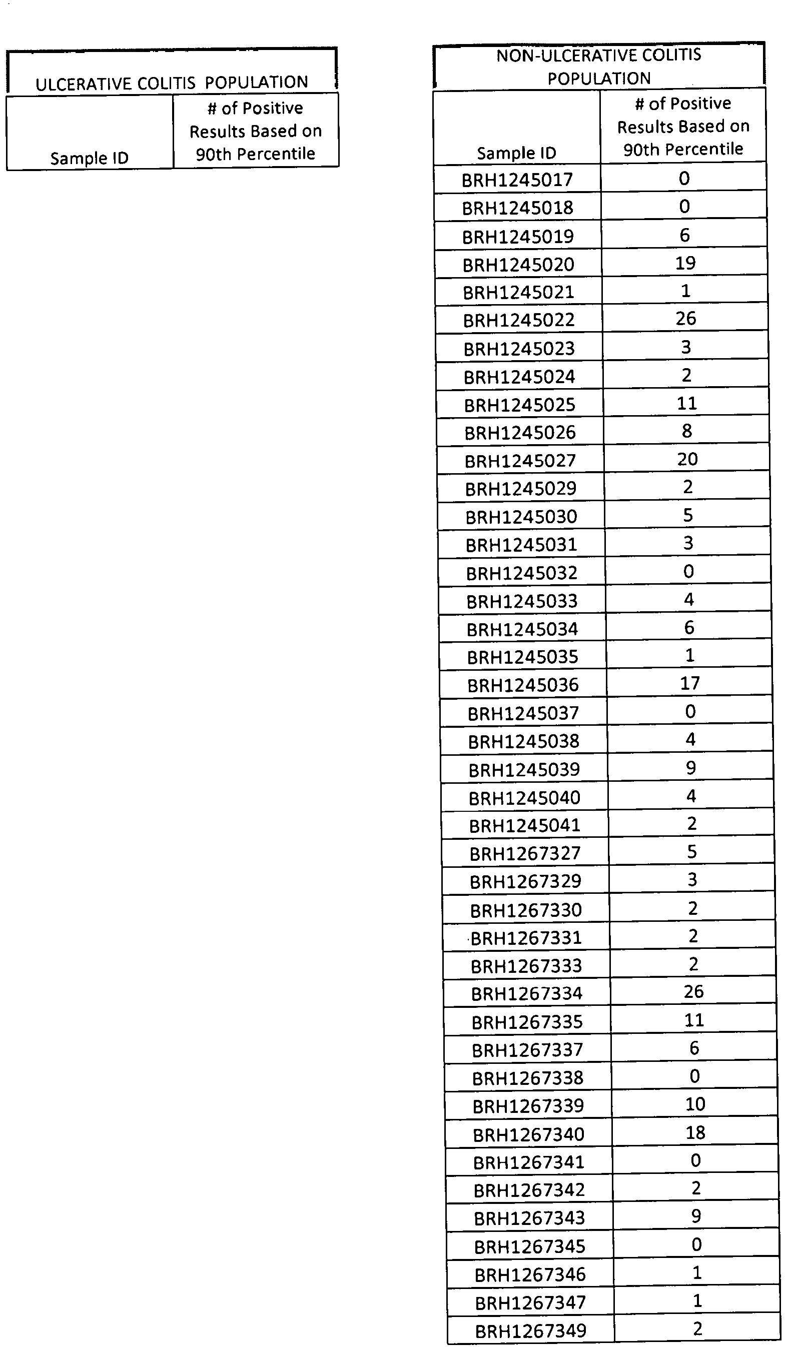

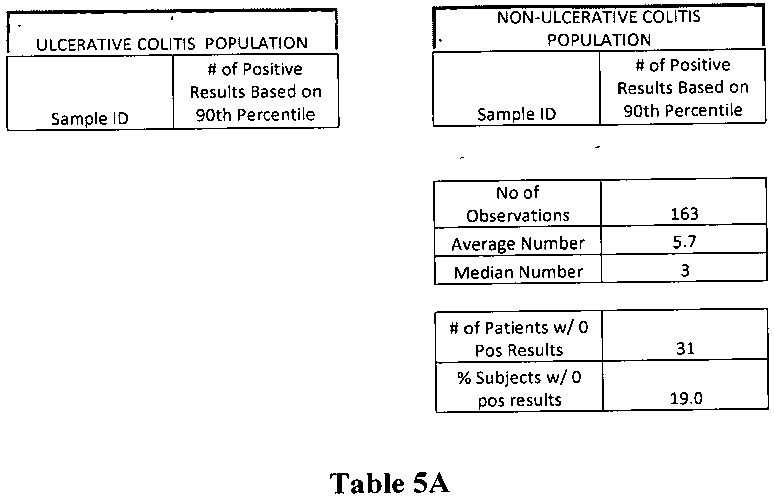

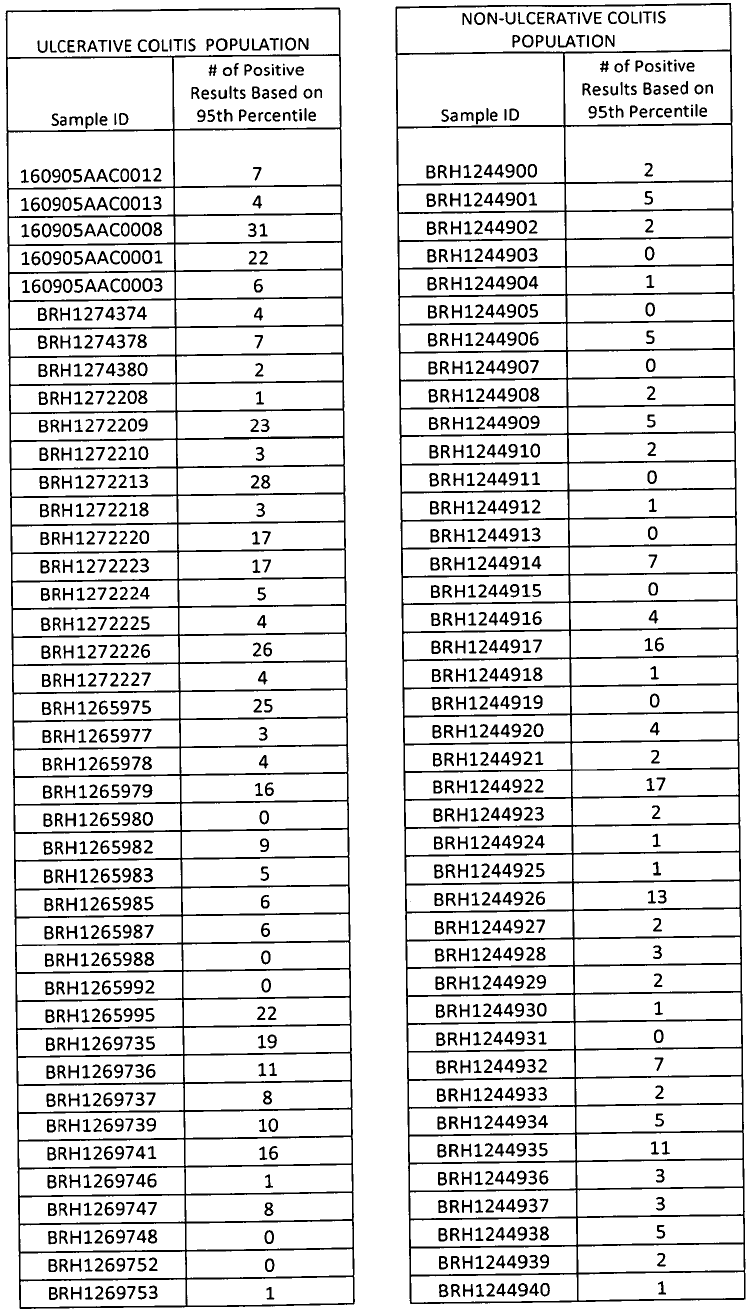

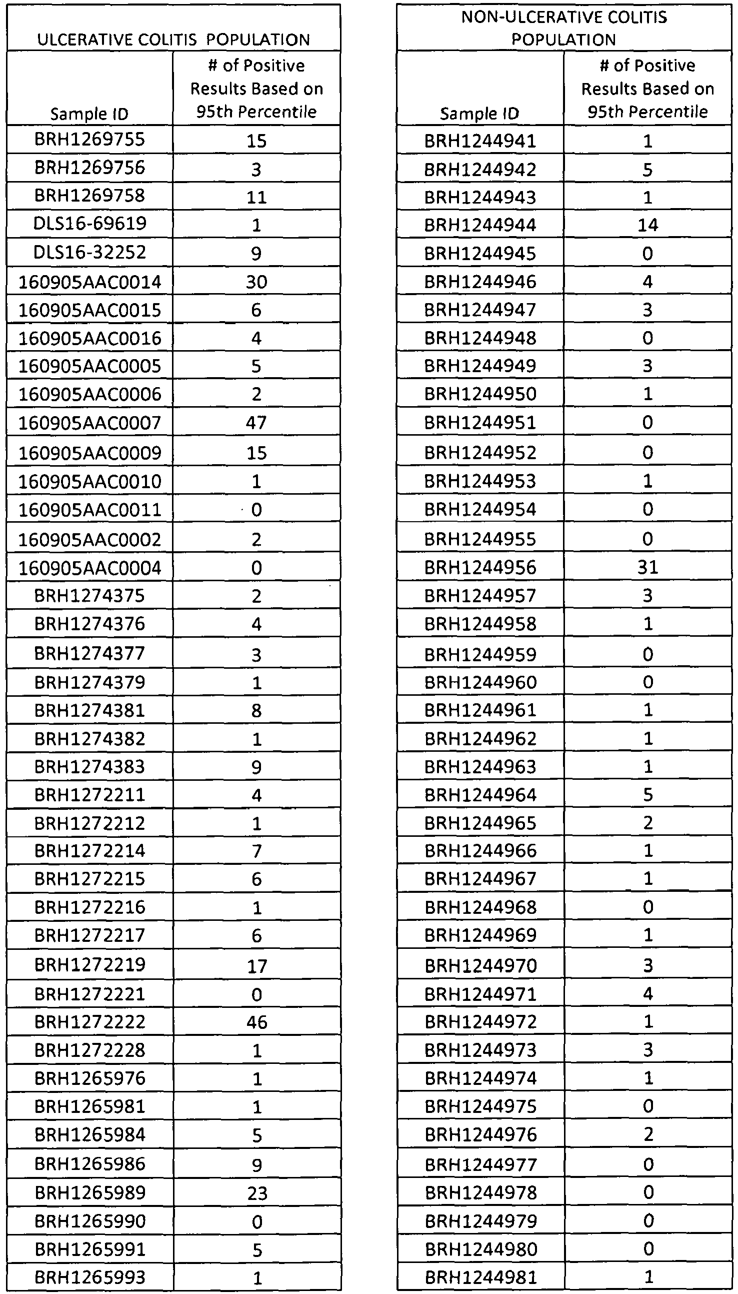

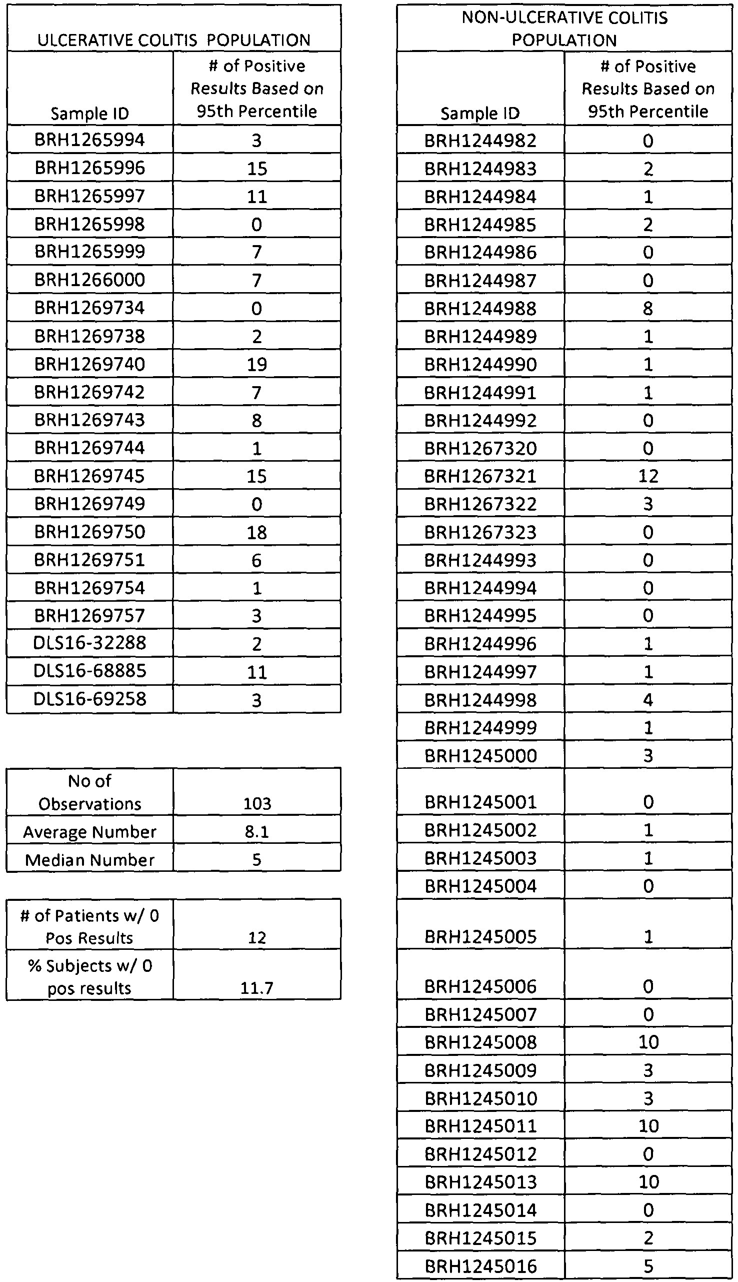

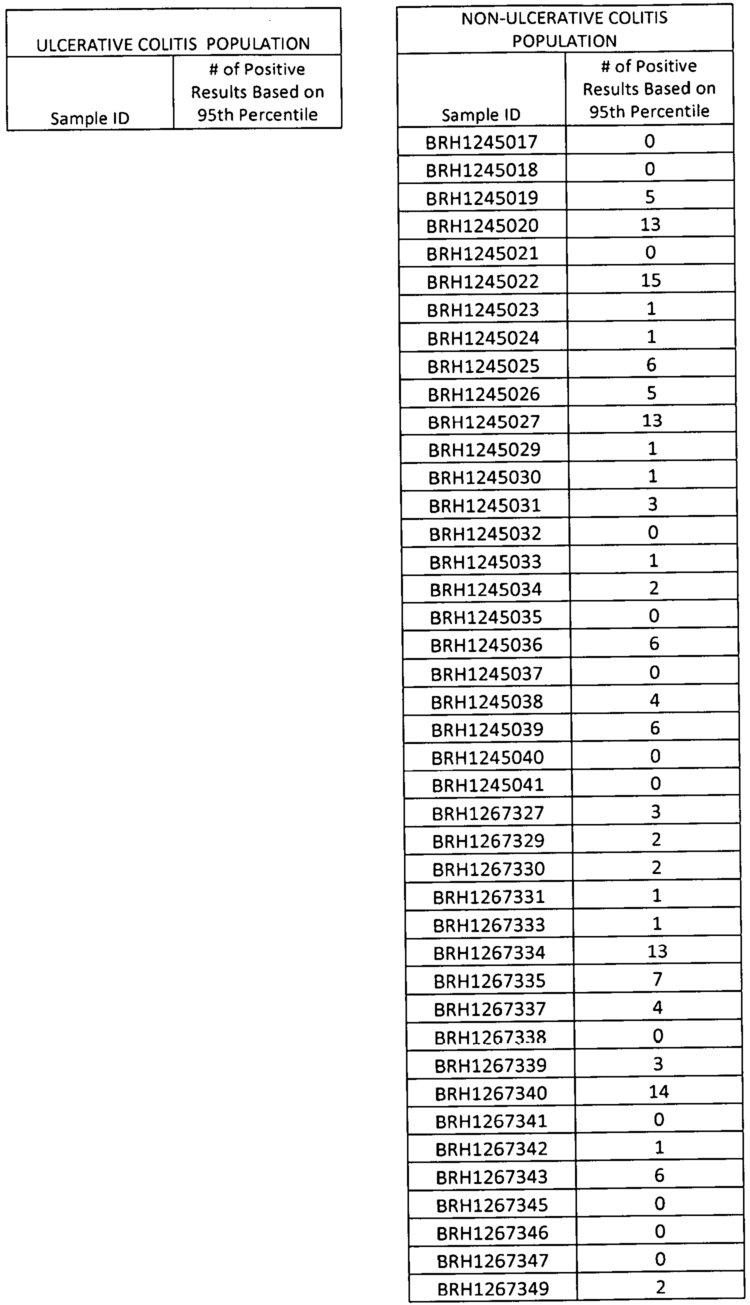

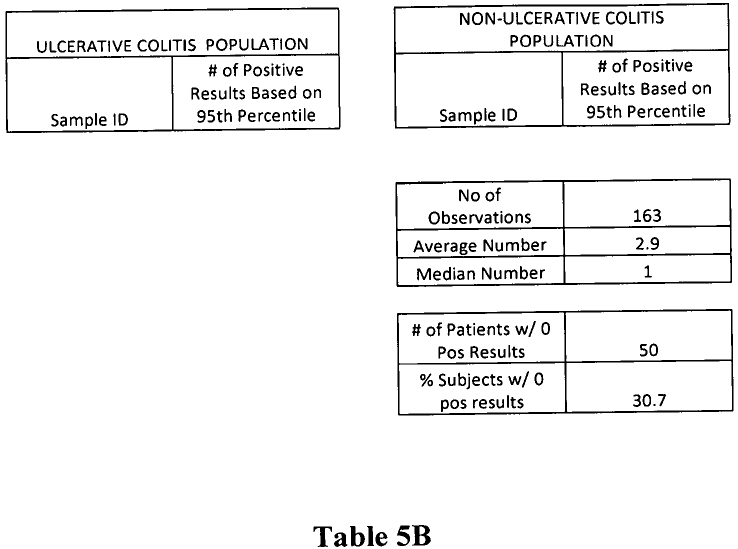

### Summary statistics

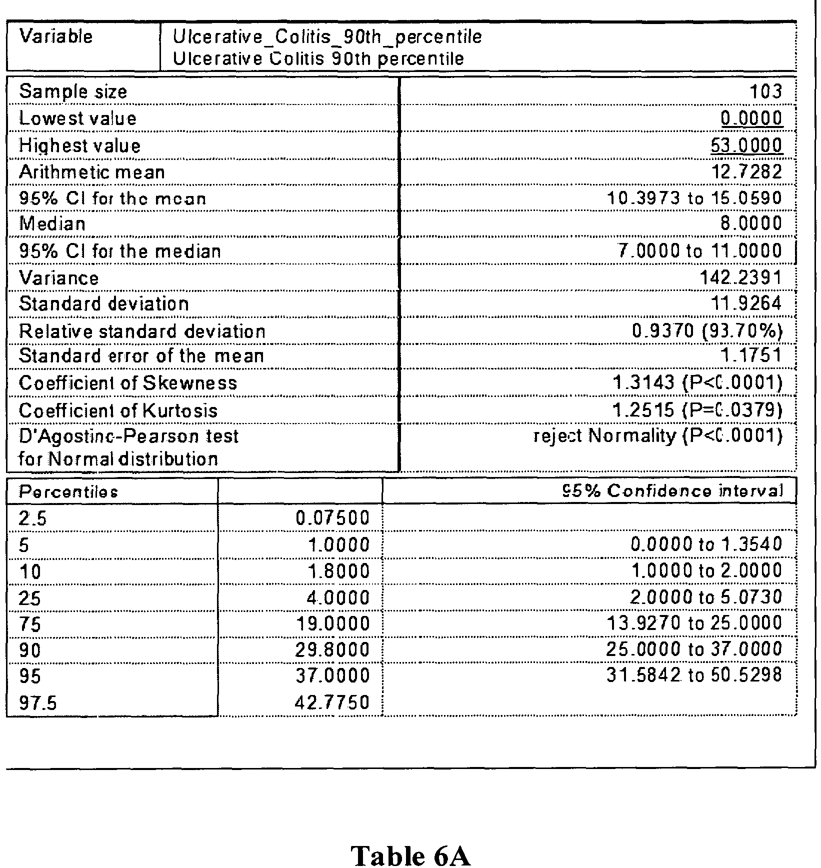

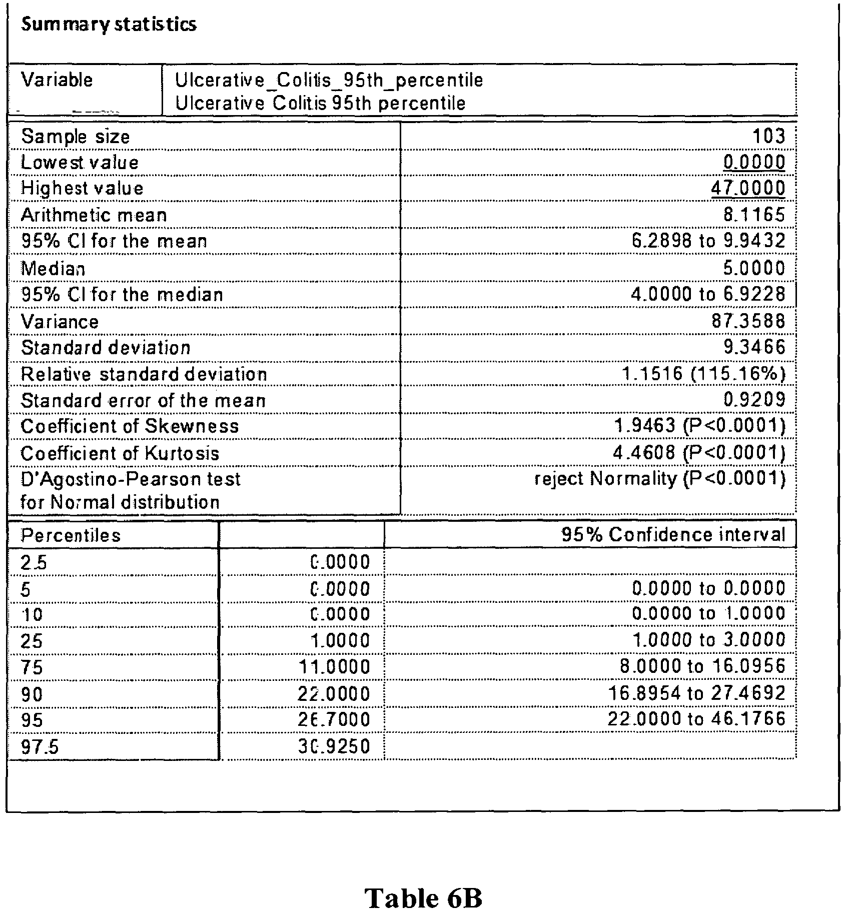

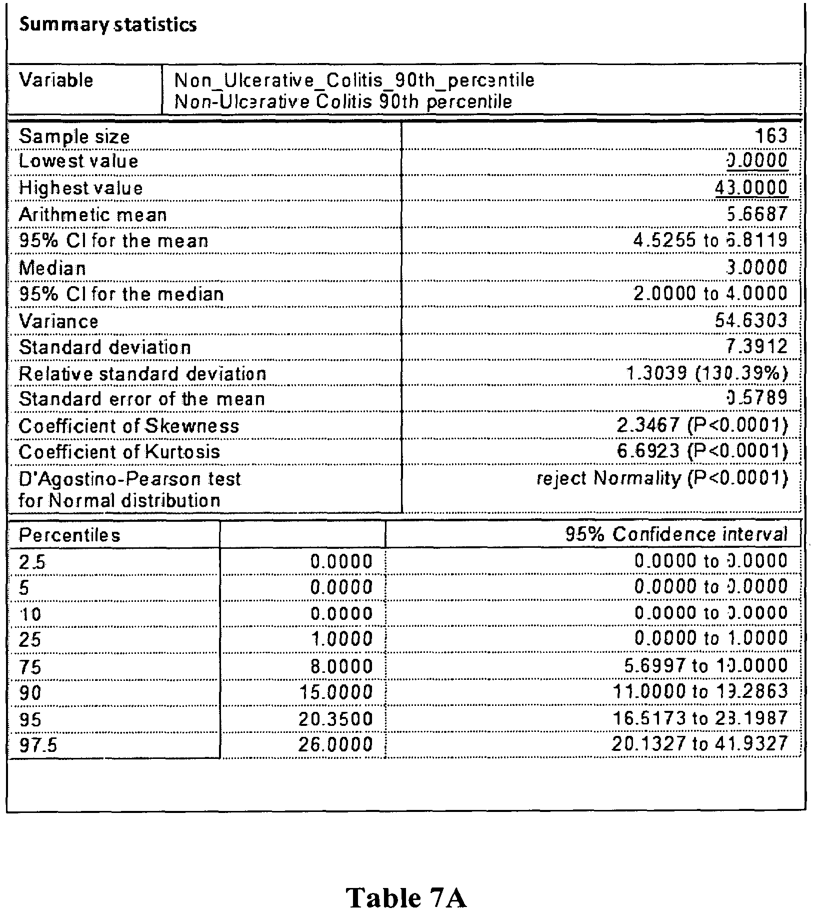

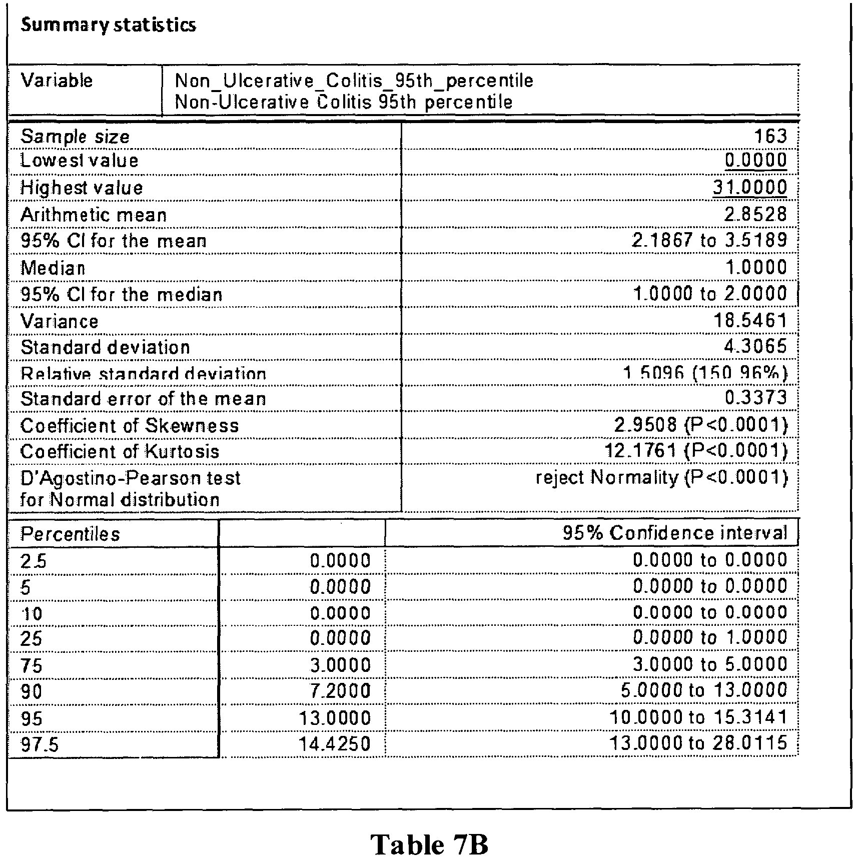

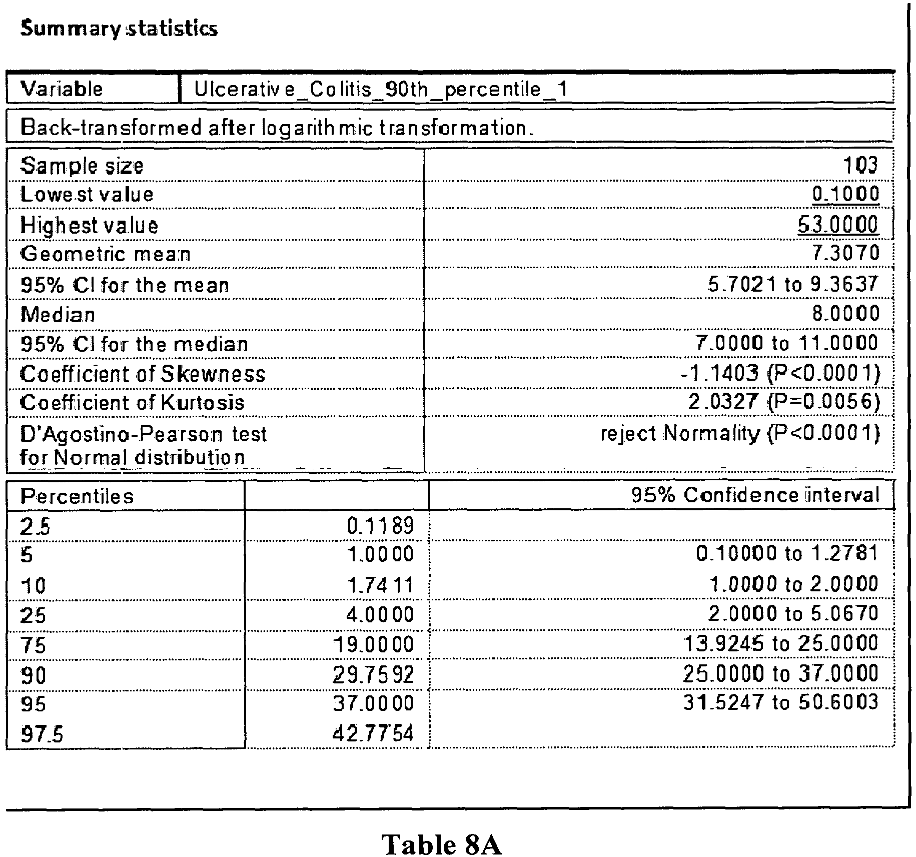

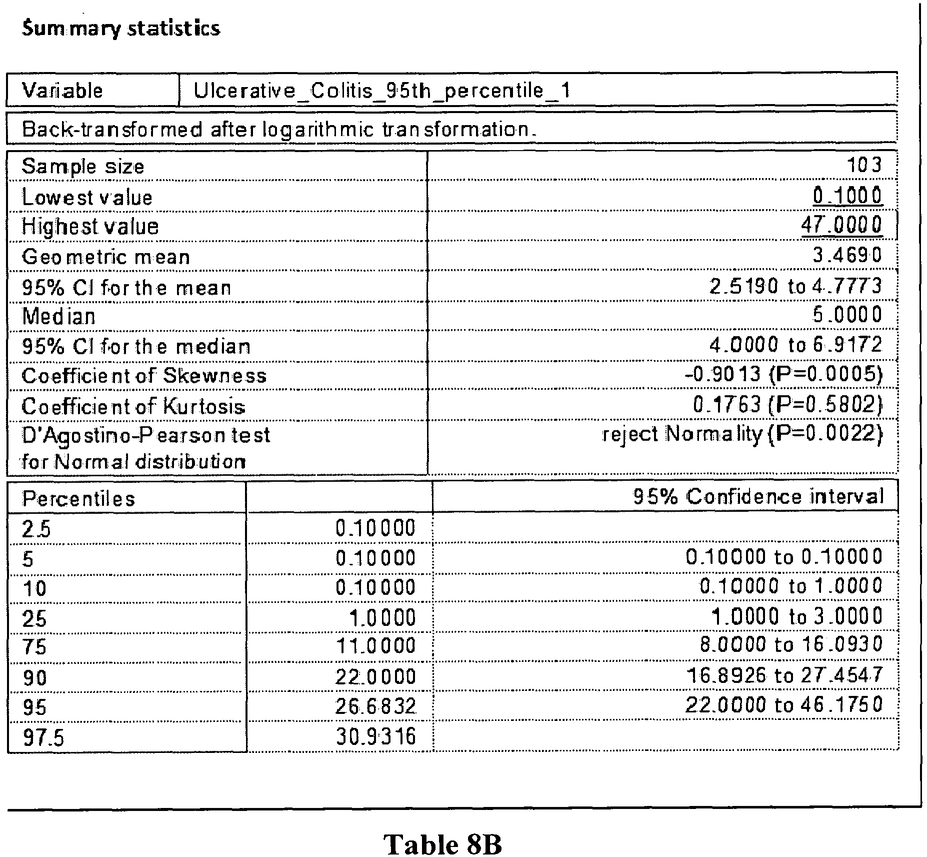

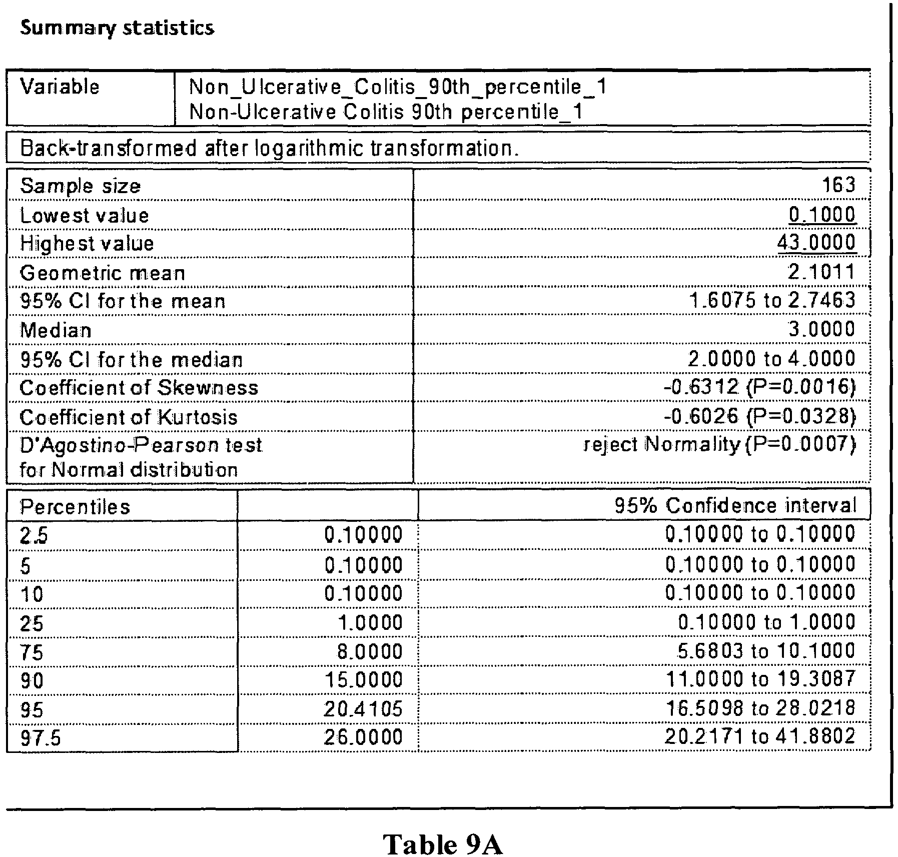

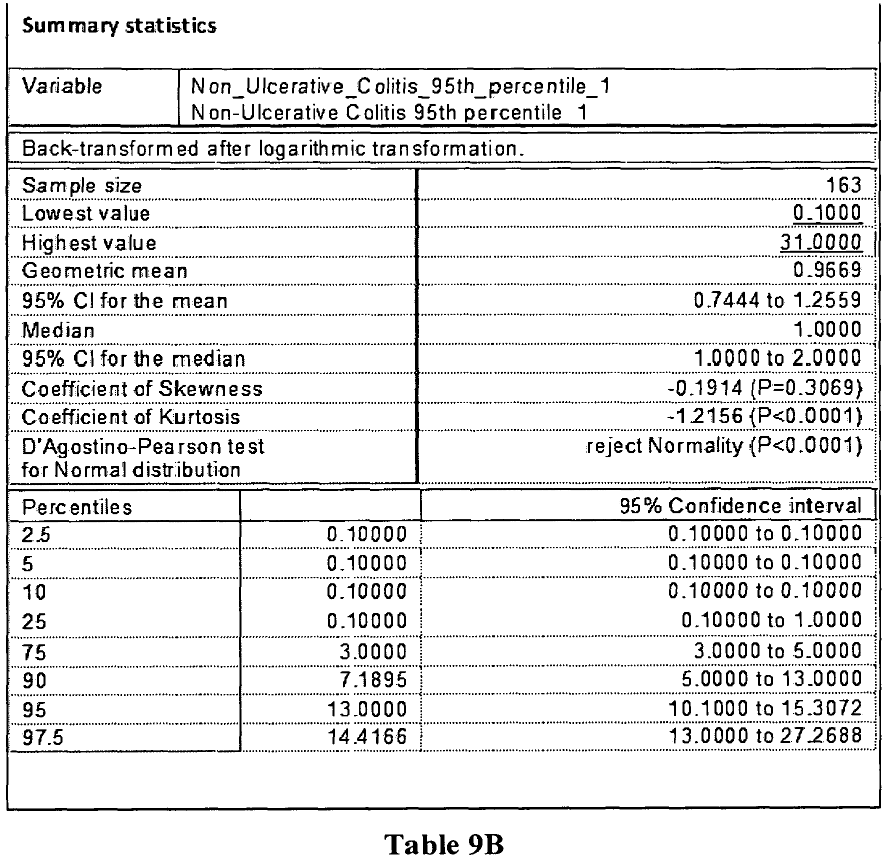

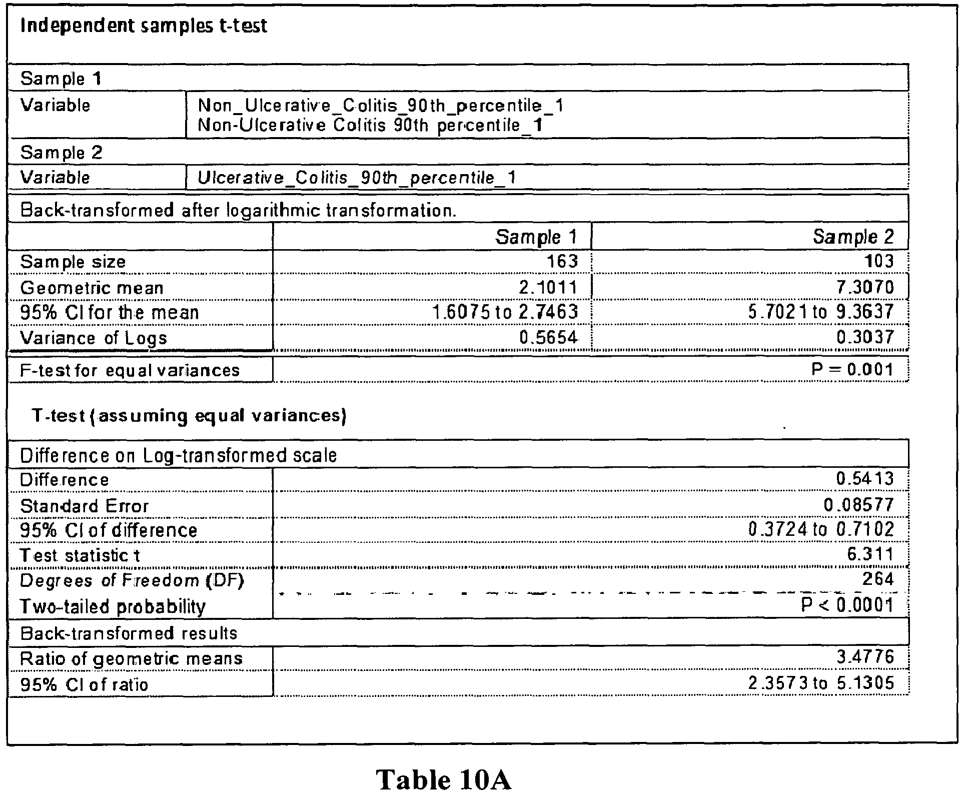

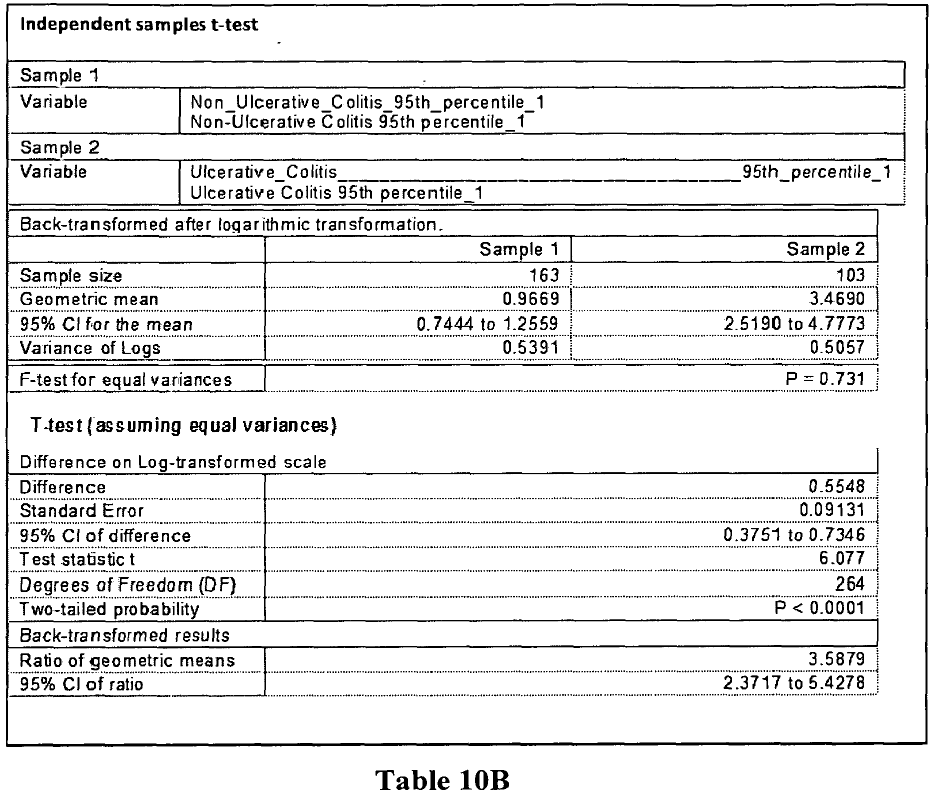

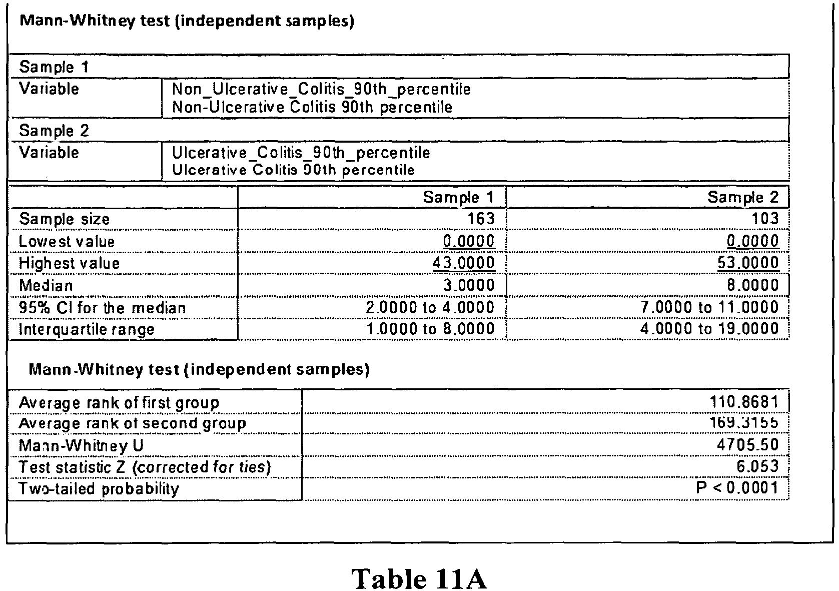

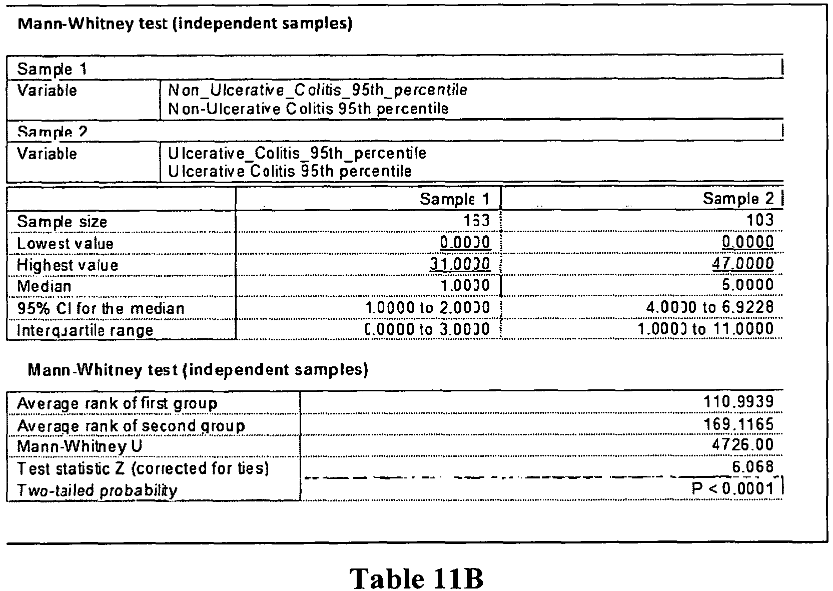

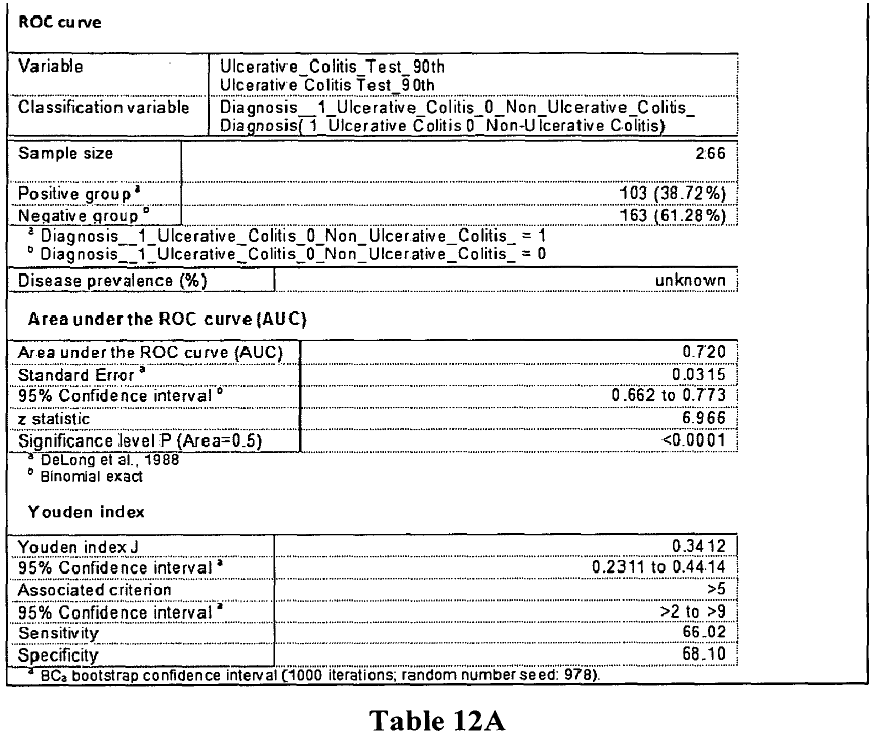

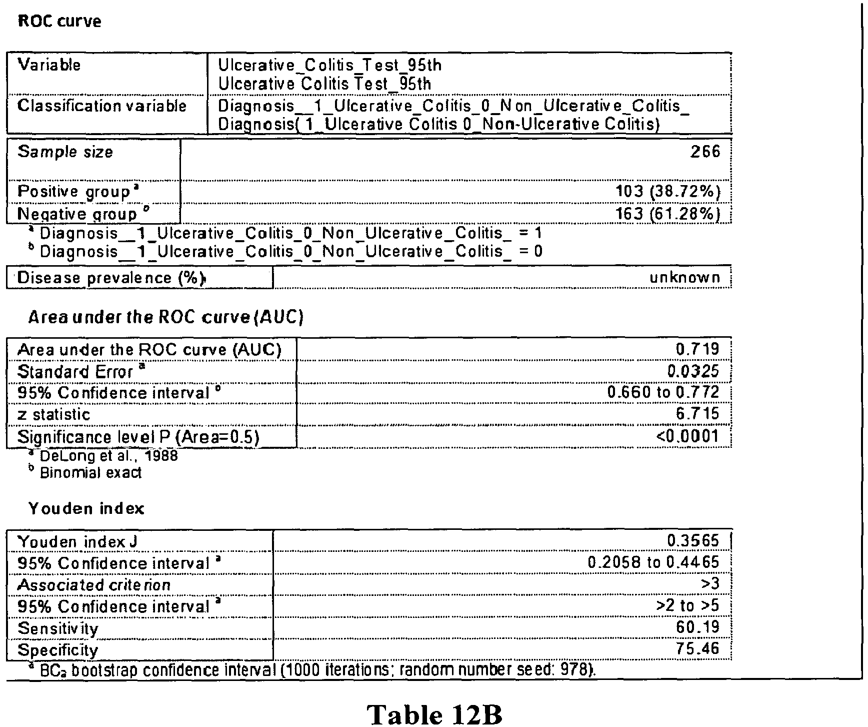

### Performance Metrics in Predicting Ulcerative Colitis Status from Number of Positive Foods Using 90th Percentile of ELISA Signal to determine Positive

**Table 13A**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| FEMALE | 1 | 0.97 | 0.14 | 0.49 | 0.83 | 0.52 |
| | 2 | 0.92 | 0.29 | 0.52 | 0.80 | 0.58 |
| | 3 | 0.85 | 0.40 | 0.55 | 0.75 | 0.61 |
| | 4 | 0.76 | 0.49 | 0.56 | 0.71 | 0.62 |
| | 5 | 0.69 | 0.57 | 0.58 | 0.68 | 0.62 |
| | 6 | 0.62 | 0.62 | 0.58 | 0.65 | 0.62 |
| | 7 | 0.55 | 0.66 | 0.58 | 0.63 | 0.61 |
| | 8 | 0.49 | 0.69 | 0.57 | 0.61 | 0.60 |
| | 9 | 0.44 | 0.72 | 0.57 | 0.60 | 0.59 |
| | 10 | 0.39 | 0.75 | 0.58 | 0.59 | 0.58 |
| | 11 | 0.34 | 0.78 | 0.58 | 0.58 | 0.58 |
| | 12 | 0.31 | 0.81 | 0.59 | 0.58 | 0.58 |
| | 13 | 0.28 | 0.83 | 0.59 | 0.57 | 0.58 |
| | 14 | 0.25 | 0.84 | 0.58 | 0.56 | 0.57 |
| | 15 | 0.23 | 0.85 | 0.57 | 0.56 | 0.56 |
| | 16 | 0.21 | 0.86 | 0.57 | 0.56 | 0.56 |
| | 17 | 0.20 | 0.87 | 0.57 | 0.56 | 0.56 |
| | 18 | 0.19 | 0.88 | 0.58 | 0.56 | 0.56 |
| | 19 | 0.18 | 0.90 | 0.60 | 0.56 | 0.56 |
| | 20 | 0.17 | 0.91 | 0.63 | 0.56 | 0.57 |
| | 21 | 0.16 | 0.93 | 0.64 | 0.56 | 0.57 |
| | 22 | 0.15 | 0.93 | 0.67 | 0.56 | 0.57 |
| | 23 | 0.15 | 0.95 | 0.67 | 0.56 | 0.57 |
| | 24 | 0.14 | 0.95 | 0.71 | 0.56 | 0.57 |
| | 25 | 0.13 | 0.95 | 0.71 | 0.56 | 0.57 |
| | 26 | 0.11 | 0.96 | 0.71 | 0.56 | 0.57 |
| | 27 | 0.11 | 0.97 | 0.75 | 0.56 | 0.57 |
| | 28 | 0.09 | 0.98 | 0.75 | 0.55 | 0.57 |
| | 29 | 0.08 | 0.98 | 0.80 | 0.55 | 0.57 |
| | 30 | 0.08 | 1.00 | 1.00 | 0.55 | 0.57 |
| | 31 | 0.08 | 1.00 | 1.00 | 0.55 | 0.57 |
| | 32 | 0.07 | 1.00 | 1.00 | 0.55 | 0.57 |
| | 33 | 0.07 | 1.00 | 1.00 | 0.55 | 0.57 |
| | 34 | 0.06 | 1.00 | 1.00 | 0.55 | 0.56 |
| | 35 | 0.06 | 1.00 | 1.00 | 0.55 | 0.56 |
| | 36 | 0.06 | 1.00 | 1.00 | 0.55 | 0.56 |
| | 37 | 0.05 | 1.00 | 1.00 | 0.55 | 0.56 |
| | 38 | 0.05 | 1.00 | 1.00 | 0.55 | 0.56 |
| | 39 | 0.05 | 1.00 | 1.00 | 0.55 | 0.56 |
| | 40 | 0.03 | 1.00 | 1.00 | 0.55 | 0.55 |
| | 41 | 0.03 | 1.00 | 1.00 | 0.54 | 0.55 |
| | 42 | 0.03 | 1.00 | 1.00 | 0.54 | 0.55 |
| | 43 | 0.03 | 1.00 | 1.00 | 0.54 | 0.55 |
| | 44 | 0.03 | 1.00 | 1.00 | 0.54 | 0.55 |
| | 45 | 0.03 | 1.00 | 1.00 | 0.54 | 0.55 |
| | 46 | 0.03 | 1.00 | 1.00 | 0.54 | 0.55 |
| | 47 | 0.03 | 1.00 | 1.00 | 0.54 | 0.55 |
| | 48 | 0.03 | 1.00 | 1.00 | 0.54 | 0.55 |
| | 49 | 0.03 | 1.00 | 1.00 | 0.54 | 0.55 |
| | 50 | 0.03 | 1.00 | 1.00 | 0.54 | 0.55 |
| | 51 | 0.03 | 1.00 | 1.00 | 0.54 | 0.55 |
| | 52 | 0.03 | 1.00 | 1.00 | 0.54 | 0.54 |
| | 53 | 0.02 | 1.00 | 1.00 | 0.54 | 0.54 |
| | 54 | 0.00 | 1.00 | 1.00 | 0.54 | 0.54 |
| | 55 | 0.00 | 1.00 | 1.00 | 0.54 | 0.54 |
| | 56 | 0.00 | 1.00 | 1.00 | 0.54 | 0.54 |
| | 57 | 0.00 | 1.00 | . | 0.53 | 0.53 |
| | 58 | 0.00 | 1.00 | . | 0.53 | 0.53 |

### Performance Metrics in Predicting Ulcerative Colitis Status from Number of Positive Foods Using 90th Percentile of ELISA Signal to determine Positive

**Table 13B**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| MALE | 1 | 0.97 | 0.15 | 0.35 | 0.90 | 0.41 |
| | 2 | 0.94 | 0.29 | 0.38 | 0.91 | 0.49 |
| | 3 | 0.88 | 0.42 | 0.42 | 0.88 | 0.57 |
| | 4 | 0.84 | 0.50 | 0.45 | 0.87 | 0.61 |
| | 5 | 0.81 | 0.56 | 0.47 | 0.86 | 0.64 |
| | 6 | 0.77 | 0.63 | 0.49 | 0.85 | 0.67 |
| | 7 | 0.72 | 0.68 | 0.51 | 0.84 | 0.69 |
| | 8 | 0.67 | 0.72 | 0.53 | 0.82 | 0.71 |
| | 9 | 0.64 | 0.76 | 0.56 | 0.81 | 0.72 |
| | 10 | 0.59 | 0.79 | 0.57 | 0.80 | 0.73 |
| | 11 | 0.56 | 0.82 | 0.59 | 0.80 | 0.73 |
| | 12 | 0.54 | 0.84 | 0.62 | 0.79 | 0.74 |
| | 13 | 0.52 | 0.86 | 0.64 | 0.79 | 0.75 |
| | 14 | 0.50 | 0.87 | 0.65 | 0.78 | 0.75 |
| | 15 | 0.46 | 0.89 | 0.67 | 0.78 | 0.75 |
| | 16 | 0.44 | 0.90 | 0.68 | 0.77 | 0.75 |
| | 17 | 0.42 | 0.92 | 0.71 | 0.77 | 0.76 |
| | 18 | 0.39 | 0.93 | 0.71 | 0.76 | 0.76 |
| | 19 | 0.38 | 0.93 | 0.71 | 0.76 | 0.75 |
| | 20 | 0.36 | 0.94 | 0.73 | 0.75 | 0.75 |
| | 21 | 0.34 | 0.94 | 0.73 | 0.75 | 0.75 |
| | 22 | 0.32 | 0.95 | 0.73 | 0.75 | 0.75 |
| | 23 | 0.31 | 0.95 | 0.75 | 0.74 | 0.75 |
| | 24 | 0.30 | 0.95 | 0.75 | 0.74 | 0.74 |
| | 25 | 0.28 | 0.95 | 0.75 | 0.74 | 0.74 |
| | 26 | 0.27 | 0.96 | 0.75 | 0.73 | 0.73 |
| | 27 | 0.23 | 0.96 | 0.75 | 0.73 | 0.73 |
| | 28 | 0.21 | 0.97 | 0.73 | 0.72 | 0.72 |
| | 29 | 0.18 | 0.97 | 0.71 | 0.71 | 0.71 |
| | 30 | 0.16 | 0.97 | 0.70 | 0.71 | 0.71 |
| | 31 | 0.14 | 0.97 | 0.67 | 0.71 | 0.71 |
| | 32 | 0.13 | 0.97 | 0.67 | 0.70 | 0.70 |
| | 33 | 0.12 | 0.97 | 0.67 | 0.70 | 0.70 |
| | 34 | 0.11 | 0.97 | 0.67 | 0.70 | 0.70 |
| | 35 | 0.10 | 0.98 | 0.67 | 0.70 | 0.70 |
| | 36 | 0.08 | 0.98 | 0.67 | 0.69 | 0.69 |
| | 37 | 0.07 | 0.98 | 0.67 | 0.69 | 0.69 |
| | 38 | 0.06 | 0.98 | 0.50 | 0.69 | 0.68 |
| | 39 | 0.04 | 0.98 | 0.50 | 0.69 | 0.68 |
| | 40 | 0.03 | 0.98 | 0.50 | 0.68 | 0.68 |
| | 41 | 0.03 | 0.98 | 0.50 | 0.68 | 0.68 |
| | 42 | 0.00 | 0.98 | 0.00 | 0.68 | 0.68 |
| | 43 | 0.00 | 0.98 | 0.00 | 0.68 | 0.67 |
| | 44 | 0.00 | 0.98 | 0.00 | 0.68 | 0.67 |
| | 45 | 0.00 | 0.99 | 0.00 | 0.68 | 0.67 |
| | 46 | 0.00 | 1.00 | 0.00 | 0.68 | 0.67 |
| | 47 | 0.00 | 1.00 | 0.00 | 0.68 | 0.67 |
| | 48 | 0.00 | 1.00 | 0.00 | 0.68 | 0.67 |
| | 49 | 0.00 | 1.00 | 0.00 | 0.68 | 0.68 |
| | 50 | 0.00 | 1.00 | 0.00 | 0.68 | 0.68 |
| | 51 | 0.00 | 1.00 | 0.00 | 0.68 | 0.68 |
| | 52 | 0.00 | 1.00 | 0.00 | 0.68 | 0.68 |
| | 53 | 0.00 | 1.00 | 0.00 | 0.68 | 0.68 |
| | 54 | 0.00 | 1.00 | 0.00 | 0.68 | 0.68 |
| | 55 | 0.00 | 1.00 | 0.00 | 0.68 | 0.68 |
| | 56 | 0.00 | 1.00 | - | 0.68 | 0.68 |
| | 57 | 0.00 | 1.00 | - | 0.68 | 0.68 |
| | 58 | 0.00 | 1.00 | - | 0.68 | 0.68 |

### Performance Metrics in Predicting Ulcerative Colitis Status from Number of Positive Foods Using 95th Percentile of ELISA Signal to determine Positive

**Table 14A**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| FEMALE | 1 | 0.89 | 0.27 | 0.51 | 0.74 | 0.56 |
| | 2 | 0.75 | 0.45 | 0.54 | 0.68 | 0.59 |
| | 3 | 0.65 | 0.58 | 0.57 | 0.66 | 0.61 |
| | 4 | 0.55 | 0.65 | 0.58 | 0.63 | 0.61 |
| | 5 | 0.49 | 0.72 | 0.60 | 0.62 | 0.62 |
| | 6 | 0.44 | 0.76 | 0.61 | 0.61 | 0.61 |
| | 7 | 0.38 | 0.80 | 0.63 | 0.60 | 0.61 |
| | 8 | 0.33 | 0.83 | 0.63 | 0.59 | 0.60 |
| | 9 | 0.29 | 0.85 | 0.63 | 0.58 | 0.59 |
| | 10 | 0.25 | 0.87 | 0.63 | 0.57 | 0.58 |
| | 11 | 0.22 | 0.88 | 0.62 | 0.57 | 0.58 |
| | 12 | 0.19 | 0.90 | 0.63 | 0.56 | 0.57 |
| | 13 | 0.18 | 0.91 | 0.64 | 0.56 | 0.57 |
| | 14 | 0.18 | 0.93 | 0.67 | 0.56 | 0.58 |
| | 15 | 0.17 | 0.94 | 0.70 | 0.57 | 0.58 |
| | 16 | 0.15 | 0.95 | 0.75 | 0.57 | 0.58 |
| | 17 | 0.14 | 0.97 | 0.80 | 0.57 | 0.58 |
| | 18 | 0.13 | 0.98 | 0.83 | 0.56 | 0.58 |
| | 19 | 0.11 | 0.98 | 0.88 | 0.56 | 0.58 |
| | 20 | 0.11 | 1.00 | 1.00 | 0.56 | 0.58 |
| | 21 | 0.09 | 1.00 | 1.00 | 0.56 | 0.58 |
| | 22 | 0.08 | 1.00 | 1.00 | 0.56 | 0.57 |
| | 23 | 0.08 | 1.00 | 1.00 | 0.55 | 0.57 |
| | 24 | 0.06 | 1.00 | 1.00 | 0.55 | 0.57 |
| | 25 | 0.06 | 1.00 | 1.00 | 0.55 | 0.56 |
| | 26 | 0.06 | 1.00 | 1.00 | 0.55 | 0.56 |
| | 27 | 0.06 | 1.00 | 1.00 | 0.55 | 0.56 |
| | 28 | 0.06 | 1.00 | 1.00 | 0.55 | 0.56 |
| | 29 | 0.05 | 1.00 | 1.00 | 0.55 | 0.56 |
| | 30 | 0.05 | 1.00 | 1.00 | 0.55 | 0.56 |
| | 31 | 0.05 | 1.00 | 1.00 | 0.55 | 0.56 |
| | 32 | 0.05 | 1.00 | 1.00 | 0.55 | 0.56 |
| | 33 | 0.03 | 1.00 | 1.00 | 0.55 | 0.55 |
| | 34 | 0.03 | 1.00 | 1.00 | 0.54 | 0.55 |
| | 35 | 0.03 | 1.00 | 1.00 | 0.54 | 0.55 |
| | 36 | 0.03 | 1.00 | 1.00 | 0.54 | 0.55 |
| | 37 | 0.03 | 1.00 | 1.00 | 0.54 | 0.55 |
| | 38 | 0.03 | 1.00 | 1.00 | 0.54 | 0.55 |
| | 39 | 0.03 | 1.00 | 1.00 | 0.54 | 0.55 |
| | 40 | 0.03 | 1.00 | 1.00 | 0.54 | 0.55 |
| | 41 | 0.03 | 1.00 | 1.00 | 0.54 | 0.55 |
| | 42 | 0.03 | 1.00 | 1.00 | 0.54 | 0.55 |
| | 43 | 0.03 | 1.00 | 1.00 | 0.54 | 0.55 |
| | 44 | 0.03 | 1.00 | 1.00 | 0.54 | 0.55 |
| | 45 | 0.03 | 1.00 | 1.00 | 0.54 | 0.55 |
| | 46 | 0.03 | 1.00 | 1.00 | 0.54 | 0.55 |
| | 47 | 0.03 | 1.00 | 1.00 | 0.54 | 0.54 |
| | 48 | 0.00 | 1.00 | 1.00 | 0.54 | 0.54 |
| | 49 | 0.00 | 1.00 | 1.00 | 0.54 | 0.54 |
| | 50 | 0.00 | 1.00 | 1.00 | 0.54 | 0.54 |
| | 51 | 0.00 | 1.00 | 1.00 | 0.54 | 0.54 |
| | 52 | 0.00 | 1.00 | 1.00 | 0.54 | 0.54 |
| | 53 | 0.00 | 1.00 | 1.00 | 0.53 | 0.53 |
| | 54 | 0.00 | 1.00 | 1.00 | 0.53 | 0.53 |
| | 55 | 0.00 | 1.00 | 1.00 | 0.53 | 0.53 |
| | 56 | 0.00 | 1.00 | - | 0.53 | 0.53 |
| | 57 | 0.00 | 1.00 | - | 0.53 | 0.53 |
| | 58 | 0.00 | 1.00 | - | 0.53 | 0.53 |

### Performance Metrics in Predicting Ulcerative Colitis Status from Number of Positive Foods Using 95th Percentile of ELISA Signal to determine Positive

**Table 14B**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| MALE | 1 | 0.90 | 0.25 | 0.36 | 0.85 | 0.46 |
| | 2 | 0.83 | 0.48 | 0.43 | 0.86 | 0.59 |
| | 3 | 0.79 | 0.64 | 0.51 | 0.87 | 0.69 |
| | 4 | 0.74 | 0.72 | 0.55 | 0.85 | 0.72 |
| | 5 | 0.64 | 0.78 | 0.58 | 0.82 | 0.73 |
| | 6 | 0.58 | 0.83 | 0.62 | 0.80 | 0.75 |
| | 7 | 0.53 | 0.87 | 0.65 | 0.79 | 0.76 |
| | 8 | 0.48 | 0.89 | 0.67 | 0.78 | 0.76 |
| | 9 | 0.44 | 0.91 | 0.69 | 0.77 | 0.76 |
| | 10 | 0.40 | 0.92 | 0.69 | 0.76 | 0.75 |
| | 11 | 0.36 | 0.92 | 0.69 | 0.75 | 0.74 |
| | 12 | 0.33 | 0.93 | 0.69 | 0.75 | 0.74 |
| | 13 | 0.31 | 0.93 | 0.69 | 0.74 | 0.73 |
| | 14 | 0.30 | 0.94 | 0.70 | 0.74 | 0.73 |
| | 15 | 0.28 | 0.95 | 0.73 | 0.74 | 0.73 |
| | 16 | 0.27 | 0.95 | 0.73 | 0.73 | 0.73 |
| | 17 | 0.24 | 0.96 | 0.75 | 0.73 | 0.73 |
| | 18 | 0.22 | 0.97 | 0.75 | 0.72 | 0.73 |
| | 19 | 0.20 | 0.97 | 0.75 | 0.72 | 0.72 |
| | 20 | 0.19 | 0.97 | 0.75 | 0.72 | 0.72 |
| | 21 | 0.17 | 0.97 | 0.75 | 0.71 | 0.72 |
| | 22 | 0.14 | 0.98 | 0.75 | 0.71 | 0.71 |
| | 23 | 0.12 | 0.98 | 0.75 | 0.70 | 0.70 |
| | 24 | 0.10 | 0.98 | 0.67 | 0.70 | 0.70 |
| | 25 | 0.08 | 0.98 | 0.67 | 0.69 | 0.70 |
| | 26 | 0.07 | 0.98 | 0.67 | 0.69 | 0.69 |
| | 27 | 0.06 | 0.98 | 0.67 | 0.69 | 0.69 |
| | 28 | 0.04 | 0.98 | 0.67 | 0.69 | 0.69 |
| | 29 | 0.04 | 0.98 | 0.50 | 0.69 | 0.68 |
| | 30 | 0.03 | 0.98 | 0.50 | 0.68 | 0.68 |
| | 31 | 0.03 | 0.98 | 0.50 | 0.68 | 0.68 |
| | 32 | 0.00 | 0.99 | 0.50 | 0.68 | 0.68 |
| | 33 | 0.00 | 1.00 | 0.00 | 0.68 | 0.68 |
| | 34 | 0.00 | 1.00 | 0.00 | 0.68 | 0.68 |
| | 35 | 0.00 | 1.00 | 0.00 | 0.68 | 0.67 |
| | 36 | 0.00 | 1.00 | 0.00 | 0.68 | 0.67 |
| | 37 | 0.00 | 1.00 | 0.00 | 0.68 | 0.68 |
| | 38 | 0.00 | 1.00 | 0.00 | 0.68 | 0.68 |
| | 39 | 0.00 | 1.00 | 0.00 | 0.68 | 0.68 |
| | 40 | 0.00 | 1.00 | 0.00 | 0.68 | 0.68 |
| | 41 | 0.00 | 1.00 | 0.00 | 0.68 | 0.68 |
| | 42 | 0.00 | 1.00 | 0.00 | 0.68 | 0.68 |
| | 43 | 0.00 | 1.00 | 0.00 | 0.68 | 0.68 |
| | 44 | 0.00 | 1.00 | 0.00 | 0.68 | 0.68 |
| | 45 | 0.00 | 1.00 | 0.00 | 0.68 | 0.68 |
| | 46 | 0.00 | 1.00 | - | 0.68 | 0.68 |
| | 47 | 0.00 | 1.00 | - | 0.68 | 0.68 |
| | 48 | 0.00 | 1.00 | - | 0.68 | 0.68 |
| | 49 | 0.00 | 1.00 | - | 0.68 | 0.68 |
| | 50 | 0.00 | 1.00 | - | 0.68 | 0.68 |
| | 51 | 0.00 | 1.00 | - | 0.68 | 0.68 |
| | 52 | 0.00 | 1.00 | - | 0.68 | 0.68 |
| | 53 | 0.00 | 1.00 | - | 0.68 | 0.68 |
| | 54 | 0.00 | 1.00 | - | 0.68 | 0.68 |
| | 55 | 0.00 | 1.00 | - | 0.68 | 0.68 |
| | 56 | 0.00 | 1.00 | - | 0.68 | 0.68 |
| | 57 | 0.00 | 1.00 | - | 0.68 | 0.68 |
| | 58 | 0.00 | 1.00 | - | 0.68 | 0.68 |

## Claims

1. A test panel for testing food intolerance in patients diagnosed with or suspected to have ulcerative colitis, comprising:
a plurality of distinct food preparations, wherein each food preparation is independently coupled to an individually addressable solid carrier;
wherein the plurality of distinct food preparations consists of green pea, cantaloupe, pinto bean, cucumber, green pepper, grapefruit, carrot, orange, almond, sardine, sweet potato, broccoli, garlic, lima bean, squashes, celery, string bean, tomato, cauliflower, black walnut, sunflower seed, cane sugar, buck wheat, soybean, lemon, barley, oat, oyster, mustard, rye, peach, chili pepper, spinach, peanut, avocado, shrimp, pineapple, cola nut, rice, cabbage, butter, eggplant, apple, egg, wheat, cottage cheese, sole, cashew, olive, parsley, corn, honey, chocolate, cow's milk, potato, onion, tea, and tobacco.

2. The test panel of claim 1, wherein the distinct food preparations are crude filtered aqueous extracts or processed aqueous extracts.

3. The test panel claim 1 or claim 2, wherein the solid carrier is a well of a multiwell plate, a bead, an electrical sensor, a chemical sensor, a microchip or an adsorptive film.

4. An *in vitro* method of testing food intolerance in patients diagnosed with or suspected to have ulcerative colitis, comprising:
contacting a food preparation with a bodily fluid of a patient that is diagnosed with or suspected to have ulcerative colitis, wherein the bodily fluid is associated with a gender identification, and wherein the step of contacting is performed under conditions that allow IgG from the bodily fluid to bind to at least one component of the food preparation;
measuring IgG bound to the at least one component of the food preparation to obtain a signal;
comparing the signal to a gender-stratified reference value for the food preparation using the gender identification to obtain a result; and
updating or generating a report using the result,
wherein the step of contacting a food preparation is performed with a plurality of distinct food preparations, and
wherein the plurality of distinct food preparations consists of green pea, cantaloupe, pinto bean, cucumber, green pepper, grapefruit, carrot, orange, almond, sardine, sweet potato, broccoli, garlic, lima bean, squashes, celery, string bean, tomato, cauliflower, black walnut, sunflower seed, cane sugar, buck wheat, soybean, lemon, barley, oat, oyster, mustard, rye, peach, chili pepper, spinach, peanut, avocado, shrimp, pineapple, cola nut, rice, cabbage, butter, eggplant, apple, egg, wheat, cottage cheese, sole, cashew, olive, parsley, corn, honey, chocolate, cow's milk, potato, onion, tea, and tobacco.

5. The method of claim 4, wherein the bodily fluid of the patient is whole blood, plasma, serum, saliva, or a fecal suspension.

6. The method of claim 4 or claim 5, wherein the gender-stratified reference value for each of the food preparations is the 90^{th} percentile rank, or higher, of signals obtained by contacting bodily fluid from a control group of subjects that is not diagnosed with or suspected of having ulcerative colitis with the food preparation.

## Patentansprüche

1. Testpanel zum Testen einer Nahrungsmittelunverträglichkeit bei Patienten mit Diagnose von oder Verdacht auf Colitis ulcerosa, umfassend:
eine Vielzahl unterschiedlicher Nahrungsmittelzubereitungen, wobei jede Nahrungsmittelzubereitung unabhängig an einen einzeln adressierbaren festen Träger gekoppelt ist;
wobei die Vielzahl unterschiedlicher Nahrungsmittelzubereitungen aus grüner Erbse, Cantaloupe-Melone, Pintobohne, Gurke, grüner Paprika, Grapefruit, Karotte, Orange, Mandel, Sardine, Süßkartoffel, Brokkoli, Knoblauch, Limabohne, Kürbissen, Sellerie, Gartenbohne, Tomate, Blumenkohl, Schwarznuss, Sonnenblumenkernen, Rohrzucker, Buchweizen, Sojabohne, Zitrone, Gerste, Hafer, Auster, Senf, Roggen, Pfirsich, Chilischote, Spinat, Erdnuss, Avocado, Garnele, Ananas, Kolanuss, Reis, Kohl, Butter, Aubergine, Apfel, Ei, Weizen, Hüttenkäse, Seezunge, Cashew, Olive, Petersilie, Mais, Honig, Schokolade, Kuhmilch, Kartoffel, Zwiebel, Tee und Tabak besteht.

2. Testpanel nach Anspruch 1, wobei die unterschiedlichen Nahrungsmittelzubereitungen rohe gefilterte wässrige Extrakte oder verarbeitete wässrige Extrakte sind.

3. Testpanel nach Anspruch 1 oder Anspruch 2, wobei der feste Träger eine Vertiefung einer Mikrotiterplatte, eine Perle, ein elektrischer Sensor, ein chemischer Sensor, ein Mikrochip oder eine Adsorptionsfolie ist.

4. In-vitro-Verfahren zum Testen einer Nahrungsmittelunverträglichkeit bei Patienten mit Diagnose oder Verdacht auf Colitis ulcerosa, umfassend:
Inkontaktbringen einer Nahrungsmittelzubereitung mit einer Körperflüssigkeit eines Patienten, der eine Diagnose von oder ein Verdacht auf Colitis ulcerosa aufweist, wobei die Körperflüssigkeit mit einer Geschlechtsidentifikation in Zusammenhang steht und wobei der Schritt des Inkontaktbringens unter Bedingungen durchgeführt wird, die ein Binden von IgG aus der Körperflüssigkeit an mindestens einen Bestandteil der Nahrungsmittelzubereitung ermöglichen;
Messen von an den mindestens einen Bestandteil der Nahrungsmittelzubereitung gebundenem IgG, um ein Signal zu erhalten;
Vergleichen des Signals mit einem nach Geschlecht stratifizierten Referenzwert für die Nahrungsmittelzubereitung unter Verwendung der Geschlechtsidentifikation, um ein Ergebnis zu erhalten; und
Aktualisieren oder Erstellen eines Berichts unter Verwendung des Ergebnisses,
wobei der Schritt des Inkontaktbringens einer Nahrungsmittelzubereitung mit einer Vielzahl unterschiedlicher Nahrungsmittelzubereitungen durchgeführt wird, und
wobei die Vielzahl unterschiedlicher Nahrungsmittelzubereitungen aus grüner Erbse, Cantaloupe-Melone, Pintobohne, Gurke, grüner Paprika, Grapefruit, Karotte, Orange, Mandel, Sardine, Süßkartoffel, Brokkoli, Knoblauch, Limabohne, Kürbissen, Sellerie, Gartenbohne, Tomate, Blumenkohl, Schwarznuss, Sonnenblumenkernen, Rohrzucker, Buchweizen, Sojabohne, Zitrone, Gerste, Hafer, Auster, Senf, Roggen, Pfirsich, Chilischote, Spinat, Erdnuss, Avocado, Garnele, Ananas, Kolanuss, Reis, Kohl, Butter, Aubergine, Apfel, Ei, Weizen, Hüttenkäse, Seezunge, Cashew, Olive, Petersilie, Mais, Honig, Schokolade, Kuhmilch, Kartoffel, Zwiebel, Tee und Tabak besteht.

5. Verfahren nach Anspruch 4, wobei die Körperflüssigkeit des Patienten Vollblut, Plasma, Serum, Speichel oder eine Stuhlsuspension ist.

6. Verfahren nach Anspruch 4 oder Anspruch 5, wobei der nach Geschlecht stratifizierte Referenzwert für jede der Nahrungsmittelzubereitungen der 90. Perzentilrang oder höher von Signalen ist, die durch das Inkontaktbringen von Körperflüssigkeit von einer Kontrollgruppe von Subjekten, die keine Diagnose von oder Verdacht auf Colitis ulcerosa aufweisen, mit der Nahrungsmittelzubereitung erhalten werden.

## Revendications

1. Panel de test pour tester l'intolérance alimentaire chez des patients diagnostiqués ou suspectés de souffrir de colite ulcéreuse, comprenant :
une pluralité de préparations alimentaires distinctes, dans lequel chaque préparation alimentaire est couplée indépendamment à un support solide adressable individuellement ;
dans lequel la pluralité de préparations alimentaires distinctes est constituée de pois verts, cantaloup, haricots pinto, concombre, poivron vert, pamplemousse, carotte, orange, amande, sardine, patate douce, brocoli, ail, haricot de Lima, courges, céleri, haricot vert, tomate, chou-fleur, noix noire, graines de tournesol, sucre de canne, sarrasin, soja, citron, orge, avoine, huître, moutarde, seigle, pêche, piment, épinards, arachide, avocat, crevette, ananas, noix de cola, riz, chou, beurre, aubergine, pomme, oeuf, blé, fromage blanc, sole, noix de cajou, olive, persil, maïs, miel, chocolat, lait de vache, pomme de terre, oignon, thé et tabac.

2. Panel de test selon la revendication 1, dans lequel les préparations alimentaires distinctes sont des extraits aqueux bruts filtrés ou des extraits aqueux traités.

3. Panel de test selon la revendication 1 ou la revendication 2, dans lequel le support solide est un puits d'une plaque multipuits, une bille, un capteur électrique, un capteur chimique, une micropuce ou un film adsorbant.

4. Procédé in vitro de test d'intolérance alimentaire chez des patients diagnostiqués ou suspectés de souffrir de colite ulcéreuse, comprenant :
la mise en contact d'une préparation alimentaire avec un fluide corporel d'un patient qui est diagnostiqué ou suspecté de souffrir de colite ulcéreuse, dans lequel le fluide corporel est associé à une identification de genre, et dans lequel l'étape de mise en contact est réalisée dans des conditions qui permettent aux IgG provenant du fluide corporel de se lier à au moins un composant de la préparation alimentaire ;
la mesure des IgG liés à l'au moins un composant de la préparation alimentaire pour obtenir un signal ;
la comparaison du signal à une valeur de référence stratifiée par genre pour la préparation alimentaire à l'aide de l'identification de genre pour obtenir un résultat ; et
la mise à jour ou la génération d'un rapport en utilisant le résultat,
dans lequel l'étape de mise en contact d'une préparation alimentaire est réalisée avec une pluralité de préparations alimentaires distinctes, et
dans lequel la pluralité de préparations alimentaires distinctes est constituée de pois verts, cantaloup, haricots pinto, concombre, poivron vert, pamplemousse, carotte, orange, amande, sardine, patate douce, brocoli, ail, haricot de Lima, courges, céleri, haricot vert, tomate, chou-fleur, noix noire, graines de tournesol, sucre de canne, sarrasin, soja, citron, orge, avoine, huître, moutarde, seigle, pêche, piment, épinards, arachide, avocat, crevette, ananas, noix de cola, riz, chou, beurre, aubergine, pomme, oeuf, blé, fromage blanc, sole, noix de cajou, olive, persil, maïs, miel, chocolat, lait de vache, pomme de terre, oignon, thé et tabac.

5. Procédé selon la revendication 4, dans lequel le fluide corporel du patient est du sang total, du plasma, du sérum, de la salive ou une suspension fécale.

6. Procédé selon la revendication 4 ou la revendication 5, dans lequel la valeur de référence stratifiée par genre pour chacune des préparations alimentaires est le rang du 90e percentile, ou plus, des signaux obtenus en mettant en contact un fluide corporel provenant d'un groupe témoin de sujets qui n'ont été diagnostiqué ou suspectés de souffrir de colite ulcéreuse avec la préparation alimentaire.
